# EUROPEAN PATENT SPECIFICATION

(11) **EP 4 669 333 B1**
(45) Date of publication and mention of the grant of the patent: **17.06.2026**
(21) Application number: 24725642.3
(22) Date of filing: 22.03.2024
(51) Int. Cl.: A61K 38/00, A61K 48/00, A61K 38/17, A61Q 19/08, A61P 43/00, C12N 9/14, C12N 9/24

(54) **KLOTHO MRNA**
KLOTHO-MRNA
ARNM DE KLOTHO

(30) Priority: 22.03.2023 EP 23163488
(43) Date of publication of application: 31.12.2025
(60) Divisional application: 26153367.3
(73) Proprietor: ADvantage Therapeutics, Inc., Miami, FL 33127 (US)
(72) Inventor: HOFFMANN, Thomas, 1220 Wien (AT); FERNANDEZ SANTANA III, Agustin, Coral Gables, FL 33134 (US); SCHMIDT, Walter, 1020 Wien (AT)
(74) Representative: SONN Patentanwälte GmbH & Co KG
(86) International application number: PCT/US2024/021148
(87) International publication number: WO 2024/197254

(56) References cited:
- EP-A1- 0 945 506
- WO-A2-2013/151666
- DATABASE Geneseq [online] 5 December 2013 (2013-12-05), "Human diseases associated protein encoding optimized ORF, SEQ ID 1493.", XP093078026, retrieved from EBI accession no. GSN:BAW43099 Database accession no. BAW43099
- DATABASE Geneseq [online] 5 December 2013 (2013-12-05), "Human diseases associated protein, SEQ ID 1117.", XP093078137, retrieved from EBI accession no. GSP:BAW42723 Database accession no. BAW42723
- DATABASE EMBL [online] 18 October 2011 (2011-10-18), "WO 1998029544-A/2: Novel polypeptide, novel DNA and novel antibody.", XP093078160, retrieved from EBI accession no. EM_PAT:HV446746 Database accession no. HV446746
- GRZEGORZ KUDLA ET AL: "High guanine and cytosine content increases mRNA levels in mammalian cells", PLOS BIOLOGY, PUBLIC LIBRARY OF SCIENCE, UNITED STATES, vol. 4, no. 6, 1 June 2006 (2006-06-01), pages 933 - 942, XP002484439, ISSN: 1544-9173, DOI: 10.1371/JOURNAL.PBIO.0040180

## Description

The present invention relates to the field of Klotho, especially Klotho mRNA and formulations, kits and uses related thereto.

Klotho is an enzyme that in humans is encoded by the KL gene. There are three subfamilies of Klotho: α-Klotho, β-Klotho, and γ-Klotho. When the subfamily is not specified, the word "Klotho" typically means the α-Klotho subfamily, because α-Klotho was discovered before the other subfamily members. Therefore, "Klotho" and "a-Klotho" are used interchangeably herein.

Klotho is described in more detail, e.g., in WO 2019/113373 A2: Two transcripts that arise from a single Klotho gene through alternative RNA splicing have been identified. The first transcript encodes Klotho isoform 1 - a full-length, 1,012 amino acid, single-pass transmembrane-membrane protein, with a short cytoplasmic tail (human residues 1003-1012), a transmembrane (TM) domain (human residues 982-1002), and an extracellular region or domain (human residues 1-981) comprising two (largely) homologous (internal repeat) domains (termed KL1 (human residues 56-506, which is 450 residues long) and KL2 (human residues 515-953, which is 438 residues long), which each share 20%-40% amino acid sequence homology to b-glucosidases, but may lack similar levels of glucosidase catalytic activity), and a signal peptide (SP) domain (human residues 1-33; also referred to as "signal sequence", "SS"). The SP, KL1, and KL2 domain-containing extracellular region (human residues 1-981) may be enzymatically cleaved by α/β-secretases, and released into the circulatory stream as a 130 kDa circulating protein, termed soluble Klotho (or sKlotho, s-Klotho, alpha soluble Klotho, etc.). The extracellular region can also be cleaved into separate 68 kDa protein (KL1 + SS) and 64 kDa protein (KL2).

The second transcript, a splicing variant of alpha-Klotho mRNA, encodes a second isoform of Klotho protein corresponding mainly to the KL1 domain. The internal splice donor site is thought to be located in exon 3 of the Klotho gene. The resultant alternatively spliced transcript contains a 50 bp insertion after exon 3, with an in-frame translation stop codon at the end thereof. The expressed protein product is secreted into the circulation and is termed secreted Klotho (or Klotho isoform 2), which differs from the canonical sequence of isoform 1 at amino acid residues 535-549, and with amino acid residues 550-1012 missing.

Klotho was first described in 1997 as a repressor of ageing. Klotho knock out mice develop severe ageing phenotypes 4-5 weeks after birth and die within 9 weeks. Mice overexpressing Klotho from birth on live up to 30% longer than their wildtype littermates.

The Klotho gene is highly expressed in the liver, kidney, and the brain. The anti-aging benefits are mostly related to its inhibition of the cellular uptake of glucose (i.e., inhibiting the interaction of insulin with the IGF-1 receptor). Furthermore, Klotho positively impacts neuroprotection and neurogenesis, stimulation of autophagy, regulation of oxidative stress, growth factor signaling, ion homeostasis and organ protection.

Several studies show that Klotho serum levels are high in children and young adults and decrease by 30-40% with age. Children also have better insulin uptake. Klotho blood levels below a certain threshold seem to correlate with AD/dementia, heart disease, and decreased lifespan. The slow decline of serum Klotho levels offers an opportunity for supplementation. In addition, the difference of Klotho levels between health and disease is very small. Therefore, a slight increase in Klotho levels may be sufficient to get above the threshold. Klotho can therefore be considered a promising anti-aging compound. EP 0 945 506 B1, for instance, relates to aging-suppressing polypeptides and DNA sequences encoding such peptides, including Klotho.

In addition to the anti-aging effects of Klotho, Klotho deficiencies can be found in many different diseases. Among others: kidney diseases, cardiovascular diseases, brain and lung diseases have been shown to benefit from the elevation of Klotho as reviewed in Prud' homme et al., 2022, table 1 and 3 (Prud' homme et al. "Pathobiology of the Klotho Antiaging Protein and Therapeutic Considerations." Frontiers in Aging 3 (2022)). Hence, Klotho also acts as a tumor suppressor in various kinds of tumors (Sachdeva, et al. "Klotho and the treatment of human malignancies." Cancers 12.6 (2020): 1665).

In view of the potential benefits of Klotho, strategies are being pursued to increase the expression level of Klotho in individuals and/or to administer Klotho, e.g., recombinant s-Klotho. Such strategies and uses of Klotho are described, e.g., in WO 2019/113373 A2, WO2022211420A1, WO2022008971A2, WO2020163017 A1, WO2020106997A1, WO2020106351A1, WO2020039425A1, WO2019140250A1, WO2019113373A2, WO2019113061A1, WO2018098375A1, WO2017210607A1, WO2016127097A1, WO2016088059A1, WO2014152993A1, WO2011084452A1, and WO2008135993A1.

However, despite the promising mechanisms in which Klotho protein has been shown to be relevant, there has yet to be an approved treatment brought to market in the approximately 25 years that Klotho has been discussed as a promising target by major or minor pharmaceutical companies.

It is an object of the present invention to address this need.

Therefore, the present invention provides a Klotho messenger-RNA (mRNA), wherein the mRNA has a 5' CAP region, a 5' untranslated region (5'-UTR), a coding region encoding a Klotho polypeptide, a 3' untranslated region (3'-UTR) and a poly-adenosine tail (poly-A tail), wherein the Klotho polypeptide comprises the signal peptide, the KL1 domain, and the KL2 domain of human Klotho, wherein the coding region encoding the Klotho polypeptide comprises an RNA sequence having at least 93 % sequence identity to SEQ ID NO: 18, and wherein the coding region encoding the Klotho polypeptide has a GC content between 54 % and 64 %.

One of the main reasons why pharmaceutical companies have so far failed to realize the potential of Klotho protein is most likely that the focus of the players in field so far has been on the application of exogeneous manufactured Klotho protein derived e.g. from E. coli or cell culture. These types of proteins miss at least two features of how the Klotho protein is endogenously expressed by the body's own cells, which are:
(1) fully humanized, fully individualized and fully personalized;
(2) the allocation and delivery strategy of the body for Klotho.

Klotho mRNA is not (only) expressed by direct transcription from the DNA within the parental e.g. kidney cell. It is also, most likely even mainly, packed into exosomes (cf Sahu "Regulation of aged skeletal muscle regeneration by circulating extracellular vesicles." Nature Aging 1.12 (2021): 1148-1161). These are then budded off the cell to spread into the blood to reach other target tissues and cells including brain cells. Also, further shortcomings/challenges on the protein level might be taken into account, such as short half-life of the protein itself, intracellular availability, targeting, lack of specificity with intravenous administration, and inability for oral administration. Therefore, despite currently available methods for administering recombinant Klotho and for increasing the expression level of endogenous Klotho, new and improved options for harnessing the benefits of this protein are needed. In particular, new and improved methods and maybe even more important new formats including exosomes for providing the Klotho benefit portfolio to individuals in order to positively impact on aging and/or to harness the therapeutic outcome are desired.

mRNA has emerged as a revolutionary tool in the field of medicine due to its ability to carry genetic instructions to cells and direct the production of specific proteins. While the use of mRNA-based vaccines has garnered much attention in recent times, mRNA-based therapeutics have also shown tremendous potential in treating a wide range of conditions and diseases. The basic principle behind exogenous mRNA-based therapeutics is to introduce such mRNA molecules into cells, which can then instruct the cells to produce therapeutic proteins that are otherwise missing or dysfunctional in the body or are just expressed at insufficient levels.

The success of such mRNA, in particular mRNA-based therapeutics, heavily relies on the level of expression of the mRNA in the body, as it determines the amount of protein produced. There are several general approaches to improve the expression levels of mRNA, including modifying the 5' and 3' untranslated regions (UTRs) of the mRNA, and/or optimizing the formulation and delivery method. Taking together, it is often the individual mRNA sequence itself that determines the level of expression achieved.

General methods for the preparation and use of mRNA molecules for such methods are described, e.g., in WO 2013/151736 A2. WO 2013/151736 A2 describes a large number of different examples for such mRNAs. Among them, Example 125 in paragraph [0001618] refers to the intravenous administration of a Klotho mRNA to CD1 mice. The RNA sequence is included in the sequence listing of WO 2013/151736 A2 as SEQ ID NO: 196. Analysis of this sequence shows that it apparently codes for Klotho isoform 2. It is stated in Example 125 of WO 2013/151736 A2 that said mRNA was chemically modified in various ways and that it was administered complexed with Lipofectamine 2000. However, WO 2013/151736 A2 does not state that the RNA sequence itself was optimized in any way.

While the use of mRNA as a therapeutic agent has shown great potential for treating various diseases, its application to specific cases can be challenging. A primary reason that may have hampered the development of effective Klotho mRNA in the past may be that inadequate expression levels are achieved. Low expression levels are a significant challenge in the development of mRNA-based therapeutics. mRNA molecules carry instructions for protein synthesis, and their expression level determines the amount of protein that will be produced in cells. Inadequate expression levels can result in insufficient therapeutic effects, thus limiting the clinical utility. If the expression level is too low, the therapeutic effect may not be sufficient, and the treatment may fail. Therefore, improving the expression levels of mRNA is necessary to enable the development of more effective therapeutic and other applications.

The RNA sequence is a critical determinant of protein expression levels, and modifying it can significantly impact the efficacy of mRNA-based therapeutics. With the help of codon optimization algorithms, it is possible to modify the RNA sequence without altering the protein sequence. These algorithms typically take into account various factors such as codon usage bias, amino acid frequency, and secondary structure of the mRNA. Many of these codon optimization algorithms are available through software or online, and researchers can easily access them to optimize the sequence of their mRNA of interest.

The present inventors set out to obtain mRNA for the expression of Klotho with the aim of achieving high expression levels. For initial experiments, they selected soluble Klotho, i.e., corresponding to amino acids M1 to S981 of Klotho isoform 1. They started from the sequence of native human Klotho isoform 1 according to NCBI Reference Sequence: NM_004795.4. As described in more detail in the Examples, the sequence coding for soluble Klotho, i.e., amino acids M1 to S981 was extracted (SEQ ID NO: 17). This sequence is 2946 nt long and can be divided into the signal peptide (amino acids 1-33; nt 1-99) and KL1 and KL2 domains including adjacent linking regions (amino acids E34 to S981; nt 100-2946). The part coding for the signal peptide was kept constant and the latter part of the sequence corresponding to nt 100-2946 was optimized using four different codon optimization algorithms, as provided through websites of different gene synthesis providers:
- "GENEius algorithm" (https://eurofinsgenomics.eu/en/gene-synthesis-molecular-biology/gene-synthesis/geneius/) provided by Eurofins Genomics Germany GmbH: resulting in SEQ ID NO: 25.
- "IDT Codon Optimization Tool" (https://eu.idtdna.com/pages/tools/codon-optimization-tool) provided by Integrated DNA Technologies, Inc.: resulting in SEQ ID NO: 26
- "ExpOptimizer" (https://www.novoprolabs.com/tools/codon-optimization) provided by NovoPro Bioscience, Inc.: resulting in SEQ ID NO: 27
- "Twist Codon Optimization Tool" (https://www.twistbioscience.com/twist-ordering-platform) provided by Twist Bioscience: resulting in SEQ ID NO: 28

The expression levels obtained with the four commercially optimized sequence variants varied quite substantially (see Examples and Figures). Therefore, the inventors set out to test if it would be possible to achieve even higher expression levels. When the inventors compared the optimized sequences to the sequence of native human Klotho (SEQ ID NO: 17), they eventually made the surprising realization that the optimized sequences all had significantly lower GC contents (see Table 1). Investigating this further, they found that the expression level appeared to follow the GC content: The sequence obtained from the "GENEius algorithm" (SEQ ID NO: 25) had the highest GC content (52.6 %) and also gave the highest expression levels (see Examples and Figures). The second best result was achieved with the sequence obtained from "ExpOptimizer" (SEQ ID NO: 27; GC content 51.1 %), followed by the sequence from the "IDT Codon Optimization Tool" (SEQ ID NO: 26; GC content: 49.9 %) and finally the "Twist Codon Optimization Tool" (SEQ ID NO: 28; GC content: 48.7%).

The inventors therefore came up with the hypothesis that for the expression of Klotho, a high GC content may be beneficial. They tested this hypothesis by creating a number of mRNA variants having different GC contents, namely:
- Variant "GC62" (SEQ ID NO: 18); GC content: 61,6 %
- Variant "GC60" (SEQ ID NO: 19); GC content: 60,1 %
- Variant "GC57" (SEQ ID NO: 22); GC content: 57,4 %
- Variant "GC55" (SEQ ID NO: 23); GC content: 54,9 %
- Variant "GC53" (SEQ ID NO: 24); GC content: 52,8 %

Surprisingly, a direct relationship between the GC content and the expression level was indeed found. The expression level continued to increase going from variant "GC53" to variant "GC62", i.e., even above the GC content of native Klotho.

A summary of the tested sequences and their GC content is given in the following Table.

**Table 1: Tested mRNA variants coding for soluble Klotho (M1 to S981 of human Klotho isoform 1) and GC content:**

| **Codon usage** | **SEQ ID NO:** | **GC content (%)** |
|---|---|---|
| Native human Klotho | 17 | 54,5 |
| Variant "GC62" | 18 | 61,6 |
| Variant "GC60" | 19 | 60,1 |
| Variant "GC57" | 22 | 57,4 |
| Variant "GC55" | 23 | 54,9 |
| Variant "GC53" | 24 | 52,8 |
| GENEius algorithm | 25 | 52,6 |
| IDT Codon Optimization Tool | 26 | 49,9 |
| ExpOptimizer | 27 | 51,1 |
| Twist Codon Optimization Tool | 28 | 48,1 |

In addition, the inventors examined the sequence identity of the various sequences. It was found that the closer the variants were in sequence identity to the best performing variant "GC62", the higher was the expression level. Interestingly, this trend was also true for four sequences obtained from the commercial sequence optimization tools. The best performing sequence obtained from the "GENEius algorithm" (SEQ ID NO: 25) had the highest sequence identity to "GC62" (80.6 %); followed by the second best sequence which was obtained from "ExpOptimizer" (SEQ ID NO: 27; 79.6 % sequence identity to "GC62"); followed by the third best sequence, which was from the "IDT Codon Optimization Tool" (SEQ ID NO: 26; 77.3 % sequence identity to "GC62") and finally the "Twist Codon Optimization Tool" that gave the lowest results (SEQ ID NO: 28; 76.4 % sequence identity to "GC62"). All the custom variants had higher sequence identities to "GC62" than the ones obtained from the commercial sequence optimization tools. Also in this case, expression levels increased continuously with increasing sequence identity to "GC62", starting from the variant "GC53" (SEQ ID NO: 24; 81.6 % sequence identity to "GC62") and going up all the way to "GC60" (SEQ ID NO: 19; 92.0% sequence identity to "GC62"). Thus, a direct relationship between sequence identity to "GC62" and expression level was found. The sequence identities between all the variants tested are summarized in the following Table.

**Table 2: Sequence identities between different mRNA variants tested. Values are given in (%).**

| **SEQ ID NO:** | **17** | **18** | **19** | **22** | **23** | **24** | **25** | **26** | **27** | **28** |
|---|---|---|---|---|---|---|---|---|---|---|
| **17** | 100 | | | | | | | | | |
| **18** | 80,3 | 100 | | | | | | | | |
| **19** | 80,1 | 92 | 100 | | | | | | | |
| **22** | 79,2 | 88,6 | 89,5 | 100 | | | | | | |
| **23** | 79,2 | 85,2 | 86 | 90,3 | 100 | | | | | |
| **24** | 78,1 | 81,6 | 83,3 | 85,4 | 85 | 100 | | | | |
| **25** | 77 | 80,4 | 79,9 | 79,8 | 79,7 | 79,4 | 100 | | | |
| **26** | 76,6 | 77,3 | 77,5 | 77,2 | 77,8 | 77,6 | 77,8 | 100 | | |
| **27** | 77,7 | 79,6 | 79,3 | 79,2 | 78,1 | 78,4 | 78,7 | 76,9 | 100 | |
| **28** | 66,2 | 76,4 | 77,1 | 77,3 | 77 | 77,1 | 78 | 78,3 | 77,9 | 100 |

It is interesting to note that the four sequences obtained from the commercial sequence optimization tools do not share a high degree of sequence identity between each other. As follows from the above table, each pair among the four sequences has a sequence identity below 79 %. Thus, even though the four sequences were apparently optimized in different ways, none of the sequence comes close to the preferred variant "GC 62", the best being the one obtained from the "GENEius algorithm" (SEQ ID NO: 25) having a sequence identity to "GC62" of 80.6 %.

In addition to the sequence variants coding for soluble Klotho (amino acids M1 to S981) described above, the present inventors also designed corresponding sequence variants coding for human Klotho isoform 2 (amino acids M1 to H549) . As laid out above, human Klotho isoform 2 shares the amino acids M1 to V534 with Klotho isoform 1. Thus, the inventors designed a "GC62" variant of isoform 2 (SEQ ID NO: 41), wherein nucleotides 1 to 1602 (coding for M1 to V534) are identical to nucleotides 1 to 1602 of the "GC62" variant of isoform 1 (SEQ ID NO: 18). Only the nucleotides 1603 to 1650 (coding for D535 to H549) are different in the isoform 2 variant SEQ ID NO: 41 compared to the isoform 1 variant SEQ ID NO: 18. The GC content of the isoform 2 variant SEQ ID NO: 41 is similarly to that of the isoform 1 variant SEQ ID NO: 18, namely 62.8 %.

It is instructive to compare the preferred isoform 2 variants created in the context of the invention to the Klotho mRNA sequence disclosed in WO 2013/151736 A2, mentioned above. SEQ ID NO: 192 of WO 2013/151736 A2 has 1793 nucleotides and contains an open reading frame of 1650 nucleotides that appears to encode for human Klotho isoform 2. The present inventors therefore compared this sequence to the best performing "GC62" variant SEQ ID NO: 41 created in the context of the invention. Strikingly, the sequence used in WO 2013/151736 A2 also has a lower GC content (53.8 % for SEQ ID NO: 192 of WO 2013/151736 A2; compared to 62.8 % for SEQ ID NO: 41 of the present application). In addition, SEQ ID NO: 192 of WO 2013/151736 A2 only has 75.7 % sequence identity to SEQ ID NO: 41 of the present application. Thus, the sequence variants created in the context of the present invention are advantageous over the mRNA sequence disclosed in WO 2013/151736 A2 for similar reasons as laid out above for the commercially optimized variants of mRNA encoding Klotho isoform 1 - namely, higher GC content and higher sequence identity to the best performing "GC62" variants created in the context of the present invention.

Kudla et al. ("High guanine and cytosine content increases mRNA levels in mammalian cells." PLoS Biology 4.6 (2006): e180) describe that high guanine and cytosine content can increase mRNA levels in mammalian cells. However, according to Kudla et al., this effect was not due to different translation rates of GC-rich and GC-poor mRNA, but the efficient expression of GC-rich genes resulted from their increased steady-state mRNA levels.

All embodiments of the invention are described together in the following detailed description and all preferred embodiments relate to all embodiments and aspects alike. All detailed descriptions, e.g. of mRNAs and compositions, relate to preferred embodiments of all aspects of the invention, e.g. compositions for use, uses and methods. All embodiments can be combined with each other, except where stated otherwise.

Human Klotho isoform 1 (UniProt Q9UEF7-1) has the following sequence; SEQ ID NO: 49:

The different domains of the sequence are highlighted above as follows:
- *Italics underline: Signal peptide ("SP") (M1 to A33)*
- **Bold underline: KL1 domain (L56 to F506)**
- ***Bold italics: KL2 domain (L515 to G952)***
- Underline: transmembrane ("TM") domain (L982 to Y1002)
- *Italics*: cytoplasmic tail ("CT") (Y1003 to K1012)
- No emphasis: linking regions ("lr") between domains

Human Klotho isoform 2 (UniProt Q9UEF7-2) has the following sequence; SEQ ID NO: 50:

MPASAPPRRPRPPPPSLSLLLVLLGLGGRRLRAEPGDGAQTWARFSRPPAPEAAGLFQGT
FPDGFLWAVGSAAYQTEGGWQQHGKGASIWDTFTHHPLAPPGDSRNASLPLGAPSPLQPA
TGDVASDSYNNVFRDTEALRELGVTHYRFSISWARVLPNGSAGVPNREGLRYYRRLLERL
RELGVQPVVTLYHWDLPQRLQDAYGGWANRALADHFRDYAELCFRHFGGQVKYWITIDNP
YVVAWHGYATGRLAPGIRGSPRLGYLVAHNLLLAHAKVWHLYNTSFRPTQGGQVSIALSS
HWINPRRMTDHSIKECQKSLDFVLGWFAKPVFIDGDYPESMKNNLSSILPDFTESEKKFI
KGTADFFALCFGPTLSFQLLDPHMKFRQLESPNLRQLLSWIDLEFNHPQIFIVENGWFVS
GTTKRDDAKYMYYLKKFIMETLKAIKLDGVDVIGYTAWSLMDGFEWHRGYSIRRGLFYVD
FLSQDKMLLPKSSALFYQKLIEKNGFPPLPENQPLEGTFPCDFAWGVVDNYIQVSQLTKP
ISSLTKPYH

The different domains of the sequence are highlighted above as follows:
- *Italics underline: Signal peptide ("SP") (M1 to A33)*
- **Bold underline: KL1 domain (L56 to F506)**
- ***Bold italics: sequence corresponding to KL2 domain of isoform 1 (L515 to V534)***
- Underline: alternative sequence (S535 to H549)
- No emphasis: linking regions ("lr") between domains

As indicated in the sequences above, herein all the sequences between the different domains are referred to as "linking regions" (lr). Which sequence a given "lr" refers to, follows from the adjacent domains. For instance, when the present application refers to the part "KL1-lr-KL2" of Klotho isoform 1, this corresponds to amino acids L56 to G952, and "lr" in this instance refers to P507 to P514. Similarly, "lr-KL1-lr-KL2-lr" refers to E34 to S981, wherein the first "lr" is E34 to G55, the second "lr" is P507 to P514, and the third "lr" is to F953 to S981.

In the sequence of Klotho isoform 2, the portion from P507 to H549 (i.e., the C-terminal part of the protein following the KL1 domain) is referred to herein as the "end region" ("end"). Thus, as used herein and as shown above, "end" corresponds to P507 to H549 and includes an lr (P507 to P514), the "sequence corresponding to KL2 domain of isoform 1" (L515 to V534) and the "alternative sequence" (S535 to H549).

All domains and regions of the sequences referred to herein, such as the KL1, KL2, SP, TM, CT, lr, end domains and regions, may refer to the sequences of SEQ ID NO: 49 and 50 shown above or to any variants thereof, in particular variants with modified or improved properties, as laid out herein below. It is preferred, that these domains and regions refer to the sequences of SEQ ID NO: 49 and 50 shown above.

In an aspect, the present invention relates to a Klotho messenger-RNA (mRNA), wherein the mRNA has a 5' CAP region, a 5' untranslated region (5'-UTR), a coding region encoding a Klotho polypeptide, a 3' untranslated region (3'-UTR) and a poly-adenosine Tail (poly-A tail), wherein the Klotho polypeptide comprises the signal peptide, the KL1 domain, and the KL2 domain of human Klotho, wherein the coding region encoding the Klotho polypeptide comprises an RNA sequence having at least 93 % sequence identity to SEQ ID NO: 18, and wherein the coding region encoding the Klotho polypeptide has a GC content between 54 % and 64 %.

It is known in the art that the KL1 domain of human Klotho provides beneficial effects independently from the other parts of the Klotho protein. This is reported, for instance, Gupta et al. ("KL1 domain of longevity factor Klotho mimics the metabolome of cognitive stimulation and enhances cognition in young and aging mice." Journal of Neuroscience 42.19 (2022): 4016-4025). Thus, when the Klotho polypeptide comprises at least the KL1 domain of human Klotho, it can provide important beneficial effects.

According to the claimed invention, the Klotho polypeptide further comprises the signal peptide and the KL2 domain of human Klotho. The Klotho polypeptide comprises the signal peptide, the KL1 domain and the KL2 domain of human Klotho.

In another preferred embodiment, the Klotho polypeptide further comprises the transmembrane (TM) domain of human Klotho. Preferably, the Klotho polypeptide further comprises the cytoplasmic tail (CT) of human Klotho. When the TM and preferably also the CT domains are present, this has the advantage that a membrane anchored version of Klotho protein can be delivered to an individual. This constitutes a significant advantage over strategies used for Klotho so far, in which recombinant protein is administered. When recombinant protein is administered, only soluble versions of Klotho can be used, which means that only the soluble side of the Klotho signalling is accessible. In contrast, by using mRNA, a membrane-anchored version of Klotho can be delivered, which allows exploiting the full breadth of Klotho signalling.

As used herein, the phrases "KL1 domain", "KL2 domain" and "signal peptide" in relation to human Klotho preferably refer to the KL1, KL2 and SP sequences described above with reference to human Klotho isoform 1 (UniProt Q9UEF7-1; SEQ ID NO: 49) and to human Klotho isoform 2 (UniProt Q9UEF7-2; SEQ ID NO: 50) or to any variants thereof, in particular any of the variants described below. Preferably, the signal peptide is SEQ ID NO: 51, the KL1 domain is SEQ ID NO: 52, and the KL2 domain is SEQ ID NO: 54.

A functional variant of Klotho called "KL-VS" is found in a certain part of the human population (see e.g. Arking et al. "Association between a functional variant of the KLOTHO gene and high-density lipoprotein cholesterol, blood pressure, stroke, and longevity" Circulation Research 96.4 (2005): 412-418; Arking et al. "Association of human aging with a functional variant of Klotho" PNAS 99.2 (2002): 856-861.). This variant contains two mutations in the KL1 domain, namely F325V and C370S. This variant is associated with overall decreased mortality and other positive effects. Thus, in a preferred embodiment, the KL1 domain of human Klotho is the VS variant. Preferably, the KL1 domain is SEQ ID NO: 53.

Any other suitable variant of the SP, KL1 domain and/or the KL2 domain of human Klotho can advantageously be used in the context of the present invention. It is preferred, when the Klotho polypeptide is a native Klotho protein, especially a native human Klotho protein, in particular a native human Klotho isoform 1 or a native human Klotho isoform 2, especially SEQ ID NO: 49 OR 50.

It is further preferred when the Klotho polypeptide is a Klotho protein, protein fragment, and/or protein variant as described in WO 2019/113373 A2. The Klotho polypeptide can comprise all or a subset of amino acid residues 1-1012, 1-981, 29- 981, 34-981, 36-981, 131-981, 1-549, 29-549, 34-549, 36-549, or 131-549 of human Klotho isoform 1 or 2. Some embodiments can include a protein having one or more amino acid variations as compared to human Klotho isoform 1. Illustratively, the protein can comprise a human C370 variant. For instance, the protein can include a C370S alteration, thereby comprising S370. The protein can include human F352 or other than F352V in some embodiments. In at least one embodiment, the protein can include the C370S alteration, thereby comprising S370, without a F352V variant, preferably with F352. The protein can include H193 or other than the H193R variant. All other standard amino acid substitutions at amino acid residue (or position) 193, 352, and/or 370 of human Klotho isoform 1 are contemplated and explicitly disclosed herein.

Some embodiments can include a variation at amino acid residue 45 of human Klotho isoform 1. At position 45, the residue can be a valine (Val; V), a phenylalanine (Phe; F), or another amino acid.

The Klotho polypeptide includes a signal peptide or signal sequence. For example, the protein can include a native Klotho signal sequence. The protein can include a non-native signal sequence. In some embodiments, the signal sequence can be an N-terminal signal sequence and/or upstream of (or N- terminal to) a Klotho protein sequence. In other embodiments, the signal sequence can be C-terminal or otherwise disposed. Preferably, the signal sequence is the SP of human Klotho as set forth in SEQ ID NO: 51.

Substituting a Phenylalanine in the place of the Valine at (native) position 45 of human Klotho (V45F) has been reported to have benefits. For example, it has been reported in WO 2019/113373 A2 that V45F soluble Klotho, and fragments or fusion proteins thereof, express at higher levels in CHO and HEK-293 cells than does the native, wild type V45 protein. Accordingly, some embodiments of the present disclosure include a Klotho polypeptide having the V45F substitution.

The mRNA used in the present invention contains (at least) five essential elements which are all known and available to a person skilled in the art (in this order from 5' to 3'): a 5' CAP region, a 5' untranslated region (5'-UTR), a coding region, a 3' untranslated region (3'-UTR) and a poly-adenosine tail (poly-A tail).

The coding region should, of course encode a (human) Klotho polypeptide, the other components may be the (native) Klotho UTRs or, preferably, other UTRs. Specifically preferred UTRs according to the present invention are UTRs which improve the properties of the mRNA molecule according to the present invention, i.e. by effecting better and/or longer and/or more effective translation of the mRNA into the Klotho polypeptide at the site of administration.

A "CAP region" ("5'CAP") refers to a structure found on the 5' end of an mRNA molecule and generally consists of a guanosine nucleotide connected to the mRNA via an unusual 5' to 5' triphosphate linkage. This guanosine nucleotide is methylated on the 7-position directly after capping in vivo by a methyl transferase ("7-methylguanylate cap" ("m7G"), "cap-0"). Further modifications include the possible methylation of the 2' hydroxy-groups of the first two ribose sugars of the 5' end of the mRNA (i.e. "CAP1" and "CAP2"): "CAP1" has a methylated 2'-hydroxy group on the first ribose sugar, while "CAP2" has methylated 2'-hydroxy groups on the first two ribose sugars. The 5' cap is chemically similar to the 3' end of an RNA molecule (the 5' carbon of the cap ribose is bonded, and the 3' unbonded). This provides significant resistance to 5' exonucleases and is therefore also providing stability in vivo. For generation of mRNAs according to the present invention also CAP analogues may be used including: monomethylated CAP analogue (mCAP), Anti-Reverse Cap Analog (ARCA CAP), m7G(5')ppp(5')A RNA CAP structure analog, G(5')ppp(5')A RNA CAP structure analog, and G(5')ppp(5')G RNA CAP structure analog.

The term "(5'- or 3'-) UTR" refers to the well-established concept of untranslated region of a mRNA in molecular genetics. There is one UTR on each side of a coding sequence on a strand of mRNA. The UTR on the 5' side, is the 5'-UTR (or leader sequence), the UTR on the 3' side, is the 3'-UTR (or trailer sequence). The 5'-UTR is upstream from the coding sequence. Within the 5'-UTR is a sequence that is recognized by the ribosome which allows the ribosome to bind and initiate translation. The mechanism of translation initiation differs in prokaryotes and eukaryotes. The 3'-UTR is found immediately following the translation stop codon. The 3'-UTR plays a critical role in translation termination as well as post-transcriptional gene expression. The UTRs as used in the present invention are usually delivering beneficial stability and expression (translation) properties to the mRNA molecules according to the present invention.

The "poly-A tail" consists of multiple adenosine monophosphates; it is a part of naturally occurring mRNA that has only adenine bases. This process called "polyadenylation" is part of the process that produces mature messenger RNA (mRNA) for translation in the course of gene expression. The natural process of polyadenylation begins as the transcription of a gene terminates. The 3'-most segment of the newly made pre-mRNA is first cleaved off by a set of proteins; these proteins then synthesize the poly(A) tail at the RNA's 3' end. In some genes, these proteins add a poly(A) tail at one of several possible sites. Therefore, polyadenylation can produce more than one transcript from a single gene (alternative polyadenylation), similar to alternative splicing. The poly(A) tail is important for the nuclear export, translation, and stability of mRNA. For the present invention, it is therefore mainly the translation and stability properties that are important for a sufficient polyadenylation of the mRNA molecules according to the present invention. During the protein generation, the tail is shortened over time, and, when it is short enough, the mRNA is enzymatically degraded. The poly-A tail according to the present invention is provided in the manner currently used and applied in the art of administering mRNA molecules in human therapy. For example, the poly-A tail may be at least 60 adenosine monophosphates long. According to a preferred embodiment, the poly-A tail is at least 100 adenosine monophosphates long, especially at least 120 adenosine monophosphates. This allows excellent stability and protein generation; however, as for the other features, the action and activity of the mRNA molecule according to the present invention can also be regulated by the poly-A tail feature.

As laid out in more detail above and as shown in the Examples herein, it has been surprisingly found in the context of the present invention that there is a direct relationship between the GC content of the Klotho mRNA and the expression level of the Klotho polypeptide. As used herein, the "GC content" of a certain mRNA or a certain part of a certain mRNA refers to the sum of G and C bases in relation to the total number of bases in said mRNA or in said part of the mRNA, expressed as a percentage. Thus, the GC content can be calculated as 100%* (C+G)/(A+C+G+U), wherein "C", "G", "A" and "U" refer to the number of C-, G-, A- and U-bases, respectively. In connection with the nucleoside variants disclosed herein below, it is important to note that the specific preferred variants described above do not influence the GC content, i.e. the variant is conservative in this respect (e.g. a cytidine variant still counts as a cytidine for the calculation of the GC content).

Therefore, in a preferred embodiment of the inventive Klotho mRNA, the coding region encoding the Klotho polypeptide has a GC content of at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63. The coding region encoding the Klotho polypeptide has a GC content of between 54 % and 64 %, more preferred between 55 % and 64 %, more preferred between 56 % and 64 %, more preferred between 57 % and 64 %, more preferred between 58 % and 64 %, more preferred between 59 % and 64 %, more preferred between 60 % and 64 %, more preferred between 61 % and 63 %.

In a preferred embodiment, the Klotho mRNA has a GC content of at least 50 %, preferably at least 51 %, more preferred at least 52 %, more preferred at least 53 %, more preferred at least 54 %, more preferred at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63. It is further preferred if the Klotho mRNA has a GC content of between 50 % and 74 %, preferably between 51 % and 73 %, more preferred between 52 % and 72 %, more preferred between 53 % and 71 %, more preferred between 54 % and 70 %, more preferred between 55 % and 69 %, more preferred between 56 % and 68 %, more preferred between 57 % and 67 %, more preferred between 58 % and 66 %, more preferred between 59 % and 65 %, more preferred between 60 % and 64 %, more preferred between 61 % and 63 %.

As laid out in more detail above, it found in the course of the present invention that the closer the Klotho mRNA is in sequence identity to the "GC62" variant created in the course of the present invention, the higher the expression level was. The sequence of the GC62 variant of the KL1 domain of native human Klotho according to SEQ ID NO: 52 is set forth in SEQ ID NO: 1. Therefore, in a preferred embodiment, the coding region encoding the Klotho polypeptide comprises an RNA sequence having at least 80 % sequence identity to SEQ ID NO: 1. Preferably, the sequence identity is at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %. Preferably the RNA sequence is SEQ ID NO: 1 or 3, especially SEQ ID NO: 1.

In preferred embodiments, the coding region encoding the Klotho polypeptide comprises SEQ ID NO: 1, 2, 3 or 4, preferably SEQ ID NO: 1.

SEQ ID NOs: 1 and 2 code for the KL1 domain of native human Klotho according to SEQ ID NO: 52, whereas SEQ ID NOs: 3 and 4 code for the KL1 domain of the VS variant described above according to SEQ ID NO: 53.

The sequence of the GC62 variant of the domains KL1-lr-KL2 of native human Klotho isoform 1 (amino acids L56 to G952 of SEQ ID NO: 49) is set forth in SEQ ID NO: 9. Therefore, in a further preferred embodiment, the coding region encoding the Klotho polypeptide comprises an RNA sequence having at least 80 % sequence identity to SEQ ID NO: 9, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 9 or 11, especially SEQ ID NO: 9.

In a preferred embodiment, the coding region encoding the Klotho polypeptide comprises SEQ ID NO: 9, 10, 11 or 12, preferably SEQ ID NO: 9.

The sequence of the GC62 variant of the domains lr-KL1-lr-KL2-lr of native human Klotho isoform 1 (amino acids E34 to S981 of SEQ ID NO: 49) is set forth in SEQ ID NO: 13. This sequence codes for the full sequence of soluble Klotho isoform 1 except for the N-Terminal SP. It should be noted that in the experiments referred to herein above and described in more detail in the Examples and Figures below, beneficial effects of increasing GC content and sequence identity to the "GC62" variant (SEQ ID NO: 18) were observed independently from the sequence of the signal peptide used. In fact, in the comparative tests comparing the four variants obtained from commercial codon optimization algorithms (GENEius algorithm, IDT Codon Optimization Tool, ExpOptimizer, Twist Codon Optimization Tool) as well as the variants "GC 53", "GC55", "GC57", "GC60" and "GC62", the same SP sequence was used and only the remaining part of the mRNA construct (corresponding to the domains lr-KL1-lr-KL2-lr; nucleotides 100-2946) were optimized. Thus, the advantageous effects of the high sequence similarity to the "GC62" variant were independent from the specific SP sequence used. Therefore, in a further preferred embodiment the coding region encoding the Klotho polypeptide comprises an RNA sequence having at least 80 % sequence identity to SEQ ID NO: 13, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 13 or 15, especially SEQ ID NO: 13.

In preferred embodiments, the coding region encoding the Klotho polypeptide comprises SEQ ID NO: 13, 14, 15 or 16, preferably SEQ ID NO: 13.

The sequence of the GC62 variant of the domains lr-KL1-lr-KL2-lr-TM-CT of native human Klotho isoform 1 (amino acids E34 to K1012 of SEQ ID NO: 49) is set forth in SEQ ID NO: 64. This sequence codes for the full sequence of Klotho isoform 1 except for the N-terminal SP but including the transmembrane domain and the cytoplasmic tail. This is particularly advantageous since, by combining it with any suitable signal peptide (native or otherwise), it allows delivering a membrane-anchored version of the Klotho protein, which makes it possible to exploit the full breadth of Klotho signalling rather than just the soluble part of it. Therefore, in a further preferred embodiment the coding region encoding the Klotho polypeptide comprises an RNA sequence having at least 80 % sequence identity to SEQ ID NO: 64, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 64 or 66, especially SEQ ID NO: 64.

In preferred embodiments, the coding region encoding the Klotho polypeptide comprises SEQ ID NO: 64, 65, 66 or 67, preferably SEQ ID NO: 64.

The sequence of the GC62 variant of the full soluble native human Klotho isoform 1 (amino acids M1 to S981 of SEQ ID NO: 49; corresponding to SEQ ID NO: 57) is set forth in SEQ ID NO: 18. This sequence was also used in the context of the comparative experiments referred to above and described in more detail below in the Examples and Figures. Therefore, the coding region encoding the Klotho polypeptide comprises an RNA sequence having at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 18 or 20, especially SEQ ID NO: 18.

In further preferred embodiments, the coding region encoding the Klotho polypeptide comprises SEQ ID NO: 18, 19, 20 or 21, preferably SEQ ID NO: 18.

The sequence of the GC62 variant of the full native human Klotho isoform 1 (coding for the full amino acid sequence of SEQ ID NO: 49) is set forth in SEQ ID NO: 68. As explained above with respect to SEQ ID NO: 64, this sequence also has the advantage that it allows delivering a membrane-anchored version of the Klotho protein, which makes it possible to exploit the full breadth of Klotho signalling rather than just the soluble part of it. Therefore, in a further preferred embodiment the coding region encoding the Klotho polypeptide comprises an RNA sequence having at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 68 or 70, especially SEQ ID NO: 68.

In preferred embodiments, the coding region encoding the Klotho polypeptide comprises SEQ ID NO: 68, 69, 70 or 71, preferably SEQ ID NO: 64.

The sequence of the GC62 variant of KL1-end domains of the native human Klotho isoform 2 (amino acids L56 to H549 of SEQ ID NO: 50) is set forth in SEQ ID NO: 33. Therefore, in a preferred embodiment, the coding region encoding the Klotho polypeptide comprises an RNA sequence having at least 80 % sequence identity to SEQ ID NO: 33, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 33 or 35, especially SEQ ID NO: 33.

In further preferred embodiments, the coding region encoding the Klotho polypeptide comprises SEQ ID NO: 33, 34, 35 or 36, preferably SEQ ID NO: 33.

The sequence of the GC62 variant of the lr-KL1-end domains of the native human Klotho isoform 2 (amino acids E34 to H549 of SEQ ID NO: 50) is set forth in SEQ ID NO: 33. This sequence codes for the full sequence of soluble Klotho isoform 2 except for the N-Terminal SP. Therefore, in a further preferred embodiment, the coding region encoding the Klotho polypeptide comprises an RNA sequence having at least 80 % sequence identity to SEQ ID NO: 37, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 37 or 39, especially SEQ ID NO: 37.

In further preferred embodiments, the coding region encoding the Klotho polypeptide comprises SEQ ID NO: 37, 38, 39 or 40, preferably SEQ ID NO: 37.

The sequence of the GC62 variant of the full soluble native human Klotho isoform 2 (amino acid sequence SEQ ID NO: 50) is set forth in SEQ ID NO: 41. Therefore, in a further preferred embodiment the coding region encoding the Klotho polypeptide comprises an RNA sequence having at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 41 or 43, especially SEQ ID NO: 41.

In preferred embodiments, the coding region encoding the Klotho polypeptide comprises SEQ ID NO: 41, 42, 43 or 44, preferably SEQ ID NO: 41.

In a particularly preferred embodiment, the coding region encoding the Klotho polypeptide has at least 80 % sequence identity to SEQ ID NO: 5, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 5, 6, 7, or 8.

In a further particularly preferred embodiment, the coding region encoding the Klotho polypeptide has at least 80 % sequence identity to SEQ ID NO: 18, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 18, 19, 20, or 21. In further embodiments, the Klotho polypeptide is SEQ ID NO: 22, 23, or 24.

In a further particularly preferred embodiment, the coding region encoding the Klotho polypeptide has at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 68, 69, 70, or 71.

In a further particularly preferred embodiment, the coding region encoding the Klotho polypeptide has at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 41, 42, 43, or 44.

As laid out herein above, it is preferred in the context of the invention if the Klotho polypeptide comprises the signal peptide, the KL1 domain and/or the KL2 domain of human Klotho.

In a preferred embodiment, the KL1 domain of the Klotho polypeptide has at least 80 % sequence identity to SEQ ID NO: 52, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, preferably wherein the KL1 domain of the Klotho polypeptide is SEQ ID NO: 52 or 53, especially SEQ ID NO: 52.

In a further preferred embodiment, the KL2 domain of the Klotho polypeptide has at least 80 % sequence identity to SEQ ID NO: 54, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the KL2 domain of the Klotho polypeptide is SEQ ID NO: 54.

In yet a further preferred embodiment, the signal peptide of the Klotho polypeptide has at least 80 % sequence identity to SEQ ID NO: 51, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the signal peptide of the Klotho polypeptide is SEQ ID NO: 51.

It is particularly preferred when the above-described embodiments relating to the SP, the KL1 domain and the KL2 domain are combined. For instance, it is preferred when the Klotho polypeptide comprises a KL1 domain of the Klotho polypeptide having at least 80 % sequence identity to SEQ ID NO: 52, a KL2 domain of the Klotho polypeptide having at least 80 % sequence identity to SEQ ID NO: 54, and/or a signal peptide of the Klotho polypeptide having at least 80 % sequence identity to SEQ ID NO: 51.

In a further preferred embodiment, the Klotho polypeptide has at least 90 % sequence identity to SEQ ID NO: 55, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 55 or 56, preferably 55.

In a further preferred embodiment, the Klotho polypeptide has at least 90 % sequence identity to SEQ ID NO: 57, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 57 or 58, preferably 57.

In a further preferred embodiment, the Klotho polypeptide has at least 90 % sequence identity to SEQ ID NO: 49, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 49 or 76, preferably 49.

In yet a further preferred embodiment, the Klotho polypeptide has at least 90 % sequence identity to SEQ ID NO: 59, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 59 or 60, preferably 59.

In the context of the present invention, several specific sequences for the 5'-UTR and the 3'-UTR were found to be particularly advantageous, especially for achieving high expression levels.

Specifically, for the 5'-UTR, the sequence of SEQ ID NO: 61 was found to be advantageous. Therefore, in a preferred embodiment of the Klotho mRNA, the 5'-UTR has at least 70 % sequence identity to SEQ ID NO: 61, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %. It is especially preferred if the 5'-UTR is SEQ ID NO: 61.

Similarly, for the 3'-UTR it was found that the sequence of SEQ ID NO: 62 is particularly advantageous. Therefore, in a preferred embodiment, the 3'-UTR has at least 70 % sequence identity to SEQ ID NO: 62, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %. It is especially preferred if the 3'-UTR is SEQ ID NO: 62.

According to a preferred embodiment of the present invention, the present mRNA comprises in the 5'-UTR and/or 3'-UTR (preferably in the 3'UTR) one or more stabilization sequences that are capable of increasing the half-life of the mRNA intracellularly. These stabilization sequences may exhibit a 100% sequence homology with naturally occurring sequences that are present in viruses, bacteria and eukaryotic cells, but may however also be partly or completely synthetic. Examples for such stabilizing sequences are described in: Nucleic Acids Res. 2010; 38 (Database issue): D75-D80. UTRdb and UTRsite (RELEASE 2010): a collection of sequences and regulatory motifs of the untranslated regions of eukaryotic mRNAs and under http://utrdb.ba.itb.cnr.it/.

In an embodiment of the Klotho mRNA the 5'-UTR or 3'-UTR or the 5'-UTR and the 3'-UTR correspond to native human Klotho mRNA, preferably native human Klotho mRNA as set forth in NCBI Reference Sequence: NM_004795.4. Alternative, in another preferred embodiment, the 5'-UTR or 3'-UTR or the 5'-UTR and the 3'-UTR are different from native human Klotho mRNA.

As a further example of stabilizing sequences that may be used in the present invention, the non-translated sequences (UTR) of the β-globin gene, for example of Homo sapiens or Xenopus laevis, may be mentioned.

Another example of a stabilization sequence has been described in Holcik et al. (Proc. Natl. Acad. Sci. USA 1997, 94: 2410 to 2414) and has the general formula:
(C/U) CCANₓCCC(U/A)PyₓUC(C/U)CC (SEQ ID NO: 63),
which is contained in the 3'UTR of the very stable mRNAs that code for example for alpha(1)-collagen or for alpha globin, and ALOX15 or for tyrosine hydroxylase (and wherein "x" is (independently in Nₓ and Pyₓ) an integer of 0 to 10, preferably of 0 to 5 (Holcik et al., 1997), especially 0, 1, 2, 4 and/or 5).

Such stabilization sequences may be used individually or in combination with one another for stabilizing the inventive mRNA as well as in combination with other stabilization sequences known to the person skilled in the art. E.g.: The stabilizing effect of human β-globin 3'-UTR sequences is further augmented by using two human β-globin 3'-UTRs arranged in a head-to-tail orientation.

Accordingly, a preferred embodiment of the Klotho mRNA according to the present invention is an mRNA molecule, wherein the 5'-UTR or 3'-UTR or the 5'-UTR and the 3'-UTR are different from the native Klotho mRNA, preferably wherein the 5'-UTR or 3'-UTR or the 5'-UTR and the 3'-UTR contain at least one stabilization sequence, preferably a stabilization sequence with the general formula (C/U)CCANₓCCC(U/A)PyₓUC(C/U)CC (SEQ ID NO:38).

Preferably, the 5'-UTR and/or 3'-UTR are the 5'-UTR and/or 3'-UTR of a different human mRNA than Klotho, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

Accordingly, the present invention preferably relates to an mRNA which comprises in the 3'-UTR one or more stabilization sequences that are capable of increasing the half-life of the mRNA in the cytosol. These stabilization sequences may exhibit a 100% sequence homology with naturally occurring sequences that are present in viruses, bacteria and eukaryotic cells, but may, however, also be partly or completely synthetic. As an example of stabilizing sequences that may be used in the present invention, the non-translated sequences (UTR) of the β-globin gene, for example of homo sapiens or xenopus laevis, may be mentioned. As already stated, another example of a stabilization sequence has the general formula (C/U)CCANₓCCC(U/A)PyₓUC(C/U)CC, which is contained in the 3'-UTR of the very stable mRNA that codes for alpha-globin, alpha-(1)-collagen, 15-lipoxygenase or for tyrosine hydroxylase (c.f. Holcik et al., Proc. Natl. Acad. Sci. USA 1997, 94: 2410 to 2414). Such stabilization sequences may be used individually or in combination with one another for stabilizing the inventive modified mRNA as well as in combination with other stabilization sequences known to the person skilled in the art.

Another preferred embodiment of the present invention is the 5'-TOP-UTR derived from the ribosomal protein 32L, followed by a stabilizing sequence derived from the albumin-3'-UTR.

Accordingly, a preferred embodiment of the Klotho mRNA according to the present invention is an mRNA molecule containing a tract of multiple adenosine monophosphates at the 3' end of the 3'-UTR. This so-called poly-adenosine (poly-A) tail consists of at least 60 adenosine monophosphates, preferably at least 100 and most preferably at least 120 adenosine monophosphates.

In certain cases, destabilizing the mRNA might be desirable as well to limit the duration of protein production. This effect can be achieved by incorporating destabilizing sequence elements (DSE) like AU-rich elements into 3'-UTRs, thus ensuring rapid mRNA degradation and a short duration of protein expression.

Although it may be desired for certain embodiments to provide an mRNA which is devoid of destabilizing sequence elements (DSE) in the 3' and/or 5' UTR, there may be other embodiments, wherein the presence or introduction of such DSEs is advantageous. In general, a "DSE" refers to a sequence, which reduces the half-life of a transcript, e.g. the half-life of the mRNA according to the present invention inside a cell and/or organism, e.g. a human patient. Accordingly, a DSE comprises a sequence of nucleotides, which reduces the intracellular half-life of an RNA transcript.

DSE sequences are found in short-lived mRNAs such as, for example: c-fos, c-jun, c-myc, GM-CSF, IL-3, TNF-alpha, IL-2, IL-6, IL-8, IL-10, Urokinase, bcl-2, SGL T1 (Na(+)-coupled glucose transporter), Cox-2 (cyclooxygenase 2), PAI-2 (plasminogen activator inhibitor type 2), beta(1)-adrenergic receptor or GAP43 (5'-UTR and 3'-UTR).

Further DSEs are AU-rich elements (AREs) and/or U-rich elements (UREs), including single, tandem or multiple or overlapping copies of the nonamer UUAUUUA(U/A) (U/A) (where U/A is either an A or a U) and/or the pentamer AUUUA and/or the tetramer AUUU. Further DSEs are described in Nucleic Acids Res. 2010; 38 (Database issue) : D75-D80. UTRdb and UTRsite (RELEASE 2010): a collection of sequences and regulatory motifs of the untranslated regions of eukaryotic mRNAs and under http://utrdb.ba.itb.cnr.it/.

Accordingly, it may also be preferred if the 5'-UTR or 3'-UTR or the 5'-UTR and the 3'-UTR contain at least one destabilization sequence element (DSE), preferably AU-rich elements (AREs) and/or U-rich elements (UREs), especially a single, tandem or multiple or overlapping copies of the nonamer UUAUUUA(U/A) (U/A), such as the pentamer AUUUA and/or the tetramer AUUU (the term "U/A" meaning either A or U).

These stabilizing and destabilizing elements can be used alone or in combination to aim at a given duration of protein production and to individualize the treatment of the present invention to the local skin hypotrophy conditions, especially atrophic skin condition, the severity of affected skin and/or the specific group of patients.

General concepts for improved mRNA-based therapeutics (see e.g. Sahin et al., Nat. Rev. Drug Disc. 2014. 13(10): 759-780) are also applicable for the present invention.

Although in most cases, the use of exclusively canonical nucleotides may be preferred, there may be certain occasions where the Klotho mRNA according to the present invention may contain other monophosphate nucleosides than those of cytidine (C), uridine (U), adenosine (A) or guanosine (G) residues (the canonical nucleotides). There are a significant number of naturally occurring analogs of these monophosphate nucleosides and also (even more) synthetic variants of these mRNA residues. Embodiments of such variants can be found e.g. in WO 2014/153052 A2.

According to an embodiment, in the present Klotho mRNA, at least 5%, preferably at least 10%, more preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

Specific embodiments are Klotho mRNAs, wherein in the Klotho mRNA, at least 5%, preferably at least 10%, more preferably at least 30%, especially at least 50% of all
- cytidine residues are replaced by 5-methyl-cytidine residues, and/or
- uridine residues are replaced by pseudo-uridine residues, and/or
- uridine residues are replaced by 2-thio-uridine residues.

In a further preferred embodiment of the Klotho mRNA, the coding region has a codon adaption index (CAI) of at least 0.70, preferably at least 0.75, more preferred at least 0.80, more preferred at least 0.85, more preferred at least 0.86, more preferred at least 0.87, more preferred at least 0.88, more preferred at least 0.89, more preferred at least 0.90, more preferred at least 0.91, more preferred at least 0.92, more preferred at least 0.93, more preferred at least 0.94, more preferred at least 0.95, more preferred at least 0.96, more preferred at least 0.97, more preferred at least 0.98, more preferred at least 0.99, more preferred 1.00. However, surprisingly, also a lower CAI can be beneficial to improve expression levels. Therefore, it is preferred if the coding region has a codon adaption index (CAI) of below 1.00, preferably below 0.99, more preferred below 0.98, more preferred below 0.97, more preferred below 0.96, more preferred below 0.95.

The CAI is a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (Sharp et al., Nucleic Acids Res. 15 (1987): 1281-1295., Jansen et al., Nucleic Acids Res. 31 (2003): 2242-2251).

In a particularly preferred embodiment of the Klotho mRNA, the Klotho mRNA has at least 80 % sequence identity to SEQ ID NO: 29, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 29, 30, 31 or 32, preferably SEQ ID NO: 29.

In a further particularly preferred embodiment of the Klotho mRNA, the Klotho mRNA has at least 80 % sequence identity to SEQ ID NO: 72, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 72, 73, 74 or 75, preferably SEQ ID NO: 72.

In a further particularly preferred embodiment of the Klotho mRNA, the Klotho mRNA has at least 80 % sequence identity to SEQ ID NO: 45, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 45, 46, 47 or 48, preferably SEQ ID NO: 45.

In the course of the present invention, it was found that slight modifications to the above-described sequences could make these sequences even more useful and easier to handle. More specifically, it was found that by exchanging a small number of nucleotides, recognition sequences of restriction enzymes could be removed, making these sequences easier to handle for molecular cloning. Thus, based on the above-mentioned sequences SEQ ID NOs: 1-21, 29-48 and 64-75, modified sequences containing up to five modified bases were prepared to remove certain restriction sites. These modifications did not have any significant impact on the expression levels achieved with the constructs. The modified sequences and the corresponding sequence on which they were based are shown in the following table:

| **original SEQ ID NO:** | **modified SEQ ID NO:** |
|---|---|
| 1 | 79 |
| 2 | 80 |
| 3 | 81 |
| 4 | 82 |
| 5 | 83 |
| 6 | 84 |
| 7 | 85 |
| 8 | 86 |
| 9 | 87 |
| 10 | 88 |
| 11 | 89 |
| 12 | 90 |
| 13 | 91 |
| 14 | 92 |
| 15 | 93 |
| 16 | 94 |
| 17 | 95 |
| 18 | 96 |
| 19 | 97 |
| 20 | 98 |
| 21 | 99 |
| 29 | 100 |
| 30 | 101 |
| 31 | 102 |
| 32 | 103 |
| 33 | 104 |
| 34 | 105 |
| 35 | 106 |
| 36 | 107 |
| 37 | 108 |
| 38 | 109 |
| 39 | 110 |
| 40 | 111 |
| 41 | 112 |
| 42 | 113 |
| 43 | 114 |
| 44 | 115 |
| 45 | 116 |
| 46 | 117 |
| 47 | 118 |
| 48 | 119 |
| 64 | 120 |
| 65 | 121 |
| 66 | 122 |
| 67 | 123 |
| 68 | 124 |
| 69 | 125 |
| 70 | 126 |
| 71 | 127 |
| 72 | 128 |
| 73 | 129 |
| 74 | 130 |
| 75 | 131 |
| | |

An overview of these sequences is also provided in Table 3B below.

All embodiments described herein that involve the original SEQ ID NOs: 1-21, 29-48 and 64-75 are also preferred with respect to the modified sequences 79-131. Thus, for instance, if it is specified herein that the coding region encoding the Klotho polypeptide comprises an RNA sequence having at least 80 % sequence identity to SEQ ID NO: 1, it would be equally preferred that the coding region encoding the Klotho polypeptide comprises an RNA sequence having at least 80 % sequence identity to SEQ ID NO: 79. Similarly, if it is specified that the coding region encoding the Klotho polypeptide comprises a SEQ ID NO: 13, it would be equally preferred that said coding region comprises SEQ ID NO: 91.

In another aspect, the present invention relates to a pharmaceutical formulation comprising the Klotho mRNA according to any one of the preceding Embodiments.

Suitable formulations for mRNA therapeutics are well available in the art (see e.g. Sahin et al., 2014; WO 2014/153052 A2 (paragraphs 122 to 136), etc.).

The present invention therefore also relates to a pharmaceutical formulation comprising a Klotho mRNA according to the present invention. The present formulation preferably comprises the mRNA in a pharmaceutically acceptable environment, e.g. with suitable components usually provided in mRNA therapeutics (excipients, carriers, buffers, auxiliary substances (e.g. stabilizers), etc.)

The mRNA formulations according to the present invention preferably contain a suitable carrier. Suitable carriers include polymer based carriers, such as cationic polymers including linear and branched PEI and viromers, lipid nanoparticles and liposomes, nanoliposomes, ceramide-containing nanoliposomes, proteolipo-somes, cationic amphiphilic lipids e.g: SAINT^{®}-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, small-molecule targeted conjugates, poly-lactic-co-glycolic acid (PLGA) polymers and other vectorial tags. Also contemplated is the use of bionanocapsules and other viral capsid protein assemblies as a suitable carrier. (Hum. Gene Ther. 2008 Sep;19(9):887-95).

Preferred carriers are cationic polymers including linear and branched PEI and viromers, lipid nanoparticles and liposomes, transfersomes, and nanoparticulates including calcium phosphate nanoparticulates (i.e. naked RNA precipitated with CaCl₂ and then administered) .

A preferred embodiment of the present invention relates to the use of non-complexed mRNA, i.e. non-complexed mRNA in a suitable aqueous buffer solution, preferably a physiological glucose buffered aqueous solution (physiological). For example, a 1xHEPES buffered solution; a 1xPhosphate buffered solution, Na-Citrate buffered solution; Na-Acetate buffered solution; Ringer's Lactate solution; preferred with Glucose (e.g.: 5% Glucose); physiologic solutions can be preferably applied.

Preferably the present invention applies liposomes, especially liposomes which are based on DOTAP, DOTMA, Dotap-DOPE, DOTAP-DSPE, Dotap-DSPE-PEG, Dotap-DOPE-PEG, Dotap-DSPE-PEG-Na-Cholate, Dotap-DOPE-PEG-Na-Cholate, DOTAP with cationic amphiphilic macromolecules (CAM) as complexes, and combinations thereof.

In a preferred embodiment of the inventive pharmaceutical formulation, the formulation comprises a further mRNA, wherein the further mRNA has a further 5' CAP region, a further 5'-UTR, a further coding region encoding a further polypeptide, a further 3'-UTR and a further poly-A tail.

The further polypeptide preferably causes further beneficial effects when expressed in the cells of an individual. It is particularly preferred if the further polypeptide has an additive, or, preferably, a synergistic effect with the Klotho polypeptide. Preferably, the further polypeptide is an anti-aging peptide or an anti-aging protein, i.e., any peptide or protein known to the skilled person to have an anti-aging effect. Alternatively, or in addition, it is preferred if the further polypeptide has a therapeutic effect, preferably, a therapeutic effect in relation to any of the therapeutic indications described herein.

In a preferred embodiment, the further polypeptide is Bactericidal/Permeability Increasing Protein Family B, member 4 (BPIFB4) protein or an isoform or variant thereof, preferably Longevity-Associated Variant of BPIFB4 (LAV-BPIFB4). Preferably, BPIFB4 is as set forth in UniProt Entry P59827-1 or P59827-2, especially P59827-1 (Sequence version 2). Preferably, the LAV-variant is as set forth in UniProt Entry P59827-1 or P59827-2, especially P59827-1 (Sequence version 2), wherein the following amino acids are substituted: Ile229Val, Asn281Thr, Leu488Phe and Ile494Thr.

In a preferred embodiment, the BPIFB4 has at least one, preferably at least two, preferably at least three mutations selected from Ile229Val, Asn281Thr, Leu488Phe and Ile494Thr; preferably the BPIFB4 has all of the named mutations.

In further preferred embodiments, the further polypeptide is a BPIFB4 protein or an isoform or variant thereof as set forth in WO 2014/102343 A1 or WO 2019/034723 A1**.**

In further preferred embodiments, the further polypeptide is a protein described in Bin-Jumah et al. "Genes and longevity of lifespan." International Journal of Molecular Sciences 23.3 (2022): 1499. In particular, it is preferred if the further polypeptide is a protein selected from Apolipoprotein E (preferably gene symbol: APOE), Tumor protein p53 (preferably gene symbol: P53), Sirtuin 1 protein (preferably gene symbol: SIRT1), FOXO1 transcription factor (preferably gene symbol: DAF-16), Cholinergic receptor nicotinic alpha 3 subunit (preferably gene symbol: CHRNA3), SH2B adaptor protein 3 (preferably gene symbol: SH2B3), Cyclin dependent kinase inhibitor 2A (preferably gene symbol: CDKN2A), Elongation of very-long-chain fatty acids-like 2 (preferably gene symbol: ELOVL2), Werner protein (preferably gene symbol: WRN), Paraoxonase 1 (preferably gene symbol: PON1), Superoxide dismutase 2 (preferably gene symbol: SOD2), Lamin A protein (preferably gene symbol: LMNA), Cholesteryl ester transfer protein (preferably gene symbol: CETP), Apolipoprotein C3 (preferably gene symbol: APOC3), Microsomal triglyceride transfer protein (preferably gene symbol: MTP), Phosphatidylinositol 3-kinase (PI3K) (preferably gene symbol: PIK3CA), Insulin-like growth factor 1 (IGF-1) receptor (preferably gene symbol: DAF-2), Protein-L-iso-aspartyl methyltransferase (preferably gene symbol: PIMT), Growth hormone (preferably gene symbol: GH1), cAMP-response element binding protein (preferably gene symbol: CREB), Mitogen-activated protein kinase (preferably gene symbol: MAPK), epidermal growth factor receptor (preferably gene symbol: EGFR), Nuclear factor kappa B (preferably gene symbol: NF-kB), Phospholipase C beta (preferably gene symbol: PLC-β), Methionine sulfoxide reductase A (preferably gene symbol: MSR-A), Mediator of cell motility 1 (preferably gene symbol: MEMO1), Nei like DNA Glycosylase 1 (preferably gene symbol: NEILl), Peroxisome proliferator-activated receptor gamma 2 (preferably gene symbol: PPARγ2), Eukaryotic translation initiation factor 3 subunit K (preferably gene symbol: EIF3K), ATM serine/threonine kinase (preferably gene symbol: ATM), B-cell lymphoma 2 (preferably gene symbol: BCL2), Cell division cycle 42 (preferably gene symbol: CDC42), Diacylglycerol O -acyltransferase 1 (preferably gene symbol: DGAT1), Early growth response 1 (preferably gene symbol: EGR1), Fibroblast growth factor 23 (preferably gene symbol: FGF23), Fibroblast growth factor 21 (preferably gene symbol: FGF21), Fructosamine 3 kinase related protein (preferably gene symbol: FN3KRP), Phosphoglycolate phosphatase (preferably gene symbol: PGP), Insulin receptor substrate 1 (preferably gene symbol: IRS1), Polycomb complex protein BMI-1 (preferably gene symbol: BMI1), Neuregulin 1 (preferably gene symbol: NRG-1), Signal transducer and activator of transcription (preferably gene symbol: STAT), E2F Transcription Factor 1 (preferably gene symbol: E2F1), Vascular endothelial growth factor A (preferably gene symbol: VEGF-A), Xenobiotic metabolizing enzymes (preferably gene symbol: XME), Myc proto-oncogene protein (preferably gene symbol: MYC), C-X-C chemokine receptor type 4 (preferably gene symbol: CXCR4), Silent information regulator 2 (preferably gene symbol: SIR-2), Extracellular signal-regulated kinase (preferably gene symbol: ERK), SLC31 (preferably gene symbol: SLC31). For each protein, the native human version is preferred. All isoforms and/or variants of these proteins are included. When multiple isoforms or variants exist, the isoform or variant that is most common in the human population is preferred.

According to another aspect, the present invention relates to a kit for administering the Klotho mRNA according to any one of the preceding Embodiments to an individual comprising
- the Klotho mRNA according to any one of the preceding Embodiments, and
- a device for administering the Klotho mRNA.

Preferably the individual is an animal, preferably a human or a non-human animal. It is particularly preferred if the individual is a mammal, especially a human.

In a preferred embodiment of the inventive kit, the device is for intravenous, intramuscular, subcutaneous, intradermal, transdermal, epidermal, or topical administration.

Preferably, the device for administering the Klotho mRNA is a syringe, a needle, an autoinjector, and/or a needle-free injection system; preferably wherein the device is for intravenous and/or intramuscular injection. It is especially preferred if the device for administering the Klotho mRNA is a skin delivery device. In preferred embodiments, the skin delivery device is an intradermal delivery device, preferably selected from the group consisting of needle based injection systems. For instance, the skin delivery device can be a transdermal delivery device, preferably selected from the group consisting of transdermal patches, hollow and solid microneedle systems, microstructured transdermal systems, electrophoresis systems, and iontophoresis systems. The skin delivery device may also be an epidermal delivery device, preferably selected from the group consisting of needle free injection systems, laser based systems, especially Erbium YAG laser systems, and gene gun systems.

These administration devices are in principle available in the art; adaption to the administration of the mRNA according to the present invention is easily possible for a person skilled in the art.

A further aspect of the present invention relates to the inventive Klotho mRNA for use as a medicament.

Therapeutic uses of Klotho are known in the art. The present invention provides a particularly advantageous way of delivering these therapeutic effects to individuals in need thereof. Therapeutic uses of Klotho are e.g. described in Prud'homme et al. "Pathobiology of the Klotho Antiaging Protein and Therapeutic Considerations." Frontiers in Aging 3 (2022); as well as in Sachdeva, et al. "Klotho and the treatment of human malignancies." Cancers 12.6 (2020): 1665.

Therefore, in an aspect, the invention relates to the inventive Klotho mRNA or the inventive pharmaceutical composition for use in the prevention or treatment of a disease selected from the group consisting of kidney diseases, cardiovascular diseases, brain diseases, lung diseases, bone diseases, and metabolic diseases.

In another aspect, the invention relates to the inventive Klotho mRNA or the inventive pharmaceutical composition for use in the prevention or treatment of a kidney disease, preferably in the prevention or treatment of a disease selected from the group consisting of chronic kidney disease, fibrosis, hyperphosphatemia, ischemic injury, nephrectomy, toxic injury, diabetic nephropathy, and calciprotein deposition.

In another aspect, the invention relates to the inventive Klotho mRNA or the inventive pharmaceutical composition for use in the prevention or treatment of a cardiovascular disease, preferably in the prevention or treatment of a disease selected from the group consisting of arterial/aortic calcification, atherosclerosis, cardiomyopathy, cardiac hypertrophy, hypertension, and myocardial ischemic injury/infarct.

In another aspect, the invention relates to the inventive Klotho mRNA or the inventive pharmaceutical composition for use in the prevention or treatment of a brain disease, preferably in the prevention or treatment of a disease selected from the group consisting of Alzheimer's disease, hippocampal neuronal loss, cognitive deficits, and frailty.

In another aspect, the invention relates to the inventive Klotho mRNA or the inventive pharmaceutical composition for use in the prevention or treatment of cancer, preferably in the prevention or treatment of a cancer selected from the group consisting of colorectal cancer, esophageal cancer, gastric cancer, pancreatic cancer, breast cancer, lung cancer, ovarian cancer, thyroid cancer, melanoma, renal cancer, and cervical cancer.

In another aspect, the invention relates to the inventive Klotho mRNA or the inventive pharmaceutical composition for use in the prevention or treatment of a lung disease, preferably in the prevention or treatment of pulmonary fibrosis or chronic obstructive pulmonary disease.

In another aspect, the invention relates to the inventive Klotho mRNA or the inventive pharmaceutical composition for use in the prevention or treatment of a bone disease, preferably in the prevention or treatment of osteoporosis or osteomalacia, especially osteomalacia from chronic kidney disease.

In another aspect, the invention relates to the inventive Klotho mRNA or the inventive pharmaceutical composition for use in the prevention or treatment of a metabolic disease, preferably in the prevention or treatment of a disease selected from the group consisting of diabetes, in particular type 1 and/or type 2 diabetes, pancreatic β-cell apoptosis, autoimmune disorders, and inflammation, especially insulitis.

In relation to all aspects described herein (both the therapeutic and the non-therapeutic uses described herein), it is preferred if the inventive Klotho mRNA or the inventive composition is administered intravenously, intramuscularly, subcutaneously, intradermally, transdermally, epidermally, or topically, especially epidermally.

Administration of the Klotho mRNA or of the pharmaceutic composition comprising the Klotho mRNA can be performed according to optimised expression aims, depending on the amount of mRNA applied, the stability of the mRNA molecule and the status of disease. For example, the Klotho mRNA / the pharmaceutical composition may be administered at least once, at least twice, at least twice within one month, preferably weekly. For example, if the Klotho mRNA / the pharmaceutical composition may be administered at least twice, at least twice within one month, preferably weekly doses applied may vary.

The amount of mRNA delivered per dose may also be made dependent on the stability of the molecule, etc.. Preferably the Klotho mRNA / the pharmaceutical composition is administered in an amount of 0.01µg to 100 mg per dose, preferably of 0.1µg to 10mg per dose, especially of 1µg to 1mg per dose.

The present invention also relates to a method for preventing or treating a disease in an individual in need thereof, comprising administering an effective amount of the inventive Klotho mRNA or of the inventive pharmaceutical composition to the individual. All features and preferred embodiments described herein with respect to the Klotho mRNA for use or the pharmaceutical composition for use are also preferred embodiments of the method for treatment, e.g., as relates to the disease to be prevented or treated, the dosing regime, the method of administration, etc.

In another aspect, the invention relates to the inventive Klotho mRNA or the inventive pharmaceutical composition for use in the prevention or treatment of an aging-related disorder and/or a symptom of aging. Preferably, the aging-related disorder or symptom of aging is selected from one or more of actinic keratosis, age-related macular degeneration (AMD), Alzheimer's disease, arthritis, atherosclerosis and cardiovascular disease, benign prostatic hyperplasia (BPH), bone atrophy, cachexia, cancer, cardiomyopathy, cataracts, chronic obstructive pulmonary disease (COPD), constipation, decrease in overall energy, decrease in visual acuity, delirium, dementia, depression, dermal atrophy (thinning of the skin), diminished peripheral vision, greater risk of heat stroke or hypothermia, hearing loss, hypertension, increased susceptibility to infection (including influenza and pneumonia), lentigines (aging spots), liver conditions (e.g ., nonalcoholic fatty liver disease, nonalcoholic steatohepatitis, and cirrhosis), memory loss, metabolic syndrome, muscle atrophy (e.g., Sarcopenia and myopenia), frailty, muscle repair or rejuvenation deficiency, muscular dystrophy, osteoarthritis, osteoporosis, periodontitis, photoaging, reduced metabolism (including increased risk for obesity), reduced reflexes and coordination including difficulty with balance, respiratory disease (including acute respiratory distress syndrome (ARDS) with or without acute lung injury (ALI) and pulmonary fibrosis (e.g. idiopathic pulmonary fibrosis)), rheumatoid arthritis, sarcopenic obesity, sexual dysfunction, shingles, type 2 diabetes, urologic changes (including incontinence), vaginal atrophy, whitening or graying of hair, prolonged/inefficient wound healing, wrinkling/sagging skin (including loss of skin elasticity), and xerosis cutis (skin dryness); or the disease includes asthma, deafness, or a viral infections and/or a symptom of the disease comprises sepsis.

In another aspect, the present invention also relates to the use of the Klotho mRNA or the pharmaceutical composition according to the invention for preventing and/or reducing a symptom of aging.

In yet another aspect, the invention relates to the use of the Klotho mRNA or the pharmaceutical composition according to the invention for increasing the lifespan in an individual, preferably in a mammal, preferably a human.

It is preferred, if the above uses are non-therapeutic uses, especially the use for increasing the lifespan. Therefore, it is preferred if the individual is a healthy individual. It is preferred if the individual does not suffer from any of the diseases described herein.

In yet another aspect, the present invention relates to a cosmetic formulation comprising the Klotho mRNA according to the invention. Preferably, the cosmetic formulation comprises a cosmetic carrier. Preferably, cosmetic carrier is an ointment, a gel, especially a hydrogel, a liposome, nano/microparticles, or an emulsion.

According to a further aspect, the present invention also relates to a cosmetic skin care method, comprising contacting a skin of an individual, preferably a human individual, with the Klotho mRNA or the cosmetic formulation according to the invention. Preferably, the skin is an ageing skin. It is especially preferred, if the cosmetic skin care method reduces any sign of aging, such as wrinkles.

In a further aspect, the present invention relates to a DNA molecule comprising a DNA sequence encoding the coding region of the inventive Klotho mRNA. This DNA molecule may be used to enable the production of the inventive mRNA, facilitating its synthesis through both in vitro transcription (IVT) processes and cellular transcription mechanisms following transfection into mammalian cells.

In a preferred embodiment, the DNA molecule comprises a DNA sequence encoding the 5'-UTR, the coding region, and the 3'-UTR of the inventive Klotho mRNA. It is particularly preferred, when the DNA molecule comprises a DNA sequence encoding the 5'-UTR, the coding region, the 3'-UTR and the poly-A tail of the inventive Klotho mRNA. Alternatively, instead of being encoded in the DNA sequence, the poly-A tail of the inventive Klotho mRNA may also be added post-transcriptionally by polyadenylation. Preferably, the DNA molecule comprises a DNA sequence encoding the inventive Klotho mRNA.

It is preferred, if the DNA molecule comprises a promoter operatively linked to said DNA sequence. In a preferred embodiment, said promoter is a promoter for in vitro transcription, preferably a T7 promoter. In a further preferred embodiment, said promoter is a promoter for mammalian expression, preferably selected from the group consisting of CMV, CAG, and/or EF1a promoters.

However, in the context of the invention, also DNA molecules lacking promoter sequences and comprising only a DNA sequence encoding the coding region of the inventive Klotho mRNA are useful. For instance, such DNA molecules may be used as a template for an IVT process, in which a linear DNA fragment is generated by PCR from said DNA molecule and said linear DNA fragment is described. Any features that are desired to be present on the linear DNA fragment, such as a T7 promoter, may be added during the PCR reaction.

A further aspect of the invention relates to a cell comprising the inventive DNA molecule. The inventive cell is particularly useful for producing the inventive Klotho mRNA. In a particularly preferred embodiment, the inventive cell may be used for producing extracellular vesicles (EVs), such as exosomes, comprising the inventive Klotho mRNA, as laid out in more detail below.

Preferably, the cell is a mammalian cell, preferably a human cell. In the context of the invention, it is particularly preferred if the cell is a Human Embryonic Kidney (HEK) 293 cell.

It is particularly preferred, when the cell is an EV-producing cell. Cell types that are particularly well suited for producing EVs, in particular mRNA-containing EVs, are known in the art, e.g. from WO 2019/092145 A1 and WO 2010/119256 A1. For instance, cell lines of particular interest include human umbilical cord endothelial cells (HUVECs), HEK cells, endothelial cell lines such as microvascular or lymphatic endothelial cells, erythrocytes, erythroid progenitors, chondrocytes, MSCs of different origin, amnion cells, amnion epithelial (AE) cells, any cells obtained through amniocentesis or from the placenta, airway or alveolar epithelial cells, fibroblasts, endothelial cells, etc. Also, immune cells such as B cells, T cells, NK cells, macrophages, monocytes, dendritic cells (DCs) can be used for producing EVs. Generally, EVs may be derived from essentially any cell source.

In a preferred embodiment, the cell is stably transfected with the inventive DNA molecule. Moreover, it is particularly preferred when the cells are exposed to a clonal selection protocol allowing for clonal selection of a single cell clone. This may e.g. be achieved using limiting dilution methods, single-cell sorting, and/or isolation of individual cells using cloning cylinders. Thus, in a preferred embodiment, the cell is a monoclonal cell and/or a monoclonal cell line.

In a particularly preferred embodiment, the cell is encapsulated in a biocompatible scaffold. Such biocompatible scaffolds may be used for the continuous delivery of therapeutic proteins.

Similarly, in a further aspect, the present invention relates to a therapeutic delivery device comprising the inventive cells, wherein the cells are encapsulated in a biocompatible scaffold.

Traditional delivery methods, such as systemic injection or infusion, often require frequent administration to maintain therapeutic levels due to the rapid clearance of proteins from the body. This not only burdens patients but also increases the risk of adverse effects and reduces the overall efficacy of the treatment. To address these challenges, cell encapsulation technology has gained more and more importance. Cell encapsulation typically involves immobilizing cells within biocompatible scaffolds such as microcapsules. These encapsulated cells can then be implanted into an individual, where they continuously produce and release the therapeutic protein over extended periods. This approach offers several advantages, including the potential for sustained and localized delivery of therapeutic proteins, reduced frequency of administration, and protection of the encapsulated cells from the host immune system.

Methods for encapsulating cells in biocompatible scaffolds and for employing such encapsulated cells for the continuous delivery of therapeutic proteins are known from the art. For instance, suitable methods are described in Acarregui et al. ("Therapeutic applications of encapsulated cells." Immobilization of Enzymes and Cells: Third Edition (2013): 349-364) and Wang et al. ("A nanofibrous encapsulation device for safe delivery of insulin-producing cells to treat type 1 diabetes." Science Translational Medicine 13.596 (2021): eabb4601).

Since the inventive Klotho mRNA enables particularly high Klotho expression levels, said Klotho mRNA is also particularly advantageous in the context of such encapsulated cells, since it can enable a high production level of Klotho by these cells.

In a further aspect, the present invention relates to the therapeutic delivery device for use as a medicament.

In the context of the therapeutic delivery device, the same therapeutic uses are preferred as defined above for the inventive Klotho mRNA or the inventive pharmaceutical composition. For instance, in an aspect the invention relates to the therapeutic delivery device or use in the prevention or treatment of a disease selected from the group consisting of kidney diseases, cardiovascular diseases, brain diseases, lung diseases, bone diseases, and metabolic diseases.

In a further aspect, the present invention relates to an extracellular vesicle (EV) comprising the inventive Klotho mRNA.

EVs are typically nanometer-sized vesicles produced by most cell types and functioning as the body's natural transport system for proteins, nucleic acids, peptides, lipids, and various other molecules between cells. EVs have emerged as a highly effective delivery system for therapeutic molecules, including mRNA, due to their innate ability to encapsulate and protect nucleic acids, facilitating their efficient delivery to recipient cells. The skilled person is familiar with methods to prepare such RNA-containing EVs and to use them for the delivery of mRNA. Such methods are disclosed, e.g. in WO 2010/119256 A1 and WO 2019/092145 A1. In addition, detailed reviews are provided in Lu et al. ("Exosome-based carrier for RNA delivery: progress and challenges." Pharmaceutics 15.2 (2023): 598) and Aslan et al. ("Exosomes for mRNA delivery: a novel biotherapeutic strategy with hurdles and hope." BMC biotechnology 21 (2021): 1-12).

EVs comprising specific mRNAs can be prepared by a variety of methods, many of which are detailed in the above references. For instance, mRNA can be loaded into EVs, e.g., by simple incubation. Alternatively, EV-producing cells may be transfected with DNA encoding the desired mRNA so that the cells produce the mRNA of interest. In the context of the invention, any suitable method may be used to load the Klotho mRNA into EVs. However, in a preferred embodiment, the EV is derived from the inventive cell. When the cell comprises the inventive DNA molecule comprising a DNA sequence encoding at least the coding region of the inventive Klotho mRNA, EVs derived from said cell contain said mRNA.

In the context of the invention, the EV may be any type of vesicle that is obtainable from a cell in any form, for instance a microvesicle (e.g. any vesicle shed from the plasma membrane of a cell), an exosome (e.g. any vesicle derived from the endo-lyso-somal pathway), an apoptotic body (e.g. obtainable from apoptotic cells), a microparticle (which may be derived from e.g. platelets), an ectosome (derivable from e.g. neutrophils and monocytes in serum), prostatosome (e.g. obtainable from prostate cancer cells), or a cardiosome (e.g. derivable from cardiac cells), etc. The sizes of EVs may vary considerably but an EV typically has a nano-sized hydrodynamic diameter, i.e. a diameter below 1000 nm. EVs may be derived from any cell type, in vivo, ex vivo, and in vitro. Preferred EVs include exosomes and microvesicles, but other EVs may also be advantageous in various circumstances. Furthermore, said terms shall also be understood to relate to extracellular vesicle mimics, cell membrane-based vesicles obtained through for instance membrane extrusion, sonication, or other techniques, etc. In a particularly preferred embodiment, the EV is an exosome.

In a further preferred embodiment, the EV comprises a targeting moiety expressed on the surface of the EV. Said targeting-moiety can enable targeted delivery to a cell, tissue, organ, and/or compartment of interest. The targeting moiety may be a peptide which is expressed as a fusion protein with a transmembrane protein typically expressed on the surface of the exosome. Suitable targeting moieties are known, e.g. from WO 2010/119256 A1 and WO 2019/092145 A1. For instance, suitable peptides may be those which bind to cell surface moieties such as receptors or their ligands found on the cell surface of the cell to be targeted. Examples of suitable targeting moieties are short peptides, scFv and complete proteins, so long as the targeting moiety can be expressed on the surface of the exosome and does not interfere with insertion of the membrane protein into the exosome.

In a further aspect, the present invention relates to a population of EVs as defined herein above. Preferably, the average number of Klotho mRNA molecules per EV throughout the population of EVs is above one per EV, preferably above 10 per EV, preferably above 100 per EV.

However, throughout the population there may also be EVs which do not comprise any Klotho mRNA molecules. In a preferred embodiment, at least 5%, at least 10%, at least 20%, at least 50%, at least 70%, at least 75%, at least 80%, at least 85%, at least 90%, and/or at least 95% of all EVs comprise at least one Klotho mRNA molecule.

In a further aspect, the present invention relates to a method for producing the inventive EV or the inventive population of EVs comprising:
- Culturing the inventive cell under conditions suitable for the formation of EVs; and
- Isolating the EVs.

The skilled person is familiar with how to produce EVs, e.g., as detailed in the above-mentioned references. Typically, the EVs may be isolated from cell culture medium, in which the inventive cell is cultured. EVs produced from the cells can be collected from the culture medium by any suitable method. Typically, EVs may be isolated by centrifugation, filtration or combinations of these methods.

In a further aspect, the present invention relates to a pharmaceutical formulation comprising the inventive EV or the inventive population of EVs. The embodiments described herein above for the inventive pharmaceutical formulation comprising the inventive Klotho mRNA are also preferred in the context of the inventive pharmaceutical formulation comprising the inventive EV or the inventive population of EVs.

Preferably, the pharmaceutical formulation may be formulated for parenteral, intramuscular, intracerebral, intravascular (including intravenous), subcutaneous, or transdermal administration. Formulations for parenteral administration may include sterile aqueous solutions which may also contain buffers, diluents and other suitable additives. The pharmaceutical formulation may include pharmaceutically acceptable carriers, thickeners, diluents, buffers, preservatives, and other pharmaceutically acceptable carriers or excipients and the like in addition to the EVs.

In a further aspect, the present invention relates to the inventive EV, the inventive population of EVs, or the inventive pharmaceutical formulation for use as a medicament.

In this context, the same therapeutic uses are preferred as defined above for the inventive Klotho mRNA. For instance, in an aspect the invention relates to the inventive EV, the inventive population of EVs, or the inventive pharmaceutical formulation for use in the prevention or treatment of a disease selected from the group consisting of kidney diseases, cardiovascular diseases, brain diseases, lung diseases, bone diseases, and metabolic diseases.

In a further aspect, the present invention relates to the use of the inventive EV, the inventive population of EVs or the pharmaceutical formulation for preventing and/or reducing a symptom of aging.

In yet another aspect, the invention relates to the use of the inventive EV, the inventive population of EVs or the pharmaceutical formulation for increasing the lifespan in an individual, preferably in a mammal, preferably a human.

It is preferred, if the above uses are non-therapeutic uses, especially the use for increasing the lifespan. Therefore, it is preferred if the individual is a healthy individual. It is preferred if the individual does not suffer from any of the diseases described herein.

In yet another aspect, the present invention relates to a cosmetic formulation comprising the inventive EV or the inventive population of EVs. Preferably, the cosmetic formulation comprises a cosmetic carrier. Preferably, cosmetic carrier is an ointment, a gel, especially a hydrogel, a liposome, nano/microparticles, or an emulsion.

According to a further aspect, the present invention also relates to a cosmetic skin care method, comprising contacting a skin of an individual, preferably a human individual, with the inventive EV or the inventive population of EVs or the cosmetic formulation according to the invention. Preferably, the skin is an ageing skin. It is especially preferred, if the cosmetic skin care method reduces any sign of aging, such as wrinkles.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The term "preventing" or "prevention" as used herein means to stop a disease state or condition from occurring in an individual completely or almost completely or at least to a (preferably significant) extent, especially when the individual is predisposed to such a risk of contracting a disease state or condition. However, these terms should not be interpreted as an absolute success in the sense that a patient can never develop an associated disease, reaction or condition but as the reduction of the chance of developing the disease, reaction or condition in a prophylactic treatment.

"Percent (%) sequence identity", "X% identical" (such as "70% identical") or similar terms with respect to a reference nucleotide sequence is defined as the percentage of nucleotides in a candidate sequence that are identical with the nucleotides in the reference sequence, after aligning the sequences and introducing gaps, if necessary, to achieve the maximum percent sequence identity, and not considering any conservative substitutions as part of the sequence identity. Gaps cause a lack of identity. Alignment for purposes of determining percent nucleotide sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % nucleotide sequence identity values are calculated using the sequence alignment of the computer programme "needle" of the EMBOSS software package (publicly available from European Molecular Biology Laboratory; Rice et al., EMBOSS: the European Molecular Biology Open Software Suite, Trends Genet. 2000 Jun;16(6):276-7, PMID: 10827456). The needle programme can be accessed under the web site http://www.ebi.ac.uk/Tools/psa/emboss_needle or downloaded for local installation as part of the EMBOSS package from http://emboss.sourceforge.net/. It runs on many widely-used UNIX operating systems, such as Linux.

To align two nucleotide sequences, the needle programme is preferably run with the following parameters:
Commandline: needle -auto -stdout -asequence SEQUENCE_FILE_A - bsequence SEQUENCE_FILE_B -datafile EDNAFULL -gapopen 10.0 -gapextend 0.5 -endopen 10.0 -endextend 0.5 -aformat3 pair -snucleotide1 -snucleotide2 (Align _format: pair Report _file: stdout)
The % nucleotide sequence identity of a given nucleotide sequence A to, with, or against a given nucleotide sequence B (which can alternatively be phrased as a given nucleotide sequence A that has or comprises a certain % nucleotide sequence identity to, with, or against a given nucleotide sequence B) is calculated as follows: 100 times the fraction X/Y
where X is the number of nucleotides scored as identical matches by the sequence alignment program needle in that program's alignment of A and B, and where Y is the total number of nucleotides in B. It will be appreciated that where the length of nucleotide sequence A is not equal to the length of nucleotide sequence B, the % nucleotide sequence identity of A to B will not equal the % nucleotide sequence identity of B to A. In cases where "a sequence of A is at least N% identical to the entire sequence of B", Y is the entire length of B. Unless specifically stated otherwise, all % nucleotide sequence identity values used herein are obtained as described in the immediately preceding paragraph using the needle computer program.

"Percent (%) sequence identity", "Percent (%) amino acid sequence identity", "X% identical" (such as "70% identical") or similar terms with respect to a reference polypeptide or protein sequence is defined in the same way as laid out above for nucleotide sequence identity, i.e., as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Gaps cause a lack of identity. Alignment for purposes of determining percent amino acid sequence identity is preferably done by the same methods as laid out above for nucleotide sequences. To align two protein sequences, the needle programme is preferably run with the following parameters:
Commandline: needle -auto -stdout -asequence SEQUENCE_FILE_A - bsequence SEQUENCE FILE_B -datafile EBLOSUM62 -gapopen 10.0 - gapextend 0.5 -endopen 10.0 -endextend 0.5 -aformat3 pair -sprotein1 -sprotein2 (Align _format: pair Report _file: stdout)
The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y
where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the entire sequence of B", Y is the entire sequence length of B (i.e. the entire number of amino acid residues in B). Unless specifically stated otherwise, all % amino acid sequence identity values used herein are obtained as described in the immediately preceding paragraph using the needle computer program.

Herein, "UniProt" refers to the Universal Protein Resource. UniProt is a comprehensive resource for protein sequence and annotation data. UniProt is a collaboration between the European Bioinformatics Institute (EMBL-EBI), the SIB Swiss Institute of Bioinformatics and the Protein Information Resource (PIR). Across the three institutes more than 100 people are involved through different tasks such as database curation, software development and support. Website: http://www.uniprot.org/

Entries in the UniProt databases are identified by their accession codes (referred to herein e.g. as "UniProt accession code" or briefly as "UniProt" followed by the accession code), usually a code of six alphanumeric letters (e.g. "Q1HVF7"). If not specified otherwise, the accession codes used herein refer to entries in the Protein Knowledgebase (UniProtKB) of UniProt. If not stated otherwise, the UniProt database state for all entries referenced herein is of 2 March 2023 (UniProt/UniProtKB Release 2023_01).

In the context of the present application, sequence variants (designated as "natural variant" in UniProt) are expressly included when referring to a UniProt database entry.

Unless specified otherwise, all parameters as used herein correspond to parameters at IUPAC SATP-conditions ("Standard Ambient Temperature and Pressure"), in particular a temperature of 25 °C and a pressure of 101.300 Pa.

Percentages (%) as used herein correspond to weight per volume (w/v) unless specified as weight per weight (w/w) or otherwise.

The present invention is further illustrated by the following figures and examples, without being limited thereto.
**Figure 1****.** Quantification of protein levels obtained after transfection of mammalian cell lines with different Klotho mRNA variants. Three different cell lines were used: (A) Hs27; (B) HEK293; (C) 3T3.
**Figure 2****.** Results of a biologically independent repetition of the experiments shown in Figure 1. (A) Hs27; (B) HEK293; (C) 3T3.
**Figure 3****.** Quantification of Klotho protein levels obtained after transfection of HEK293 cells with different Klotho mRNA variants over extended time spans. (A) Time course experiment up to 144 hours. (B) Cumulative Klotho protein levels over 7 days.
**Figure 4****.** Comparative tests quantifying Klotho protein levels after transfection of HEK293 cells with different Klotho mRNA variants. The sequences designated WO666_SEQ_xxxx correspond to mRNA sequences disclosed in WO 2013/151666 A2. These were tested against the GC62, GC60, GC57, GC55, GC53 variants disclosed herein as well as the native Klotho mRNA sequence. (A) Klotho protein expression levels. (B) Correlation between Klotho protein expression level (relative to the expression level obtained with the native Klotho mRNA sequence) and sequence identity to the variant GC62.
**Figure 5****.** Klotho protein expression from monoclonal HEK293 cells stably transfected with a DNA vector encoding for Klotho mRNA variant GC62 ("clone _10") compared to transient transfection with GC62 mRNA and untreated cells ("untr.").
**Figure 6****.** Serum Klotho protein levels of samples from mice injected with the Klotho mRNA variant GC62 as compared to the native sequences. Serum protein levels were determined at various time points following injection.

### Example 1. Klotho expression levels obtained from different mRNA sequence variants.

The Klotho protein expression levels obtained with different sequence variants were tested in order to quantify the effect of the different mRNA sequences on the expression levels in different cell lines.

### Sequence variants

The mRNA sequence of native human Klotho isoform 1 according to NCBI Reference Sequence: NM_004795.4 was used as a starting point. From this, the sequence coding for soluble Klotho, i.e., amino acids M1 to S981 was extracted (SEQ ID NO: 17). This sequence is 2946 nt long and can be divided into the signal peptide (amino acids 1-33; nt 1-99) and KL1 and KL2 domains including adjacent linking regions (amino acids E34 to S981; nt 100-2946). The part coding for the signal peptide was kept constant for the following comparative tests. The latter part of the sequence corresponding to nt 100-2946 was varied. As explained in the description herein above, four different publicly available codon optimization algorithms were used. In addition, custom variants with increasing GC-contents were created. To all these variants, the sequence for the signal peptide was then added. This resulted in the following variants:
- Variant "GC62" (SEQ ID NO: 18); GC content: 61.6 %
- Variant "GC60" (SEQ ID NO: 19); GC content: 60.1 %
- Variant "GC57" (SEQ ID NO: 22); GC content: 57.4 %
- Variant "GC55" (SEQ ID NO: 23); GC content: 54.9 %
- Variant "GC53" (SEQ ID NO: 24); GC content: 52.8 %
- "GENEius algorithm" (SEQ ID NO: 25); GC content: 52.6 %
- "ExpOptimizer" (SEQ ID NO: 27); GC content: 51.1 %
- "IDT Codon Optimization Tool" (SEQ ID NO: 26); GC content: 49.9 %
- "Twist Codon Optimization Tool" (SEQ ID NO: 28); GC content: 48.7 %

In order to finalize the constructs for synthesis, the sequences for the 5'-UTR (SEQ ID NO: 61) and 3'-UTR (SEQ ID NO: 62) and a T7 promotor sequence were added. The full mRNA sequences of the "GC62" and "GC60" variants are given in SEQ ID NOs: 29 and 30, respectively.

### Cloning and in vitro transcription (IVT)

Standard gene synthesis services and cloning techniques were used to obtain vectors containing the coding sequences of the different Klotho variants described above. The poly A tail needed for the in vitro transcription was added by polymerase chain reaction (PCR). The vectors containing the different coding sequences were used as templates. For the amplification the Phusion^{™} Plus DNA Polymerase Kit (Thermo Fisher) was used in accordance with the manufacturer's instructions.

For the PCR, 10ng plasmid DNA, 0.7µM of the forward primer, 5'-TGCTGCCGTAATACGACTCACTATAAGG-3' (SEQ ID NO: 77) and 0.7µM of the reverse primer, 5'-T120-TGCCGCCCACTCAGACTTTATTC-3' (SEQ ID NO: 78) (IDT technologies), were used. PCR was performed using the following cycling protocol: initial activation step at 98°C for 30s, followed by 35cycles of denaturation at 98 °C for 5s, annealing at 60°C for 10s, extension at 72 °C for 1:45min, and final extension at 72°C for 5min. After the DNA amplification, PCR products were purified using QIAquick PCR purification kit (Qiagen) and eluted in 2 × 20µl nuclease-free water (Qiagen). The quality and purity of the DNA was assessed by 1% agarose gel electrophoresis.

The IVT of the DNA into mRNA was performed using MEGAscript^{®} T7 Kit (Life Technologies, Darmstadt, Germany) according to the manufacturer's instructions. In brief, 20ul IVT reaction mixture containing 7.5mM ATP, GTP, CTP and UDP and 6mM CleanCAP (TriLink), 40U RiboLock RNase inhibitor (Thermo Fisher Scientific, Waltham, USA), 100ng PCR product, 1x reaction buffer and 1x T7 RNA polymerase enzyme mix was prepared. The mixture was incubated for 4h at 37 °C, then 1 µL TURBO DNase (from MEGAscript^{®} T7 Kit) was added to the IVT reaction mixture and incubated for 15min at 37°C to remove the template DNA. After the incubation, mRNA was purified using RNeasy Mini Kit (Qiagen) according to the manufacturer's instructions and eluted in 2 × 35 µL nuclease-free water. Subsequently, dephosphorylation was performed with 10U Antarctic phosphatase (New England Biolabs) at 37°C for 30min. The mRNA was purified and eluted in 2 × 35 µL nuclease-free water using RNeasy Mini Kit. The quality and purity of the synthesized and modified mRNA were confirmed in a 1% agarose gel. The synthesized mRNA was stored at -80°C and used for transfections.

### In vitro transfection

In preparation for mRNA transfections cells were plated at ~80% density (human: HEK, Hs27; mouse: 3T3). After 4 hours the mRNA was complexed with JetMessenger (Polyplus) at a ratio of 1:2 (µg mRNA : µL JetMessenger) according to the manufacturers manual. After 15min of incubation the mRNA complex was added dropwise to the cells.

0.75µg mRNA was added to the cells contained in a 24 well plate. The cell numbers per well were as follows: 70,000 (for Hs27), 200,000 (for HEK), 100,000 (for 3T3).

Depending on the type of the experiment, the cell pellets and/or the supernatant were harvested and frozen at -80°C until further analysis.

### ELISA

Klotho levels in cell culture supernatants were measured by ELISA (R&D systems, DY5334-05) according to the manufacturer's instructions.

### Cell lines

All cell lines were cultured at 37 °C and 5% CO2 atmosphere. Medium and supplements were used as recommend by the ATCC.

NIH/3T3 fibroblasts (ATCC, Manassas, Virginia, USA) were cultivated in DMEM with high glucose containing 10% fetal calf serum (FCS), and 1% penicillin/streptomycin. Cell culture medium and supplements were obtained from Thermo Fisher Scientific (Waltham, USA). Cells were kept at 37°C with 5% CO2 and medium was changed every 3days. Cells were passaged using TrypLE^{™} Express Enzyme (Thermo Fisher Scientific, 1260401).

Hs27 human fibroblasts (ATCC, Manassas, Virginia, USA) were cultivated in DMEM with high glucose containing 20% fetal calf serum (FCS), and 1% penicillin/streptomycin. Cell culture medium and supplements were obtained from Thermo Fisher Scientific (Waltham, USA). Cells were kept at 37 °C with 5% CO2 and medium was changed every 3 days. Cells were passaged using TrypLE^{™} Express Enzyme (Thermo Fisher Scientific, 1260401).

HEK293 cells (ATCC, Manassas, Virginia, USA) were cultivated in DMEM with high glucose containing 10% fetal calf serum (FCS), and 1% penicillin/streptomycin. Cell culture medium and supplements were obtained from Thermo Fisher Scientific (Waltham, USA). Cells were kept at 37°C with 5% CO2 and medium was changed every 3days. Cells were passaged using TrypLE^{™} Express Enzyme (Thermo Fisher Scientific, 1260401).

### Results

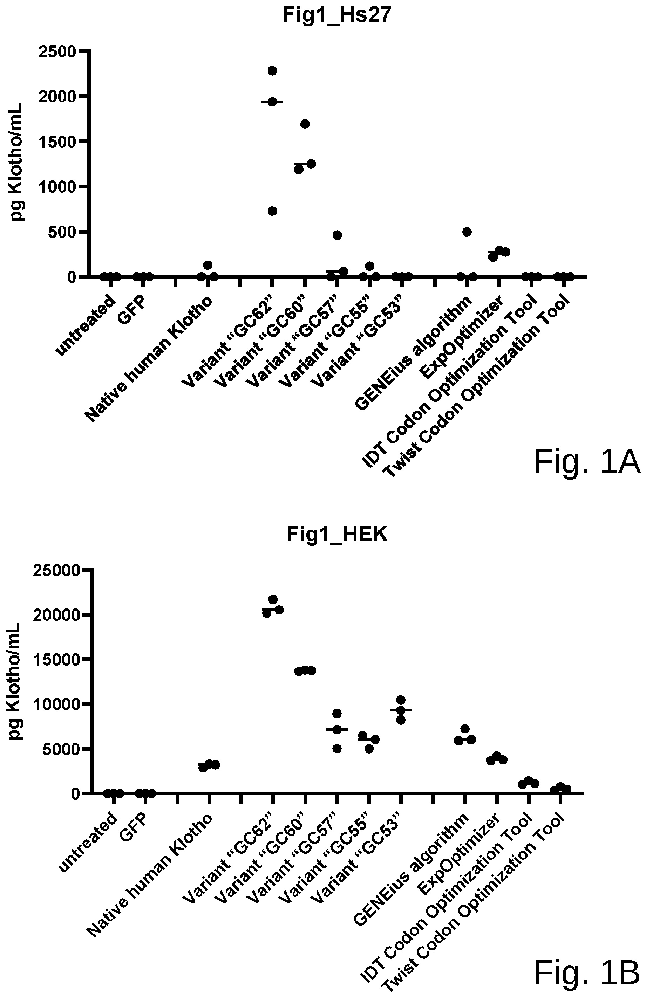

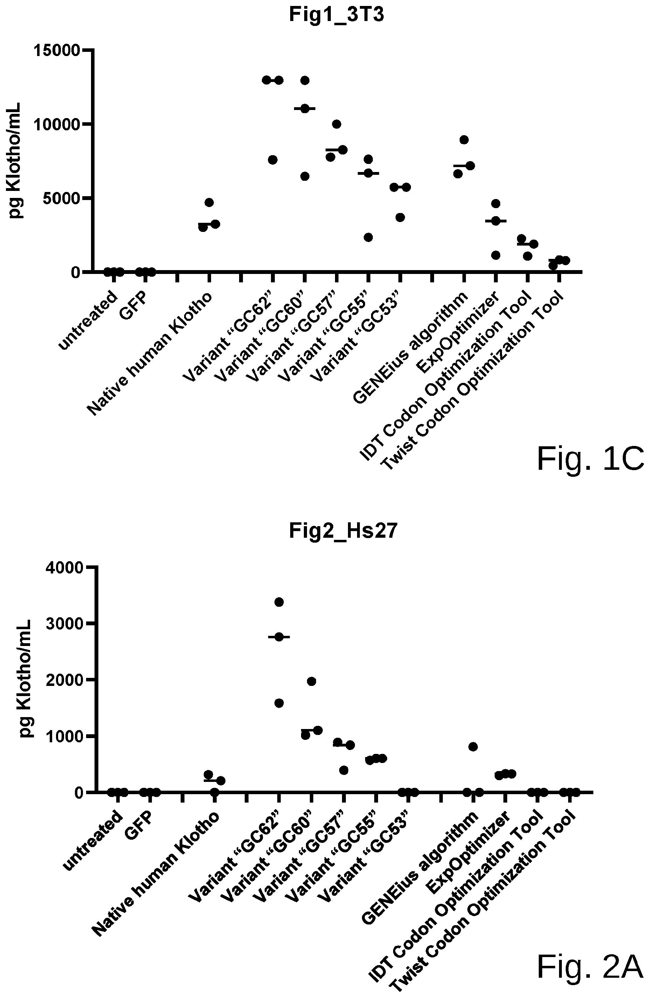

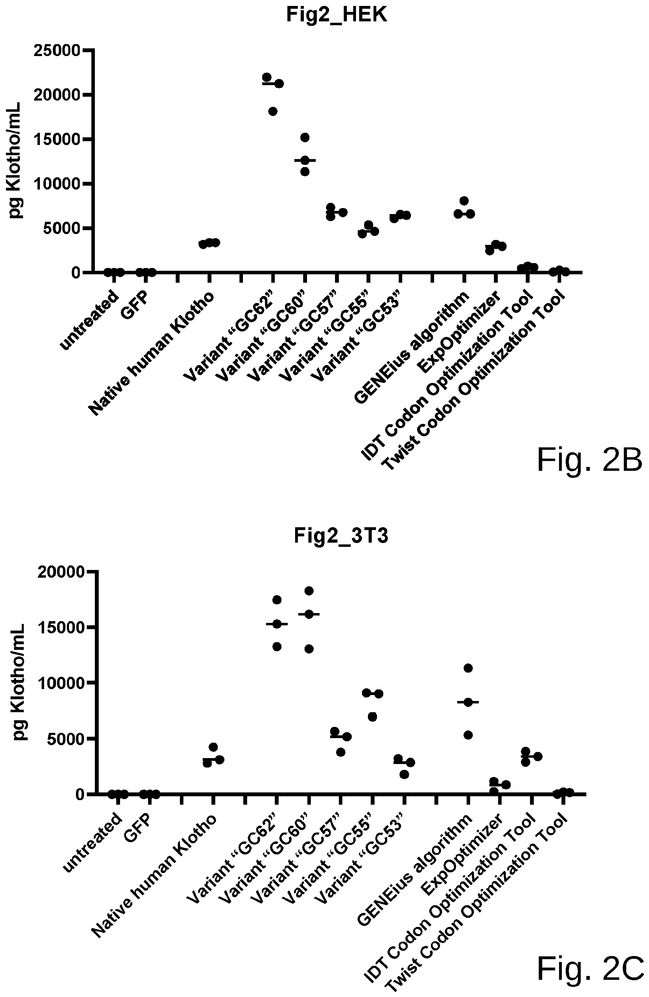

Figure 1 and Figure 2 show two biologically independent in vitro experiments in 3 different cell lines (human: Hs27 and HEK, murine: 3T3). The cell culture supernatant was harvested 24h after the mRNA transfection and analyzed by ELISA.

Over all the experiments and cell lines, the "GC62" variant outperformed all other variants as well as the native Klotho sequence. The increases in expression levels compared to the native sequence were between 3-fold and 6-fold in HEK and 3T3 cells and more than 10-fold in Hs27 cells. In addition, surprisingly, a direct relationship between the GC content and the expression level was found.

### Example 2. Time course experiments with different mRNA sequence variants

In order to investigate how the mRNA sequence impacts Klotho expression levels over time, experiments over longer time scales were carried out. These experiments were carried out essentially as in described Example 1 above. HEK293 cells were transfected as described above. Samples from cell culture supernatants were collected at various time points following transfection and analysed for Klotho protein levels as described above. Supernatants were exchanged every 24 hours during the time course experiment.

The following Klotho mRNA variants were tested: native human Klotho (SEQ ID NO: 17), variant "GC62" (SEQ ID NO: 18), and variant "GC60" (SEQ ID NO: 19). Based on these sequences, mRNAs were synthesized as described in Example 1.

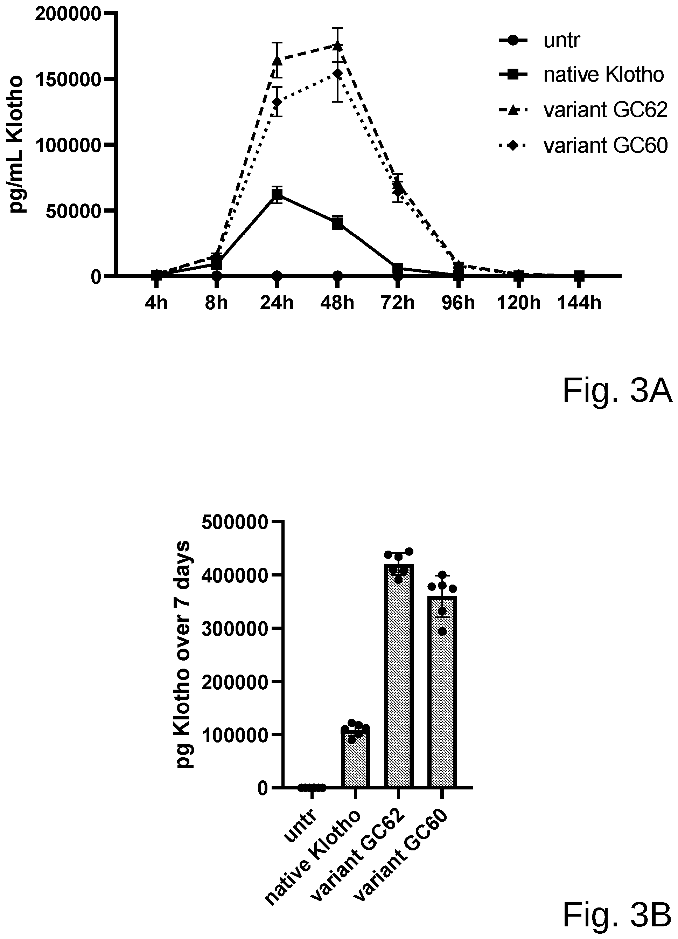

The results of this experiment are shown in Figures 3A and 3B. As can be seen from the time course in Figure 3A, Klotho expression levels following transfection with the inventive variants GC62 and GC60 are already significantly higher at 24 hours post transfection. However, strikingly, this difference is even exacerbated at 48 hours. At this point, the expression level obtained from the native sequence had already declined, while those obtained with the inventive variants had continued to increase. At 72 hours, the expression levels obtained from the GC62 and GC60 variants were still at least as high as the peak expression level obtained with the native sequence, while expression level obtained with the native sequence had already declined to close to zero. This demonstrates that not only the total expression level is strongly increased with the inventive variants relative to the native sequence (see Figure 3B for the cumulative expression over 7 days), but that the time window in which expression remains high is also much longer (Figure 3A).

### Example 3. Comparative tests with Klotho mRNA sequences mentioned in WO 2013/151666 A2

After the present invention had been made, the inventors found out that other Klotho mRNA sequences had been mentioned in the prior art. WO 2013/151666 A2 ("WO'666") broadly relates to mRNA for use in therapy. In Table 6, WO'666 mentions 655 different "targets", among which also klotho is mentioned as target No. 364. For each target, a large number of mRNA sequences are mentioned. This is also the case for target No. 364, klotho, for which the following sequence identification numbers are listed in Table 6: "1493, 2019, 2639, 3259, 3879, 4499, 14948-15347". Even though these sequences are only mentioned arbitrarily in WO'666 without any clear rationale, let alone experimental data, the present inventors set out to examine how these sequences compare to the sequences of the Klotho mRNA according to the present invention.

The sequences disclosed in WO'666 code for full-length human Klotho isoform 1 (amino acids M1 to K1012). Thus, as also described in Example 1, the sequence coding for soluble Klotho, i.e., amino acids M1 to S981, were extracted. These sequences are referred to herein as follows:
- "WO666_SEQ _1493": SEQ ID NO: 132
- "WO666_SEQ _14948": SEQ ID NO: 133
- "WO666_SEQ_4499": SEQ ID NO: 134
- "WO666_SEQ_2639": SEQ ID NO: 135
- "WO666_SEQ_3879": SEQ ID NO: 136
- "WO666_SEQ_3259": SEQ ID NO: 137
- "WO666_SEQ_2019": SEQ ID NO: 138

These sequences were tested against the GC62, GC60, GC57, GC55, GC53 variants disclosed herein as well as the native Klotho mRNA sequence. mRNAs based on all these sequences were synthesized and HEK293 cells were transfected and analysed as described in Example 1.

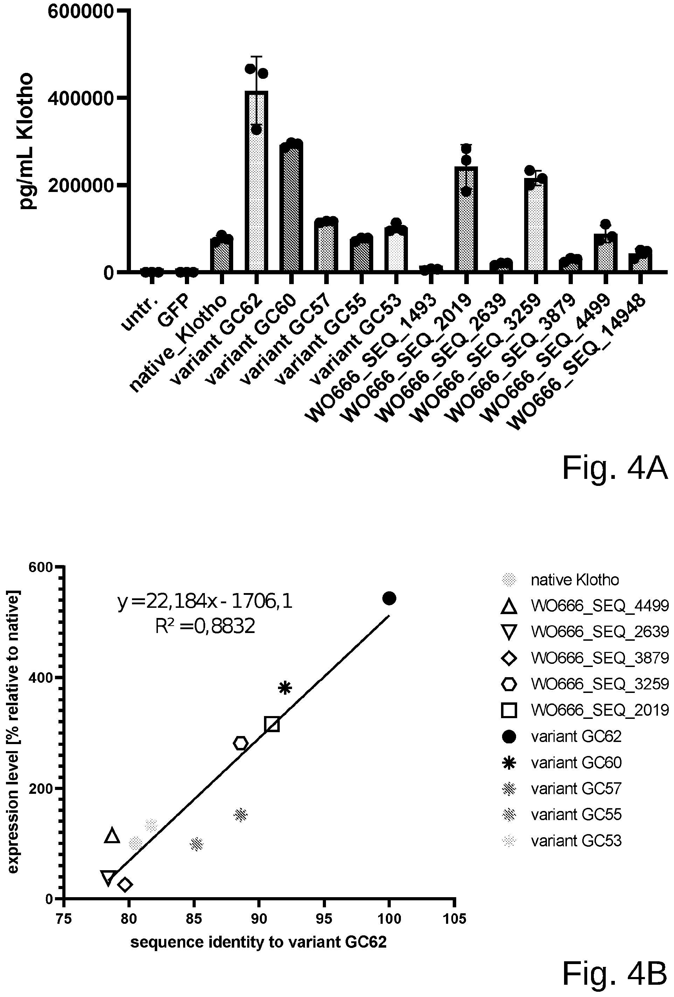

The results are shown in Figure 4A. The highest Klotho protein expression levels were observed for the inventive variants GC62 and GC60. Among the sequences mentioned in WO'666, the highest expression levels were observed for sequences 2019 (SEQ ID NO: 138) and 3259 (SEQ ID NO: 137); however, they were clearly lower than the inventive variants GC62 and GC60.

The obtained results are shown in the table below, together with the GC contents of each sequence as well as the sequence identity to the GC62 variant (SEQ ID NO: 18):

| | SEQ ID NO: | GC content (%) | seq identity to GC62 | Expr level (% of native) |
|---|---|---|---|---|
| native | 17 | 54.5 | 80.5 | 100 |
| GC62 | 18 | 61.6 | 100.0 | 543.46 |
| GC60 | 19 | 60.1 | 92.0 | 381.46 |
| GC57 | 22 | 57.4 | 88.6 | 151.57 |
| GC55 | 23 | 54.9 | 85.2 | 99.02 |
| GC53 | 24 | 52.6 | 81.7 | 132.9 |
| WO666_SEQ_1493 | 132 | 67.0 | 92.8 | 8.03 |
| WO666_SEQ_14948 | 133 | 41.1 | 65.0 | 56.33 |
| WO666_SEQ_4499 | 134 | 51.3 | 78.7 | 115.02 |
| WO666_SEQ_2639 | 135 | 52.3 | 78.4 | 37.27 |
| WO666_SEQ_3879 | 136 | 53.0 | 79.7 | 25.51 |
| WO666_SEQ_3259 | 137 | 61.6 | 88.6 | 281.49 |
| WO666_SEQ_2019 | 138 | 63.2 | 92.0 | 315.79 |

As can be seen from the above table, there is a striking correlation between the expression level and the sequence identity to the inventive variant GC62. The higher the sequence identity to GC 62, the higher the expression level. The only constructs of WO'666 that do not follow this trend are the two sequences with extremely high (sequence 1493, 67.0%); SEQ ID NO:132) and extremely low (sequence 14948, 41.1 %) GC contents. Removing these outliers with extreme GC contents, the remaining 11 datapoints (including the native sequence as well as the GC53-GC62 variants) follow a remarkable linear correlation, giving an R² value of 0.88 (see Figure 4B).

These results demonstrate that sequence identity to the GC62 variant is highly predictive of the obtained expression level.

### Example 4. Monoclonal cells for Klotho expression

To generate monoclonal cell lines expressing Klotho, HEK293 cells were stably transfected with a DNA vector encoding for Klotho mRNA variant GC62.

### Plasmid design:

Plasmids were designed in silico. The backbone of the used plasmid was pcDNA3.1/Hygro(+). It contained a hygromycin selection cassette and a CMV promotor. A DNA sequence encoding the inventive GC62 mRNA variant (SEQ ID NO: 18) was inserted into the multiple cloning site, right after the CMV promotor to ensure strong and stable expression of the gene of interest.

### Transfection and selection:

HEK293 cells were transfected with Lipofectamine 3000, according to the manufacturer protocol. 72h after transfection, the growth medium was supplemented with 100ug/mL hygromycin to select for transfected cells. After 7 days, all untransfected cells had died.

### Single cell cloning:

The transfected and selected cells were trypsinized and counted. The cell suspension was diluted to 8 cells/mL and plated onto several 96 well plates (100uL/well). After 10 days the wells with single colonies were expanded to 24 and later 6well plates.

### Characterization of single cell clones:

To characterize the single cell clones, 240.000 cells of each clone were seeded into 24 well plates and supernatant was harvested and replaced after 24, 48 and 72 hours. Klotho concentration in the supernatants was determined with ELISA as described in Example 1. The best performing clones were selected for further experiments.

### Results:

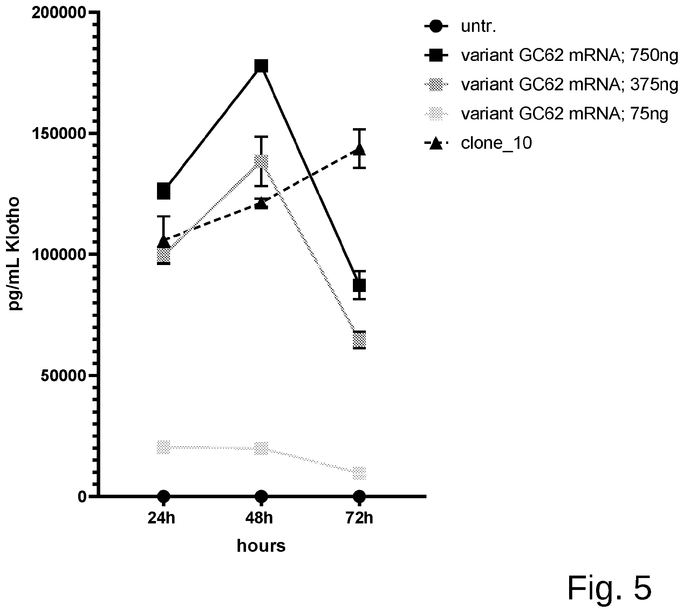

The Klotho protein expression level from cells of a single high-expressing clone compared to transient transfection with GC62 mRNA is shown in Figure 5. As can be seen from the figure, the expression levels obtained from the stably transfected cells continued to increase at 72 hours, while those obtained from the transiently transfected cells started to decrease.

### Example 5. In vivo experiments

In order to test the effects of the inventive mRNA in vivo, experiments with mice were carried out. In these experiments, Klotho protein serum levels obtained from treatment with the inventive Klotho mRNA variant GC62 was compared to those obtained with the native Klotho mRNA sequence. For these experiments, modified versions of the mRNA sequences were used, in which certain restriction enzyme recognition sites had been removed to facilitate molecular cloning. Thus, for the Klotho mRNA variant GC62 SEQ ID NO: 96 was used instead of SEQ ID NO: 18. For the native Klotho mRNA sequence SEQ ID NO: 95 was used instead of SEQ ID NO: 17. In both cases, the mRNA sequence coded for soluble Klotho (amino acids M1 to S981). Other than the modifications to remove restriction enzyme recognition sites, the mRNA constructs were the same as described in Example 1.

These mRNA constructs were formulated with LNP. 100 µg of the LNP-formulated mRNA were injected per mouse. For each the native sequence and the GC62 variant, 5 mice were used per timepoint. Terminal blood samples were taken at 6, 24, 48 and 72 hours respectively. Klotho protein levels in the serum were determined by ELISA.

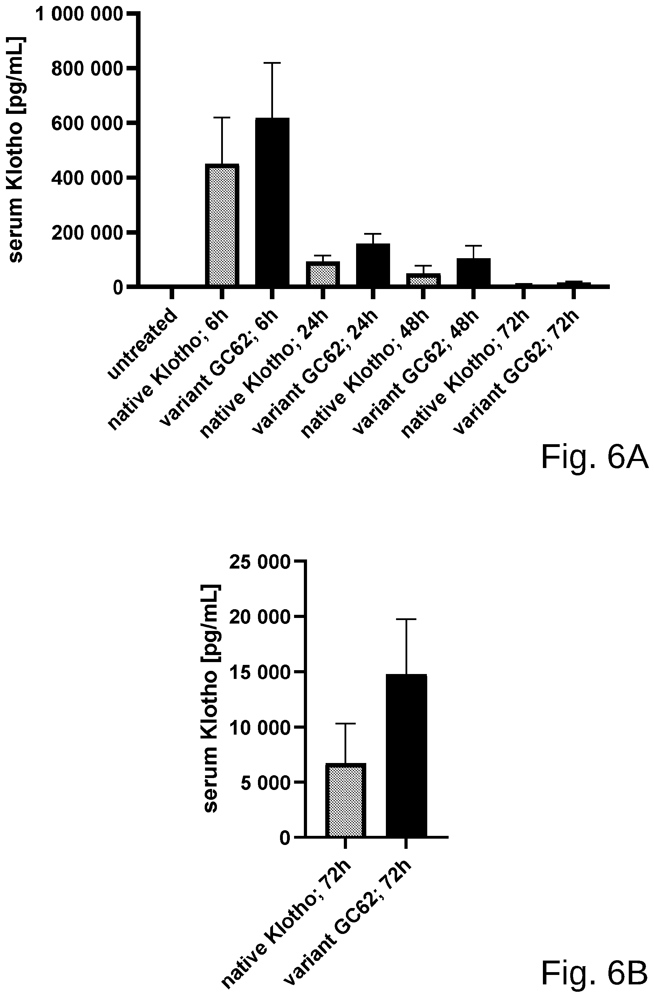

The results are shown in Figure 6. Serum Klotho levels were significantly higher using the inventive Klotho mRNA variant GC62 than when the native sequence was used, even though both the amount of mRNA injected and the amino acid sequence were the same. Thus, the strong increase in Klotho serum levels was directly caused by the improved mRNA sequence.

The difference in Klotho serum levels was particularly striking at later time points. As shown in Figure 6B, the serum Klotho levels 72 hours after injection were more than double those obtained with the native Klotho mRNA sequence. Thus, not only was the total expression level enhanced with the inventive mRNA; also the time window of the expression was strongly expanded.

### Sequences

The following tables provides an overview of the RNA sequences disclosed herein.

**Table 3A: Overview of a first set of disclosed RNA sequences.**

| **Klotho Isoform** | **Domains** | **Amino acids** | **SEQ ID NO:** | **GC content (%)** | **Length** |
|---|---|---|---|---|---|
| Isoforms 1/2 | KL1 | L56 to F506 | 1 | 61,9 | 1353 |
| | | | 2 | 59,9 | 1353 |
| | | | 3 | 61,9 | 1353 |
| | | | 4 | 60,1 | 1353 |
| Isoforms 1/2 | SP-lr-KL1 | M1 to F506 | 5 | 63,1 | 1521 |
| | | | 6 | 60,8 | 1521 |
| | | | 7 | 63,1 | 1521 |
| | | | 8 | 60,9 | 1521 |
| Isoform 1 | KL1-lr-KL2 | L56 to G952 | 9 | 60,9 | 2691 |
| | | | 10 | 59,5 | 2691 |
| | | | 11 | 60,9 | 2691 |
| | | | 12 | 59,6 | 2691 |
| Isoform 1 | lr-KL1-lr-KL2-lr | E34 to S981 | 13 | 61,3 | 2844 |
| | | | 14 | 59,7 | 2844 |
| | | | 15 | 61,3 | 2844 |
| | | | 16 | 59,8 | 2844 |
| Isoform 1 | SP-lr-KL1-lr-KL2-lr | M1 to S981 | 17 | 54,5 | 2946 |
| | | | 18 | 61,6 | 2946 |
| | | | 19 | 60,1 | 2946 |
| | | | 20 | 61,6 | 2946 |
| | | | 21 | 60,1 | 2946 |
| | | | 22 | 57,4 | 2946 |
| | | | 23 | 54,9 | 2946 |
| | | | 24 | 52,8 | 2946 |
| | | | 25 | 52,6 | 2946 |
| | | | 26 | 49,9 | 2946 |
| | | | 27 | 51,1 | 2946 |
| | | | 28 | 48,1 | 2946 |
| Isoform 1 | full_mRNA | M1 to S981 | 29 | 59,5 | 3249 |
| | | | 30 | 58,2 | 3249 |
| | | | 31 | 59,6 | 3249 |
| | | | 32 | 58,2 | 3249 |
| Isoform 2 | KL1-end | L56 to H549 | 33 | 61,7 | 1482 |
| | | | 34 | 59,5 | 1482 |
| | | | 35 | 61,7 | 1482 |
| | | | 36 | 59,6 | 1482 |
| Isoform 2 | lr-KL1-end | E34 to H549 | 37 | 62,3 | 1548 |
| | | | 38 | 59,8 | 1548 |
| | | | 39 | 62,4 | 1548 |
| | | | 40 | 59,9 | 1548 |
| Isoform 2 | SP-lr-KL1-end | M1 to H549 | 41 | 62,8 | 1650 |
| | | | 42 | 60,4 | 1650 |
| | | | 43 | 62,8 | 1650 |
| | | | 44 | 60,5 | 1650 |
| Isoform 2 | full_mRNA | M1 to H549 | 45 | 59,2 | 1953 |
| | | | 46 | 57,1 | 1953 |
| | | | 47 | 59,2 | 1953 |
| | | | 48 | 57,2 | 1953 |
| Isoform 1 | lr-KL1-lr-KL2-lr-TM-CT | E34 to K1012 | 64 | 60,5 | 2937 |
| | | | 65 | 59,0 | 2937 |
| | | | 66 | 60,6 | 2937 |
| | | | 67 | 59,1 | 2937 |
| Isoform 1 | SP-lr-KL1-lr-KL2-lr-TM-CT | M1 to K1012 | 68 | 60,8 | 3039 |
| | | | 69 | 59,4 | 3039 |
| | | | 70 | 60,9 | 3039 |
| | | | 71 | 59,5 | 3039 |
| Isoform 1 | full_mRNA | M1 to K1012 | 72 | 58,9 | 3342 |
| | | | 73 | 57,6 | 3342 |
| | | | 74 | 58,9 | 3342 |
| | | | 75 | 57,7 | 3342 |

**Table 3B: Overview of a second set of disclosed RNA sequences, which were modified to remove recognition sequences of restriction enzymes.**

| **Klotho Isoform** | **Domains** | **Amino acids** | **SEQ ID NO:** | **GC content (%)** | **Length** |
|---|---|---|---|---|---|
| Isoforms 1/2 | KL1 | L56 to F506 | 79 | 61,6 | 1353 |
| | | | 80 | 59,8 | 1353 |
| | | | 81 | 61,7 | 1353 |
| | | | 82 | 59,9 | 1353 |
| Isoforms 1/2 | SP-lr-KL1 | M1 to F506 | 83 | 62,9 | 1521 |
| | | | 84 | 60,7 | 1521 |
| | | | 85 | 62,9 | 1521 |
| | | | 86 | 60,8 | 1521 |
| Isoform 1 | KL1-lr-KL2 | L56 to G952 | 87 | 60,8 | 2691 |
| | | | 88 | 59,4 | 2691 |
| | | | 89 | 60,8 | 2691 |
| | | | 90 | 59,5 | 2691 |
| Isoform 1 | lr-KL1-lr-KL2-lr | E34 to S981 | 91 | 61,1 | 2844 |
| | | | 92 | 59,6 | 2844 |
| | | | 93 | 61,2 | 2844 |
| | | | 94 | 59,7 | 2844 |
| Isoform 1 | SP-lr-KL1-lr-KL2-lr | M1 to S981 | 95 | 54,6 | 2946 |
| | | | 96 | 61,4 | 2946 |
| | | | 97 | 60,0 | 2946 |
| | | | 98 | 61,5 | 2946 |
| | | | 99 | 60,0 | 2946 |
| Isoform 1 | full_mRNA | M1 to S981 | 100 | 59,4 | 3249 |
| | | | 101 | 58,1 | 3249 |
| | | | 102 | 59,4 | 3249 |
| | | | 103 | 58,1 | 3249 |
| Isoform 2 | KL1-end | L56 to H549 | 104 | 61,5 | 1482 |
| | | | 105 | 59,4 | 1482 |
| | | | 106 | 61,5 | 1482 |
| | | | 107 | 59,5 | 1482 |
| Isoform 2 | lr-KL1-end | E34 to H549 | 108 | 62,1 | 1548 |
| | | | 109 | 59,6 | 1548 |
| | | | 110 | 62,2 | 1548 |
| | | | 111 | 59,8 | 1548 |
| Isoform 2 | SP-lr-KL1-end | M1 to H549 | 112 | 62,6 | 1650 |
| | | | 113 | 60,2 | 1650 |
| | | | 114 | 62,7 | 1650 |
| | | | 115 | 60,4 | 1650 |
| Isoform 2 | full_mRNA | M1 to H549 | 116 | 59,0 | 1953 |
| | | | 117 | 57,0 | 1953 |
| | | | 118 | 59,1 | 1953 |
| | | | 119 | 57,1 | 1953 |
| Isoform 1 | lr-KL1-lr-KL2-lr-TM-CT | E34 to K1012 | 120 | 60,4 | 2937 |
| | | | 121 | 58,9 | 2937 |
| | | | 122 | 60,4 | 2937 |
| | | | 123 | 59,0 | 2937 |
| Isoform 1 | SP-lr-KL1- | M1 to K1012 | 124 | 60,7 | 3039 |
| | lr-KL2-lr-TM-CT | | 125 | 59,3 | 3039 |
| | | | 126 | 60,7 | 3039 |
| | | | 127 | 59,4 | 3039 |
| Isoform 1 | full_mRNA | M1 to K1012 | 128 | 58,8 | 3342 |
| | | | 129 | 57,5 | 3342 |
| | | | 130 | 58,8 | 3342 |
| | | | 131 | 57,6 | 3342 |

The following table provides an overview of the protein sequences disclosed herein.

**Table 4: Overview of disclosed protein sequences.**

| **Klotho Isoform** | **Domains** | **Amino acids** | **SEQ ID NO:** | **Length** |
|---|---|---|---|---|
| Isoform 1 | native sequence | M1 to K1012 | 49 | 1012 |
| Isoform 2 | native sequence | M1 to H549 | 50 | 549 |
| Isoforms 1/2 | SP | M1 to A33 | 51 | 33 |
| Isoforms 1/2 | KL1 | L56 to F506 | 52 | 451 |
| | | | 53 | 451 |
| Isoforms 1 | KL2 | L515 to G952 | 54 | 438 |
| Isoforms 1/2 | SP-lr-KL1 | M1 to F506 | 55 | 506 |
| | | | 56 | 506 |
| Isoform 1 | SP-lr-KL1-lr-KL2-lr | M1 to S981 | 57 | 981 |
| | | | 58 | 981 |
| Isoform 2 | SP-lr-KL1-end | M1 to H549 | 59 | 549 |
| | | | 60 | 549 |
| Isoform 1 | native sequence | M1 to K1012 | 76 | 1012 |

SEQ ID NO: 1: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 2: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
 
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUC
SEQ ID NO: 3: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 4: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUC
SEQ ID NO: 5: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 6: AUGCCCGCUAGCGCCCCACCGAGAAGACCGCGGCCGCCACCGCCAUCGCUGUCGCUGCUGCUGGUGCUGCUGGGACUGGGCGGAAG 
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 7: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 8: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 9: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
 
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGACCUGCCCAUGUAUAUCAUCAGCAACGGCAUCGACGACGGCCUGCACGCCGAGGACGACCAGCUGA
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 10: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGACCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 11: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
 
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGACCUGCCCAUGUAUAUCAUCAGCAACGGCAUCGACGACGGCCUGCACGCCGAGGACGACCAGCUGA
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 12: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGACCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 13: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
 
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 14: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 15: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
 
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 16: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 17: 
AUGCCCGCCAGCGCCCCGCCGCGCCGCCCGCGGCCGCCGCCGUCGCUGUCGCUGCUGGUGCUGCUGGGCCUGGGCGGCCG
CCGCCUGCGUGCGGAGCCGGGCGACGGCGCGCAGACCUGGGCCCGUUUCUCGCGGCCUCCUGCCCCCGAGGCCGCGGGCCUCUUCC
AGGGCACCUUCCCCGACGGCUUCCUCUGGGCCGUGGGCAGCGCCGCCUACCAGACCGAGGGCGGCUGGCAGCAGCACGGCAAGGGU
GCGUCCAUCUGGGAUACGUUCACCCACCACCCCCUGGCACCCCCGGGAGACUCCCGGAACGCCAGUCUGCCGUUGGGCGCCCCGUC
GCCGCUGCAGCCCGCCACCGGGGACGUAGCCAGCGACAGCUACAACAACGUCUUCCGCGACACGGAGGCGCUGCGCGAGCUCGGGG
UCACUCACUACCGCUUCUCCAUCUCGUGGGCGCGAGUGCUCCCCAAUGGCAGCGCGGGCGUCCCCAACCGCGAGGGGCUGCGCUAC
UACCGGCGCCUGCUGGAGCGGCUGCGGGAGCUGGGCGUGCAGCCCGUGGUCACCCUGUACCACUGGGACCUGCCCCAGCGCCUGCA
GGACGCCUACGGCGGCUGGGCCAACCGCGCCCUGGCCGACCACUUCAGGGAUUACGCGGAGCUCUGCUUCCGCCACUUCGGCGGUC
AGGUCAAGUACUGGAUCACCAUCGACAACCCCUACGUGGUGGCCUGGCACGGCUACGCCACCGGGCGCCUGGCCCCCGGCAUCCGG
GGCAGCCCGCGGCUCGGGUACCUGGUGGCGCACAACCUCCUCCUGGCUCAUGCCAAAGUCUGGCAUCUCUACAAUACUUCUUUCCG
UCCCACUCAGGGAGGUCAGGUGUCCAUUGCCCUAAGCUCUCACUGGAUCAAUCCUCGAAGAAUGACCGACCACAGCAUCAAAGAAU
GUCAAAAAUCUCUGGACUUUGUACUAGGUUGGUUUGCCAAACCCGUAUUUAUUGAUGGUGACUAUCCCGAGAGCAUGAAGAAUAAC
CUUUCAUCUAUUCUGCCUGAUUUUACUGAAUCUGAGAAAAAGUUCAUCAAAGGAACUGCUGACUUUUUUGCUCUUUGCUUUGGACC
CACCUUGAGUUUUCAACUUUUGGACCCUCACAUGAAGUUCCGCCAAUUGGAAUCUCCCAACCUGAGGCAACUGCUUUCCUGGAUUG
ACCUUGAAUUUAACCAUCCUCAAAUAUUUAUUGUGGAAAAUGGCUGGUUUGUCUCAGGGACCACCAAGAGAGAUGAUGCCAAAUAU
AUGUAUUACCUCAAAAAGUUCAUCAUGGAAACCUUAAAAGCCAUCAAGCUGGAUGGGGUGGAUGUCAUCGGGUAUACCGCAUGGUC
CCUCAUGGAUGGUUUCGAGUGGCACAGAGGUUACAGCAUCAGGCGUGGACUCUUCUAUGUUGACUUUCUAAGCCAGGACAAGAUGU
 
UGUUGCCAAAGUCUUCAGCCUUGUUCUACCAAAAGCUGAUAGAGAAAAAUGGCUUCCCUCCUUUACCUGAAAAUCAGCCCCUAGAA
GGGACAUUUCCCUGUGACUUUGCUUGGGGAGUUGUUGACAACUACAUUCAAGUAGAUACCACUCUGUCUCAGUUUACCGACCUGAA
UGUUUACCUGUGGGAUGUCCACCACAGUAAAAGGCUUAUUAAAGUGGAUGGGGUUGUGACCAAGAAGAGGAAAUCCUACUGUGUUG
ACUUUGCUGCCAUCCAGCCCCAGAUCGCUUUACUCCAGGAAAUGCACGUUACACAUUUUCGCUUCUCCCUGGACUGGGCCCUGAUU
CUCCCUCUGGGUAACCAGUCCCAGGUGAACCACACCAUCCUGCAGUACUAUCGCUGCAUGGCCAGCGAGCUUGUCCGUGUCAACAU
CACCCCAGUGGUGGCCCUGUGGCAGCCUAUGGCCCCGAACCAAGGACUGCCGCGCCUCCUGGCCAGGCAGGGCGCCUGGGAGAACC
CCUACACUGCCCUGGCCUUUGCAGAGUAUGCCCGACUGUGCUUUCAAGAGCUCGGCCAUCACGUCAAGCUUUGGAUAACGAUGAAU
GAGCCGUAUACAAGGAAUAUGACAUACAGUGCUGGCCACAACCUUCUGAAGGCCCAUGCCCUGGCUUGGCAUGUGUACAAUGAAAA
GUUUAGGCAUGCUCAGAAUGGGAAAAUAUCCAUAGCCUUGCAGGCUGAUUGGAUAGAACCUGCCUGCCCUUUCUCCCAAAAGGACA
AAGAGGUGGCUGAGAGAGUUUUGGAAUUUGACAUUGGCUGGCUGGCUGAGCCCAUUUUCGGCUCUGGAGAUUAUCCAUGGGUGAUG
AGGGACUGGCUGAACCAAAGAAACAAUUUUCUUCUUCCUUAUUUCACUGAAGAUGAAAAAAAGCUAAUCCAGGGUACCUUUGACUU
UUUGGCUUUAAGCCAUUAUACCACCAUCCUUGUAGACUCAGAAAAAGAAGAUCCAAUAAAAUACAAUGAUUACCUAGAAGUGCAAG
AAAUGACCGACAUCACGUGGCUCAACUCCCCCAGUCAGGUGGCGGUAGUGCCCUGGGGGUUGCGCAAAGUGCUGAACUGGCUGAAG
UUCAAGUACGGAGACCUCCCCAUGUACAUAAUAUCCAAUGGAAUCGAUGACGGGCUGCAUGCUGAGGACGACCAGCUGAGGGUGUA
UUAUAUGCAGAAUUACAUAAACGAAGCUCUCAAAGCCCACAUACUGGAUGGUAUCAAUCUUUGCGGAUACUUUGCUUAUUCGUUUA
ACGACCGCACAGCUCCGAGGUUUGGCCUCUAUCGUUAUGCUGCAGAUCAGUUUGAGCCCAAGGCAUCCAUGAAACAUUACAGGAAA
AUUAUUGACAGCAAUGGUUUCCCGGGCCCAGAAACUCUGGAAAGAUUUUGUCCAGAAGAAUUCACCGUGUGUACUGAGUGCAGUUU
UUUUCACACCCGAAAGUCUUGA
SEQ ID NO: 18: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCUGA
SEQ ID NO: 19: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
 
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 20: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCUGA
SEQ ID NO: 21: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
 
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 22: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCAGGCGAUGGCGCCCAGACUUGGGCCCGCUUCAGCAGACCUCCCGCCCCCGAGGCCGCCGGAUUGUUCC
AGGGAACCUUCCCUGACGGUUUCCUGUGGGCCGUGGGUAGCGCCGCCUACCAGACCGAGGGCGGCUGGCAGCAGCACGGCAAGGGC
GCAAGCAUCUGGGACACCUUUACCCACCACCCCCUGGCCCCCCCCGGCGACAGUAGAAAUGCUAGUCUCCCCCUGGGCGCCCCCAG
CCCCCUUCAGCCCGCCACCGGAGACGUGGCCUCAGACAGCUACAACAACGUGUUCAGAGACACCGAAGCUCUGAGGGAGCUCGGCG
UGACCCACUAUCGGUUUAGCAUUAGCUGGGCCAGAGUGCUGCCCAACGGCAGCGCCGGCGUGCCCAACCGCGAAGGCCUCAGAUAC
UAUCGCAGACUGCUGGAGAGGCUGCGGGAGCUGGGCGUGCAGCCCGUGGUAACUCUGUACCACUGGGAUCUGCCCCAGAGAUUGCA
AGACGCUUACGGCGGAUGGGCCAACCGCGCUCUCGCCGACCACUUCCGGGACUACGCAGAGCUGUGCUUCCGCCACUUCGGCGGUC
AGGUGAAAUAUUGGAUUACAAUCGACAACCCCUACGUUGUGGCCUGGCACGGGUACGCUACUGGGCGCCUGGCACCCGGCAUCCGC
GGCAGCCCCAGACUGGGCUACCUUGUUGCUCACAACCUCCUGCUGGCUCAUGCAAAGGUGUGGCACUUGUACAACACAAGUUUCCG
CCCCACCCAGGGUGGACAGGUGUCAAUCGCACUGUCUUCACACUGGAUUAACCCCCGGCGCAUGACUGACCACUCAAUUAAGGAAU
GCCAGAAGAGCCUGGACUUCGUUCUGGGCUGGUUCGCCAAGCCCGUGUUUAUCGACGGGGACUACCCCGAGAGCAUGAAGAAUAAU
CUGAGCAGCAUCUUGCCCGACUUUACCGAGAGCGAAAAGAAAUUCAUCAAGGGCACCGCCGACUUUUUCGCACUGUGUUUCGGCCC
CACUCUGAGCUUUCAACUGCUCGAUCCCCACAUGAAGUUUAGACAACUGGAGUCCCCUAAUCUGCGCCAGCUUCUGAGCUGGAUUG
AUCUGGAAUUUAACCAUCCCCAGAUUUUUAUAGUUGAGAAUGGGUGGUUUGUUAGCGGCACCACUAAGCGCGAUGACGCAAAGUAC
AUGUAUUAUCUGAAGAAAUUUAUCAUGGAAACUCUGAAGGCCAUAAAACUCGACGGCGUUGACGUGAUCGGUUAUACCGCAUGGAG
CCUUAUGGACGGGUUCGAGUGGCACCGCGGGUACUCCAUCAGGAGGGGCUUGUUCUACGUCGACUUCCUCAGUCAGGACAAGAUGU
UGCUGCCUAAGAGCAGCGCCUUGUUCUACCAGAAGUUGAUCGAAAAGAACGGCUUUCCACCACUCCCCGAGAACCAACCCCUGGAG
GGCACCUUUCCCUGCGAUUUCGCUUGGGGCGUGGUGGACAACUACAUCCAGGUGGAUACCACUCUGUCUCAGUUCACUGAUCUCAA
CGUGUAUCUGUGGGACGUCCACCACUCCAAGAGGCUGAUCAAGGUGGACGGCGUCGUGACCAAGAAGAGAAAGUCCUACUGCGUGG
ACUUUGCCGCCAUUCAGCCCCAGAUCGCCCUGCUGCAGGAGAUGCAUGUCACACACUUCCGCUUUUCUCUGGACUGGGCCCUGAUU
CUGCCCCUGGGGAACCAGUCCCAGGUCAACCACACCAUUCUCCAGUACUACCGCUGUAUGGCCAGUGAGCUGGUACGCGUGAACAU
CACCCCCGUCGUGGCCCUCUGGCAGCCCAUGGCCCCCAAUCAGGGCCUGCCACGCCUGCUCGCCCGGCAGGGAGCCUGGGAAAAUC
CAUACACUGCACUCGCCUUCGCUGAGUACGCCAGGCUGUGCUUCCAGGAGCUGGGACAUCACGUGAAGCUGUGGAUUACCAUGAAC
GAACCAUACACUAGAAAUAUGACCUACUCCGCCGGACACAAUCUCCUGAAGGCCCACGCACUGGCUUGGCACGUGUACAACGAGAA
GUUCAGGCACGCCCAGAAUGGAAAGAUUAGCAUCGCCCUGCAGGCCGACUGGAUCGAGCCCGCCUGUCCCUUCAGCCAGAAAGACA
AAGAGGUGGCUGAGCGCGUGCUGGAGUUUGAUAUUGGCUGGCUUGCUGAGCCAAUAUUCGGCAGUGGAGACUACCCCUGGGUGAUG
AGAGAUUGGCUUAACCAGAGAAACAACUUUCUCCUGCCCUACUUCACCGAGGACGAAAAGAAACUGAUCCAGGGUACCUUCGAUUU
CCUUGCUCUGAGCCACUAUACUACCAUCCUGGUCGACAGCGAGAAGGAGGACCCCAUCAAAUAUAACGACUACCUGGAGGUGCAAG
AGAUGACUGACAUCACAUGGCUGAAUAGCCCUAGCCAGGUCGCCGUGGUGCCUUGGGGCCUGCGCAAGGUGCUCAACUGGCUGAAG
UUCAAGUAUGGCGACCUGCCCAUGUACAUCAUCAGCAACGGCAUUGAUGACGGACUGCACGCCGAGGAUGAUCAACUCAGAGUAUA
CUAUAUGCAGAACUAUAUCAACGAGGCAUUGAAGGCCCACAUCCUGGACGGCAUCAACCUGUGUGGGUACUUUGCAUACUCCUUCA
ACGACAGAACCGCUCCCCGCUUUGGUCUGUAUAGGUACGCUGCCGACCAGUUCGAGCCCAAGGCCAGCAUGAAGCACUACCGCAAG
AUUAUCGAUAGCAAUGGCUUCCCUGGCCCCGAGACCCUUGAGCGCUUUUGCCCCGAGGAGUUCACCGUGUGUACCGAAUGUUCCUU
CUUCCACACACGCAAGUCAUGA
SEQ ID NO: 23: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGAUGGCGCUCAGACUUGGGCUCGCUUCAGCAGACCCCCAGCCCCCGAAGCAGCCGGAUUGUUCC
AGGGAACCUUCCCCGAUGGUUUCCUGUGGGCCGUAGGUAGCGCCGCCUACCAGACUGAGGGCGGAUGGCAGCAGCACGGCAAGGGC
GCAAGCAUCUGGGACACCUUCACCCACCACCCCCUGGCCCCCCCUGGCGACUCCCGCAAUGCUAGUCUCCCCCUGGGCGCCCCUAG
CCCCCUUCAGCCCGCCACUGGCGACGUGGCCUCAGACAGCUACAACAAUGUGUUCCGGGACACAGAAGCUCUGAGGGAGCUUGGCG
UGACCCAUUAUAGGUUUAGCAUCUCAUGGGCCCGCGUGCUGCCAAAUGGCAGCGCCGGCGUGCCUAACCGCGAAGGACUUCGCUAC
UAUCGCAGACUGCUGGAGAGGCUGCGGGAACUGGGGGUGCAACCUGUGGUGACAUUGUACCACUGGGAUCUGCCUCAGCGCUUGCA
AGACGCUUACGGUGGAUGGGCCAACCGCGCUCUGGCCGACCAUUUCCGGGACUACGCAGAGCUUUGCUUCCGCCACUUCGGGGGUC
 
AAGUGAAAUAUUGGAUUACAAUCGAUAACCCCUACGUGGUUGCAUGGCAUGGCUACGCCACAGGGCGCCUUGCUCCCGGCAUCAGA
GGAAGCCCCAGGCUGGGUUAUCUUGUGGCACACAACCUCCUGCUGGCACACGCAAAAGUGUGGCACUUGUAUAACACAAGUUUCCG
CCCCACCCAGGGUGGACAGGUGUCAAUAGCACUGUCUUCACACUGGAUUAAUCCUCGGAGGAUGACAGAUCACUCCAUCAAGGAAU
GCCAGAAGAGCCUGGACUUCGUCCUGGGCUGGUUUGCUAAGCCCGUGUUUAUCGACGGGGACUACCCCGAGAGCAUGAAGAAUAAU
CUGAGCAGCAUAUUGCCAGAUUUUACCGAGUCCGAGAAGAAAUUUAUCAAAGGGACCGCUGACUUUUUCGCUCUGUGUUUCGGCCC
CACUCUGAGCUUUCAAUUGCUGGAUCCACACAUGAAGUUUAGACAACUGGAAAGUCCCAAUCUGCGGCAGUUGCUGAGCUGGAUUG
AUCUGGAAUUCAACCAUCCCCAGAUUUUUAUUGUUGAGAAUGGGUGGUUUGUUAGUGGCACUACAAAGAGGGAUGACGCCAAGUAC
AUGUACUAUCUGAAGAAAUUCAUCAUGGAGACCCUGAAGGCCAUAAAGCUCGACGGAGUUGACGUGAUCGGUUAUACCGCAUGGAG
CCUGAUGGACGGGUUCGAGUGGCACCGCGGGUACAGCAUCAGGAGAGGCUUGUUCUACGUCGACUUCUUGUCACAGGACAAAAUGU
UGUUGCCUAAGUCAAGUGCCUUGUUUUACCAGAAACUGAUUGAAAAAAACGGCUUUCCUCCUUUGCCCGAGAACCAACCUCUGGAG
GGAACCUUUCCCUGCGAUUUUGCUUGGGGCGUGGUGGACAACUACAUCCAGGUGGACACAACCCUGUCUCAGUUCACCGAUUUGAA
UGUGUAUCUCUGGGAUGUCCACCACUCCAAGCGCCUGAUCAAGGUGGAUGGCGUCGUGACCAAGAAAAGAAAGAGCUACUGCGUGG
ACUUUGCCGCCAUUCAGCCCCAGAUUGCCCUGCUCCAGGAGAUGCACGUCACACACUUUAGGUUCUCCCUGGACUGGGCCCUGAUA
CUGCCCCUGGGUAACCAGUCACAAGUGAACCAUACCAUUUUGCAGUACUACCGCUGUAUGGCUUCAGAGCUGGUACGCGUGAACAU
CACCCCCGUAGUUGCCCUCUGGCAGCCCAUGGCCCCCAAUCAGGGCCUGCCUCGCCUGCUCGCCCGGCAGGGAGCCUGGGAAAACC
CAUACACCGCACUUGCCUUCGCUGAGUACGCCAGGUUGUGCUUCCAGGAGCUGGGACAUCACGUGAAGCUUUGGAUUACCAUGAAC
GAGCCAUACACCAGGAACAUGACAUACUCCGCCGGACAUAAUCUGCUGAAGGCUCACGCACUGGCCUGGCACGUGUACAACGAGAA
GUUUCGGCAUGCCCAGAAUGGAAAGAUCAGCAUCGCUCUGCAGGCAGACUGGAUCGAGCCUGCCUGUCCUUUCAGUCAGAAAGAUA
AAGAGGUGGCCGAGCGGGUGCUGGAAUUCGAUAUCGGAUGGCUGGCUGAACCAAUCUUCGGCAGUGGGGACUACCCCUGGGUGAUG
AGAGAUUGGCUGAACCAGCGCAACAAUUUUCUGCUGCCCUACUUUACCGAGGACGAAAAGAAGCUUAUCCAGGGUACCUUCGAUUU
CCUUGCCCUGUCCCACUAUACUACCAUACUUGUCGACUCUGAGAAAGAGGACCCCAUCAAAUACAAUGACUACCUGGAGGUGCAAG
AAAUGACAGACAUAACCUGGCUGAAUAGCCCUAGUCAGGUCGCUGUGGUGCCUUGGGGCCUCCGCAAAGUGUUGAACUGGCUGAAG
UUUAAGUAUGGCGACCUGCCCAUGUACAUCAUUAGCAACGGGAUUGAUGACGGACUGCACGCUGAGGAUGAUCAACUGAGAGUAUA
CUAUAUGCAGAAUUACAUCAAUGAGGCUUUGAAGGCCCACAUCCUGGAUGGAAUUAACCUGUGCGGGUACUUUGCAUACUCCUUCA
ACGAUAGAACCGCUCCCCGCUUUGGUCUGUAUAGGUACGCUGCCGAUCAGUUCGAGCCCAAAGCCAGCAUGAAGCACUACCGCAAG
AUCAUCGAUAGCAACGGCUUCCCUGGGCCCGAGACUCUCGAGAGGUUUUGCCCCGAGGAGUUCACCGUGUGUACCGAAUGCAGUUU
UUUCCACACAAGGAAAUCAUGA
SEQ ID NO: 24: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGACGGCGCUCAGACAUGGGCCCGGUUCAGCCGGCCACCAGCCCCCGAAGCUGCUGGAUUGUUCC
AGGGAACCUUUCCUGACGGGUUCCUGUGGGCAGUAGGCUCUGCUGCCUAUCAGACUGAGGGUGGAUGGCAGCAGCACGGUAAAGGC
GCAAGCAUUUGGGAUACAUUCACCCACCACCCACUGGCACCCCCAGGCGACAGCCGCAACGCUUCUCUUCCACUGGGAGCCCCCAG
UCCUCUUCAGCCCGCCACAGGCGACGUUGCAUCAGAUAGCUAUAACAAUGUGUUCCGGGACACCGAAGCUCUGAGGGAGCUCGGCG
UGACACACUAUCGGUUCAGCAUCAGUUGGGCCAGGGUGCUGCCAAACGGCAGUGCUGGUGUGCCCAACCGCGAAGGUCUCAGAUAC
UAUCGCCGCCUUCUGGAGAGGCUGAGAGAACUGGGCGUGCAGCCAGUGGUGACUCUGUAUCACUGGGAUCUGCCACAGAGACUGCA
AGAUGCUUAUGGUGGUUGGGCCAACCGCGCCCUGGCAGAUCAUUUCCGGGACUAUGCAGAACUGUGCUUCCGGCACUUUGGAGGAC
AGGUUAAAUAUUGGAUUACUAUCGACAACCCCUAUGUUGUGGCCUGGCACGGGUACGCCACUGGGCGCCUCGCUCCCGGCAUAAGG
GGCUCCCCCAGGCUGGGUUACCUUGUCGCCCACAACCUCUUGCUCGCUCACGCCAAGGUCUGGCAUCUGUACAAUACUUCUUUCCG
CCCCACCCAGGGUGGUCAGGUGUCAAUCGCACUGAGCAGCCACUGGAUUAACCCCAGGCGCAUGACCGAUCAUUCUAUUAAGGAGU
GCCAGAAGAGCCUUGAUUUCGUUCUGGGCUGGUUUGCUAAACCAGUCUUUAUUGACGGUGACUACCCUGAGAGCAUGAAAAAUAAU
CUGUCCAGCAUCUUGCCAGAUUUUACCGAGAGCGAAAAGAAAUUUAUCAAGGGUACCGCUGAUUUUUUCGCUUUGUGUUUCGGCCC
CACAUUGAGCUUCCAGCUGCUGGACCCACACAUGAAGUUUCGCCAGCUGGAGUCACCCAACUUGCGGCAGCUGCUGUCAUGGAUCG
ACCUGGAAUUUAAUCACCCCCAGAUCUUUAUUGUUGAGAAUGGGUGGUUUGUUUCUGGCACUACUAAGAGGGAUGACGCAAAGUAU
AUGUAUUAUCUGAAGAAGUUUAUUAUGGAGACCCUGAAAGCAAUCAAGCUCGAUGGAGUGGAUGUGAUCGGUUAUACCGCUUGGAG
CCUUAUGGAUGGAUUUGAGUGGCACAGGGGGUAUUCAAUAAGGAGAGGCUUGUUCUACGUGGACUUUCUGUCACAGGAUAAAAUGU
UGCUGCCCAAAUCAAGCGCCCUUUUUUACCAGAAAUUGAUAGAAAAGAACGGCUUCCCUCCUCUCCCAGAGAACCAGCCACUGGAG
GGAACAUUUCCUUGCGAUUUCGCAUGGGGAGUGGUGGAUAACUACAUACAGGUGGACACCACUCUGUCUCAGUUUACCGAUCUCAA
UGUGUAUCUGUGGGACGUUCACCAUUCCAAGCGCCUCAUUAAAGUGGAUGGCGUUGUCACUAAGAAAAGAAAGAGCUACUGUGUGG
ACUUUGCUGCCAUUCAGCCACAGAUCGCUCUGCUCCAGGAGAUGCACGUGACCCACUUCCGGUUCUCCCUGGACUGGGCCUUGAUA
CUGCCCUUGGGCAACCAGAGUCAGGUCAAUCAUACCAUCCUGCAGUACUAUCGCUGUAUGGCCUCAGAGCUGGUUCGCGUGAACAU
AACCCCCGUGGUGGCACUGUGGCAGCCCAUGGCACCAAAUCAGGGCCUGCCCAGACUGCUGGCUAGACAGGGCGCCUGGGAAAAUC
CCUACACUGCACUGGCAUUUGCUGAGUACGCCAGGUUGUGUUUUCAGGAGCUGGGACAUCACGUUAAGCUUUGGAUUACCAUGAAC
GAACCAUAUACUAGAAACAUGACCUAUUCCGCCGGACACAAUCUGCUGAAAGCCCACGCCCUGGCAUGGCAUGUGUAUAACGAGAA
AUUCAGGCACGCCCAGAACGGAAAGAUCAGCAUUGCCCUGCAAGCAGACUGGAUUGAGCCAGCCUGUCCUUUCUCACAAAAAGACA
AAGAGGUGGCCGAACGGGUCCUGGAGUUCGACAUCGGCUGGCUCGCUGAACCAAUCUUUGGCAGUGGCGACUACCCUUGGGUUAUG
AGGGACUGGCUCAACCAGAGAAACAACUUUCUUCUUCCCUAUUUUACAGAGGAUGAGAAGAAACUGAUUCAGGGCACCUUCGAUUU
UCUUGCUCUCAGCCAUUAUACUACUAUCCUUGUGGAUAGUGAAAAAGAGGACCCAAUUAAGUACAAUGACUAUCUCGAAGUGCAAG
AGAUGACCGACAUCACCUGGCUGAAUAGCCCAUCCCAGGUCGCAGUGGUGCCUUGGGGCCUGCGGAAGGUGCUGAACUGGCUGAAG
UUUAAGUAUGGCGACCUGCCCAUGUACAUUAUUAGCAACGGGAUAGACGAUGGACUGCACGCAGAAGAUGAUCAGCUGCGCGUGUA
UUAUAUGCAAAAUUAUAUCAACGAAGCCUUGAAGGCCCACAUCCUGGAUGGAAUCAACCUGUGUGGCUAUUUUGCAUACUCAUUCA
ACGACCGCACCGCACCACGGUUUGGUCUGUAUAGGUACGCUGCCGACCAGUUUGAGCCAAAAGCCAGUAUGAAGCACUACCGGAAA
AUCAUCGAUUCUAAUGGCUUCCCUGGCCCUGAGACCCUGGAGAGAUUUUGCCCCGAGGAAUUCACCGUGUGUACAGAAUGUUCAUU
UUUUCACACCCGGAAAAGUUGA
SEQ ID NO: 25: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAACCCGGCGAUGGAGCCCAAACAUGGGCCAGAUUCAGCAGACCUCCCGCUCCAGAAGCUGCUGGUCUGUUUC
AGGGUACGUUUCCUGACGGUUUUCUCUGGGCAGUCGGCUCUGCUGCCUAUCAGACUGAGGGAGGUUGGCAACAACAUGGCAAAGGU
GCUAGCAUAUGGGACACUUUCACGCAUCACCCACUGGCCCCGCCUGGGGAUAGUCGCAACGCAAGUCUGCCACUGGGAGCACCCUC
UCCUCUGCAACCGGCUACCGGGGAUGUGGCUUCCGACUCUUACAACAACGUGUUCAGGGAUACUGAAGCGCUUAGGGAAUUGGGCG
 
UGACGCAUUACCGCUUUAGCAUUAGCUGGGCCAGAGUCUUGCCUAAUGGAUCAGCCGGUGUGCCAAAUCGCGAAGGGCUGCGAUAC
UAUAGGCGGCUGUUGGAGAGGCUCCGGGAACUGGGUGUUCAGCCUGUGGUGACACUCUAUCACUGGGACCUCCCACAGAGACUUCA
AGACGCGUACGGAGGCUGGGCAAAUCGGGCCUUGGCUGAUCAUUUCCGGGACUAUGCAGAGCUGUGUUUCCGUCACUUUGGCGGCC
AGGUAAAGUAUUGGAUUACCAUAGAUAACCCUUAUGUCGUAGCUUGGCAUGGCUAUGCGACAGGGAGACUUGCUCCUGGGAUUCGC
GGAUCUCCCAGGCUGGGAUAUCUGGUCGCUCACAAUCUGUUGCUGGCCCAUGCCAAAGUAUGGCACCUGUACAACACCUCAUUCCG
GCCGACCCAAGGCGGCCAGGUGUCAAUUGCCCUUAGCAGCCACUGGAUUAAUCCCCGCCGAAUGACCGAUCACAGCAUUAAGGAGU
GCCAGAAAUCCCUGGACUUUGUUUUGGGGUGGUUUGCGAAACCGGUGUUCAUCGAUGGGGACUACCCCGAGUCCAUGAAGAACAAU
CUGAGUUCUAUCCUGCCAGACUUCACAGAAUCCGAGAAGAAGUUCAUCAAGGGGACUGCCGACUUCUUCGCACUGUGCUUUGGCCC
CACACUCUCCUUUCAGCUGCUCGAUCCUCACAUGAAAUUUCGCCAGCUUGAGAGCCCAAAUCUCCGUCAGCUGCUUUCAUGGAUAG
ACCUGGAGUUCAACCAUCCCCAGAUCUUUAUCGUCGAAAACGGUUGGUUCGUGAGUGGAACCACAAAGAGGGACGAUGCCAAAUAC
AUGUAUUACCUCAAGAAGUUCAUUAUGGAGACUCUGAAGGCCAUCAAACUCGACGGAGUUGACGUGAUCGGCUAUACUGCAUGGUC
CUUGAUGGAUGGAUUCGAAUGGCAUAGAGGGUACAGCAUCCGAAGAGGCCUGUUUUACGUGGACUUUCUGAGCCAGGACAAGAUGC
UGCUCCCCAAAAGCUCAGCCCUCUUCUACCAGAAACUGAUCGAGAAGAACGGCUUUCCACCCUUGCCCGAGAAUCAGCCUCUGGAG
GGCACAUUCCCCUGUGACUUUGCCUGGGGCGUUGUGGACAAUUACAUUCAGGUGGAUACCACCCUGUCUCAGUUCACCGAUCUGAA
CGUGUACCUCUGGGAUGUUCACCACAGUAAACGUCUGAUCAAGGUCGAUGGGGUGGUUACAAAGAAACGCAAGAGUUAUUGCGUCG
AUUUUGCAGCCAUCCAGCCGCAGAUUGCCCUGCUCCAAGAGAUGCACGUGACACACUUCAGAUUUUCCCUGGAUUGGGCCCUGAUU
CUCCCUCUGGGAAACCAGAGCCAGGUAAACCACACCAUUCUGCAGUACUACCGUUGCAUGGCAAGUGAACUGGUGAGAGUGAACAU
UACUCCAGUGGUAGCUUUGUGGCAGCCUAUGGCCCCUAAUCAAGGACUGCCAAGACUGCUCGCCCGUCAAGGCGCAUGGGAAAAUC
CCUAUACGGCUCUUGCCUUUGCUGAGUAUGCAAGGCUGUGCUUUCAGGAACUGGGACAUCACGUGAAGUUGUGGAUUACCAUGAAU
GAGCCUUAUACUCGAAACAUGACCUAUUCAGCUGGCCAUAAUCUCCUCAAAGCCCAUGCAUUGGCUUGGCACGUCUACAACGAGAA
GUUUCGCCAUGCCCAAAAUGGCAAGAUCAGCAUUGCCCUCCAAGCAGACUGGAUCGAGCCAGCAUGUCCCUUUUCUCAGAAAGACA
AGGAAGUCGCCGAACGGGUGCUGGAGUUUGACAUCGGAUGGCUGGCUGAGCCCAUAUUUGGGAGUGGCGAUUACCCCUGGGUGAUG
AGGGACUGGUUGAACCAGAGGAACAACUUUCUUCUCCCGUAUUUCACAGAGGACGAGAAGAAGCUGAUCCAGGGUACAUUUGACUU
CCUGGCUCUGUCCCAUUACACCACCAUUCUGGUUGAUUCUGAGAAGGAGGAUCCCAUCAAGUACAAUGACUACCUGGAAGUACAGG
AGAUGACGGACAUUACCUGGCUGAAUUCCCCAUCACAGGUUGCUGUGGUUCCAUGGGGUUUGCGCAAAGUGCUGAACUGGCUCAAG
UUCAAAUAUGGGGAUCUCCCGAUGUAUAUUAUCUCUAAUGGCAUAGACGAUGGGCUUCAUGCAGAAGAUGAUCAGCUGAGAGUCUA
CUAUAUGCAGAACUACAUCAACGAAGCGCUUAAAGCGCACAUAUUGGACGGAAUUAAUCUGUGUGGGUACUUCGCCUAUAGCUUCA
AUGAUCGGACUGCUCCCCGGUUUGGCCUGUAUCGCUAUGCCGCGGACCAGUUCGAGCCUAAAGCGAGCAUGAAGCACUACAGGAAA
AUCAUCGACUCAAACGGCUUCCCAGGUCCUGAAACUCUUGAACGGUUUUGUCCCGAGGAAUUCACCGUCUGCACUGAGUGUAGCUU
CUUCCACACACGAAAAUCCUGA
SEQ ID NO: 26: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCCGGCGAUGGGGCCCAAACCUGGGCUCGGUUCAGUAGACCGCCUGCACCUGAAGCUGCAGGGUUGUUCC
AGGGCACUUUUCCGGAUGGGUUUCUCUGGGCGGUAGGGUCCGCGGCAUAUCAGACCGAGGGGGGGUGGCAACAACACGGGAAGGGU
GCGUCAAUAUGGGACACUUUUACUCAUCACCCACUUGCGCCUCCCGGAGAUUCAAGGAAUGCCUCUCUGCCUCUGGGAGCACCCAG
UCCUCUCCAACCCGCAACUGGCGAUGUCGCAUCAGACAGUUACAACAACGUUUUUAGGGACACAGAAGCGCUCCGAGAACUGGGGG
UUACUCAUUAUCGGUUUUCCAUUUCAUGGGCCCGAGUACUCCCAAAUGGGUCCGCUGGAGUACCGAACCGCGAGGGCCUCCGCUAU
UAUAGAAGACUCCUCGAGCGCCUGCGCGAACUCGGUGUUCAACCGGUUGUCACCCUGUACCAUUGGGAUCUCCCGCAACGCCUGCA
AGAUGCUUACGGGGGCUGGGCAAACCGCGCACUGGCGGAUCAUUUUCGAGAUUACGCCGAGCUGUGCUUCCGGCAUUUUGGUGGGC
AAGUCAAGUAUUGGAUCACCAUUGACAACCCAUAUGUCGUUGCAUGGCAUGGAUAUGCAACCGGGAGGCUUGCACCGGGGAUUAGG
GGGAGCCCUAGACUUGGAUACCUGGUCGCCCAUAAUCUGUUGCUCGCUCAUGCGAAGGUCUGGCAUCUGUAUAACACUAGCUUCCG
GCCGACGCAAGGGGGCCAAGUAUCCAUUGCACUGUCUUCCCACUGGAUUAAUCCCAGACGCAUGACCGAUCACAGCAUAAAGGAGU
GUCAAAAGUCCCUGGAUUUUGUCCUGGGCUGGUUUGCAAAGCCCGUCUUCAUAGACGGCGACUACCCUGAGAGCAUGAAGAAUAAC
CUGUCUUCUAUUCUUCCGGAUUUCACGGAGUCAGAAAAAAAGUUUAUCAAGGGCACCGCCGACUUCUUCGCACUGUGUUUCGGACC
GACAUUGUCCUUUCAGCUUUUGGACCCUCAUAUGAAAUUCAGGCAGCUCGAGUCCCCCAAUCUGCGACAGCUGUUGUCAUGGAUUG
AUUUGGAAUUUAACCACCCACAAAUUUUUAUUGUCGAAAAUGGUUGGUUUGUUAGUGGGACUACCAAACGCGACGAUGCGAAGUAC
AUGUACUAUUUGAAGAAGUUUAUAAUGGAGACCCUUAAAGCCAUCAAAUUGGAUGGCGUGGAUGUGAUAGGUUAUACUGCAUGGAG
CUUGAUGGACGGCUUCGAGUGGCAUCGAGGGUACAGCAUAAGGAGGGGUCUUUUCUACGUUGAUUUCUUGUCUCAGGAUAAGAUGC
UUCUGCCAAAAAGUUCAGCGCUUUUUUAUCAGAAAUUGAUAGAAAAGAAUGGUUUUCCGCCGCUUCCAGAAAAUCAGCCCCUCGAA
GGUACGUUUCCGUGCGACUUUGCCUGGGGCGUGGUCGACAACUACAUACAGGUAGACACUACUUUGUCCCAAUUCACUGAUCUCAA
UGUGUAUCUCUGGGAUGUUCAUCACAGCAAGCGACUGAUAAAGGUAGAUGGGGUCGUUACUAAAAAGAGGAAGUCAUACUGCGUCG
AUUUUGCAGCCAUCCAGCCUCAGAUAGCGUUGCUCCAGGAGAUGCACGUGACACAUUUUCGGUUUUCACUGGACUGGGCCCUUAUU
CUGCCGCUCGGGAACCAAUCUCAAGUUAAUCAUACUAUUCUGCAAUAUUACAGGUGCAUGGCGAGCGAGCUUGUUCGAGUAAAUAU
UACGCCUGUGGUUGCACUCUGGCAGCCGAUGGCGCCUAAUCAGGGGCUUCCGAGACUUCUUGCUCGACAAGGUGCUUGGGAGAACC
CAUAUACAGCAUUGGCCUUUGCAGAAUACGCGCGCUUGUGUUUCCAAGAGCUGGGCCAUCAUGUGAAACUUUGGAUAACCAUGAAU
GAGCCUUAUACACGGAACAUGACAUACUCAGCAGGGCACAACUUGCUGAAGGCUCAUGCUCUUGCAUGGCAUGUUUAUAAUGAAAA
AUUUCGACACGCACAAAACGGGAAAAUCAGCAUUGCUCUGCAGGCUGAUUGGAUAGAACCCGCAUGUCCAUUUUCACAAAAAGACA
AGGAAGUAGCGGAGCGCGUACUUGAGUUCGACAUAGGCUGGCUUGCCGAGCCCAUAUUUGGUUCCGGUGAUUAUCCGUGGGUAAUG
CGGGAUUGGCUUAAUCAACGCAACAACUUCCUCCUGCCUUACUUUACAGAGGACGAGAAAAAACUCAUUCAGGGCACCUUUGAUUU
CCUCGCUCUUAGCCAUUAUACAACUAUACUCGUUGACAGUGAAAAGGAAGACCCUAUCAAGUACAACGAUUAUCUUGAAGUCCAGG
AAAUGACAGAUAUAACCUGGCUUAACAGCCCGUCCCAGGUAGCUGUUGUUCCUUGGGGGUUGCGGAAAGUUUUGAAUUGGCUGAAA
UUCAAGUAUGGGGAUCUUCCCAUGUAUAUUAUUUCAAACGGCAUCGAUGAUGGUUUGCAUGCCGAAGAUGACCAACUUAGGGUAUA
UUACAUGCAAAACUACAUCAACGAGGCGCUCAAGGCCCAUAUCCUUGACGGUAUCAACUUGUGCGGUUAUUUUGCCUAUUCCUUUA
AUGAUAGAACAGCCCCUAGGUUCGGUUUGUACCGCUACGCAGCGGACCAAUUUGAGCCGAAAGCGUCAAUGAAACACUAUAGGAAG
AUAAUAGAUAGCAAUGGAUUUCCUGGGCCCGAGACUCUCGAGAGGUUCUGUCCAGAGGAAUUCACAGUCUGCACAGAAUGUAGCUU
UUUUCAUACGAGAAAGUCCUGA
SEQ ID NO: 27: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAACCGGGGGAUGGAGCUCAGACUUGGGCUAGGUUCUCUAGACCUCCAGCUCCCGAAGCUGCAGGACUGUUUC
 
AGGGAACUUUCCCCGAUGGGUUUCUGUGGGCCGUGGGAUCUGCUGCCUACCAAACCGAGGGCGGAUGGCAACAGCAUGGAAAAGGU
GCAUCCAUUUGGGAUACCUUCACCCACCAUCCUCUGGCACCCCCCGGAGAUUCAAGAAAUGCUUCUCUCCCUCUCGGUGCUCCAUC
ACCACUGCAGCCUGCUACAGGAGAUGUUGCCAGUGAUUCAUACAACAAUGUGUUUAGGGACACCGAAGCUUUGCGAGAGCUCGGGG
UCACUCAUUACCGCUUUUCUAUUUCAUGGGCCCGCGUACUUCCAAAUGGAUCUGCGGGAGUCCCAAAUAGAGAGGGCUUGAGAUAU
UACCGAAGGUUGCUGGAACGCCUGAGGGAACUUGGAGUGCAACCCGUAGUGACAUUGUACCACUGGGAUCUCCCCCAGAGGCUUCA
GGAUGCUUACGGUGGUUGGGCUAACAGAGCUCUCGCAGACCACUUCAGAGAUUAUGCAGAACUGUGCUUUCGACAUUUCGGCGGCC
AGGUAAAAUACUGGAUCACCAUAGACAAUCCCUAUGUGGUUGCGUGGCAUGGUUAUGCCACAGGAAGACUGGCUCCCGGUAUCCGG
GGGAGUCCUAGACUUGGCUACCUCGUGGCCCACAAUCUGCUGUUGGCGCACGCUAAGGUGUGGCAUCUGUAUAAUACUAGCUUUAG
ACCGACCCAGGGAGGUCAAGUGAGCAUCGCCCUGUCCUCUCACUGGAUAAAUCCCCGCAGAAUGACCGACCACUCCAUUAAGGAGU
GUCAAAAGUCUCUCGACUUCGUGCUCGGAUGGUUUGCUAAGCCGGUUUUCAUUGAUGGAGACUACCCCGAGAGCAUGAAAAACAAC
UUGAGCUCCAUUCUGCCUGACUUUACAGAAAGCGAAAAAAAAUUUAUCAAGGGUACUGCCGACUUUUUCGCACUCUGCUUUGGCCC
CACUCUUAGCUUUCAGUUGCUCGAUCCACACAUGAAGUUCCGCCAACUGGAGAGUCCUAAUCUGCGCCAACUCCUUUCUUGGAUUG
ACUUGGAGUUCAAUCACCCGCAGAUCUUCAUCGUGGAAAACGGCUGGUUCGUGUCAGGAACCACGAAGCGCGACGAUGCCAAGUAC
AUGUACUACCUCAAAAAGUUCAUCAUGGAAACUCUCAAAGCAAUUAAACUGGACGGCGUCGACGUGAUCGGCUAUACCGCGUGGUC
UCUGAUGGACGGUUUCGAGUGGCAUAGGGGCUAUUCCAUAAGGCGAGGGUUGUUUUACGUUGAUUUUCUCAGCCAAGAUAAAAUGC
UGCUGCCCAAGUCUUCAGCCUUGUUUUAUCAGAAAUUGAUUGAGAAGAAUGGCUUCCCGCCACUCCCCGAGAAUCAACCCCUCGAG
GGGACAUUUCCAUGUGACUUCGCCUGGGGGGUAGUCGAUAAUUACAUCCAAGUGGAUACAACCCUCAGCCAGUUUACCGACCUGAA
CGUUUACUUGUGGGACGUGCACCACAGUAAGAGACUGAUUAAAGUGGAUGGAGUGGUUACUAAAAAGCGGAAGAGUUAUUGUGUAG
AUUUUGCCGCAAUCCAGCCACAAAUCGCCCUGCUGCAGGAGAUGCACGUGACCCAUUUCAGAUUUAGUCUCGACUGGGCUCUGAUU
UUGCCCUUGGGCAAUCAGAGCCAGGUGAAUCAUACAAUCCUCCAGUACUAUCGGUGUAUGGCCAGUGAAUUGGUCCGAGUCAACAU
CACCCCAGUGGUGGCACUCUGGCAGCCAAUGGCUCCUAACCAAGGGCUGCCAAGACUGCUGGCAAGGCAGGGUGCCUGGGAGAAUC
CUUACACUGCUCUGGCCUUUGCCGAGUACGCUCGCCUUUGUUUUCAGGAGCUCGGGCAUCAUGUGAAACUGUGGAUUACAAUGAAU
GAACCAUAUACUCGCAACAUGACCUAUUCCGCAGGUCACAAUCUCCUCAAGGCUCAUGCACUCGCAUGGCACGUCUACAAUGAAAA
AUUCCGACACGCCCAGAAUGGGAAGAUUAGUAUCGCCCUGCAGGCUGAUUGGAUCGAGCCCGCAUGUCCAUUUUCACAAAAGGACA
AAGAGGUAGCCGAGAGAGUGCUGGAGUUCGAUAUCGGCUGGCUUGCCGAACCUAUUUUCGGCUCUGGCGAUUACCCCUGGGUGAUG
AGGGAUUGGUUGAACCAGAGGAACAAUUUCCUCCUGCCGUAUUUUACCGAGGACGAAAAGAAGUUGAUCCAAGGCACAUUCGAUUU
CCUCGCCCUUUCUCACUACACAACCAUACUUGUGGAUAGCGAGAAGGAGGACCCAAUUAAAUAUAAUGACUAUCUGGAAGUUCAGG
AGAUGACUGAUAUCACAUGGCUGAAUUCCCCCAGCCAAGUAGCAGUCGUGCCCUGGGGUCUGAGAAAGGUGCUUAAUUGGUUGAAG
UUUAAGUACGGUGAUCUGCCUAUGUAUAUAAUUUCUAACGGCAUAGAUGAUGGGCUGCACGCUGAGGACGAUCAACUGCGAGUGUA
CUACAUGCAGAAUUAUAUCAACGAAGCCUUGAAAGCCCACAUUCUGGACGGCAUCAAUCUUUGUGGCUACUUCGCUUAUUCAUUCA
AUGAUAGAACAGCCCCCCGCUUUGGAUUGUAUCGGUAUGCCGCCGACCAAUUCGAGCCAAAGGCUUCUAUGAAGCACUACCGGAAG
AUUAUCGACUCCAACGGGUUUCCAGGGCCAGAGACACUGGAAAGGUUUUGCCCAGAGGAGUUCACCGUGUGUACAGAGUGCAGCUU
UUUCCACACCCGGAAAUCAUGA
SEQ ID NO: 28: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAACCAGGAGAUGGUGCCCAAACUUGGGCUAGGUUCUCUCGUCCACCCGCACCUGAAGCUGCUGGGCUGUUUC
AAGGUACUUUUCCUGAUGGAUUUCUUUGGGCUGUCGGGUCCGCUGCUUAUCAAACUGAAGGUGGAUGGCAACAACAUGGGAAAGGC
GCUUCAAUUUGGGACACCUUUACACAUCAUCCUCUCGCCCCACCUGGUGAUAGUCGCAAUGCGAGCUUACCUCUCGGAGCACCAUC
UCCCUUGCAACCAGCAACAGGCGAUGUGGCUUCAGAUUCCUAUAAUAAUGUGUUUAGAGAUACUGAAGCUCUCAGAGAAUUGGGAG
UGACCCAUUAUAGAUUUUCUAUUUCUUGGGCCCGGGUCCUGCCUAACGGGUCCGCCGGGGUUCCUAAUCGAGAAGGUUUGCGGUAU
UAUAGAAGGCUUCUCGAAAGGUUGCGAGAACUUGGAGUACAACCUGUCGUGACUUUGUAUCAUUGGGAUCUUCCGCAAAGACUUCA
AGAUGCAUAUGGUGGAUGGGCAAAUCGUGCACUCGCUGAUCAUUUUCGGGACUAUGCCGAACUUUGUUUUAGGCAUUUUGGCGGCC
AAGUUAAAUAUUGGAUUACAAUAGAUAAUCCUUAUGUAGUUGCAUGGCAUGGUUAUGCAACAGGCCGGUUGGCACCAGGGAUUCGA
GGUUCACCACGACUUGGAUAUUUAGUCGCCCAUAAUCUUCUUUUGGCCCACGCGAAGGUAUGGCACUUAUAUAACACCUCUUUUAG
GCCGACGCAAGGUGGACAAGUCUCUAUCGCAUUGUCCUCACAUUGGAUUAACCCCAGGCGUAUGACAGAUCAUUCUAUUAAGGAAU
GCCAGAAGAGUCUCGAUUUCGUGUUAGGCUGGUUCGCAAAGCCAGUCUUCAUCGACGGAGAUUACCCGGAAUCAAUGAAGAACAAU
CUGAGCAGUAUCCUUCCCGACUUCACCGAGAGUGAAAAGAAAUUUAUUAAGGGCACCGCAGAUUUCUUCGCCCUGUGUUUCGGCCC
GACUCUCUCCUUCCAGCUGCUCGAUCCACAUAUGAAAUUUCGGCAGCUCGAGUCACCUAAUUUAAGACAGCUCCUGAGUUGGAUCG
AUCUGGAGUUCAAUCACCCACAGAUCUUCAUCGUUGAGAACGGGUGGUUCGUGUCUGGCACGACUAAACGCGACGACGCUAAGUAC
AUGUACUAUCUGAAGAAAUUUAUAAUGGAGACACUUAAGGCUAUAAAAUUGGACGGCGUAGACGUGAUUGGUUACACUGCUUGGAG
CCUGAUGGACGGCUUUGAAUGGCAUCGUGGGUAUUCAAUACGGCGGGGCUUAUUUUACGUGGAUUUCUUGUCUCAAGAUAAAAUGC
UGCUGCCCAAAAGCAGUGCACUUUUCUAUCAGAAACUCAUCGAAAAGAACGGAUUUCCACCCUUGCCAGAGAACCAACCUCUGGAA
GGAACCUUCCCAUGCGAUUUCGCGUGGGGUGUGGUGGAUAAUUAUAUCCAGGUGGACACUACCUUAAGUCAAUUCACAGAUCUCAA
CGUGUAUUUAUGGGACGUGCAUCAUAGCAAGAGACUGAUCAAGGUCGACGGUGUGGUUACUAAGAAGCGCAAGAGCUAUUGCGUCG
AUUUCGCAGCAAUUCAACCACAAAUUGCCCUGCUGCAAGAGAUGCAUGUGACCCACUUCAGAUUUAGUCUCGAUUGGGCAUUGAUC
CUGCCCCUCGGAAAUCAAUCACAAGUAAAUCAUACUAUACUCCAAUAUUACAGGUGUAUGGCUUCCGAACUGGUUCGAGUAAAUAU
UACACCUGUCGUUGCUUUGUGGCAACCGAUGGCACCUAAUCAGGGGCUUCCAAGGCUGCUCGCAAGACAAGGAGCGUGGGAAAAUC
CAUAUACAGCGUUAGCUUUCGCUGAAUACGCUAGGCUCUGUUUCCAGGAAUUGGGUCACCAUGUGAAAUUGUGGAUCACUAUGAAC
GAACCUUACACUCGGAACAUGACCUAUUCUGCAGGACAUAAUCUCCUCAAAGCUCACGCAUUAGCCUGGCACGUUUAUAACGAGAA
AUUCCGACACGCCCAAAACGGUAAGAUUUCUAUUGCGCUUCAAGCCGACUGGAUUGAGCCCGCUUGUCCCUUUAGCCAGAAAGAUA
AGGAAGUAGCAGAACGAGUACUGGAGUUCGAUAUAGGAUGGCUCGCGGAACCGAUAUUUGGAUCCGGCGACUACCCGUGGGUCAUG
CGAGAUUGGCUCAAUCAGCGCAAUAACUUCCUGCUCCCAUACUUUACAGAGGACGAGAAGAAACUCAUUCAAGGCACGUUCGAUUU
CCUUGCCCUGUCCCACUACACGACGAUUCUCGUGGAUUCCGAGAAAGAGGACCCUAUCAAGUAUAACGACUAUUUGGAAGUCCAGG
AGAUGACUGAUAUAACUUGGCUGAAUUCACCUUCCCAAGUCGCCGUUGUCCCGUGGGGUCUGAGAAAGGUACUUAAUUGGUUGAAA
UUUAAAUAUGGCGAUCUUCCUAUGUAUAUCAUUAGCAACGGGAUUGACGAUGGCUUGCACGCAGAAGAUGAUCAAUUGCGUGUCUA
CUACAUGCAAAACUAUAUCAAUGAGGCCCUGAAGGCUCAUAUCCUCGACGGCAUUAACUUGUGUGGGUAUUUCGCAUACUCAUUCA
AUGAUAGAACCGCACCUCGCUUCGGGCUUUACCGCUACGCCGCUGACCAAUUCGAACCUAAAGCCUCAAUGAAGCACUAUAGAAAG
AUCAUCGAUUCCAACGGGUUUCCCGGGCCUGAGACACUUGAGAGGUUCUGCCCCGAGGAGUUUACGGUCUGCACAGAAUGUAGCUU
CUUCCAUACACGGAAAUCCUGA
SEQ ID NO: 29: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 30: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
 
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAA
SEQ ID NO: 31: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
 
SEQ ID NO: 32:
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
 
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAA
SEQ ID NO: 33: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 34: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 35: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
 
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 36: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 37: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 38: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 39: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
 
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 40: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 41: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 42: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
 
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 43: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 44: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 45: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
 
CUUUGAAUAAAGUCUGAGUGGGCGGCAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAA
SEQ ID NO: 46: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAA
SEQ ID NO: 47: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAA
SEQ ID NO: 48: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
 
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
SEQ ID NO: 64: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 65: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
 
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 66: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 67: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
 
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 68: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 69: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
 
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 70: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 71: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
 
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 72: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 73: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
 
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAA
SEQ ID NO: 74: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
 
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 75: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 79: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 80: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
 
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUC
SEQ ID NO: 81: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 82: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUC
SEQ ID NO: 83: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 84: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
 
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 85: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 86: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 87: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
 
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGAUCUGCCCAUGUAUAUCAUCAGCAACGGCAUCGACGACGGCCUGCACGCCGAGGACGACCAGCUGA
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 88: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGAUCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 89: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
 
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGAUCUGCCCAUGUAUAUCAUCAGCAACGGCAUCGACGACGGCCUGCACGCCGAGGACGACCAGCUGA
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 90: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGAUCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 91: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
 
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
AUCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 92: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
AUCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 93: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
 
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
AUCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 94: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
AUCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 95: 
AUGCCCGCCAGCGCCCCGCCGCGCCGCCCGCGGCCGCCGCCGUCGCUGUCGCUGCUGGUGCUGCUGGGCCUGGGCGGCCG
CCGCCUGCGUGCGGAGCCGGGCGACGGCGCGCAGACCUGGGCCCGUUUCUCGCGGCCUCCUGCCCCCGAGGCCGCGGGCCUCUUCC
AGGGCACCUUCCCCGACGGCUUCCUCUGGGCCGUGGGCAGCGCCGCCUACCAGACCGAGGGCGGCUGGCAGCAGCACGGCAAGGGU
GCGUCCAUCUGGGACACGUUCACCCACCACCCCCUGGCACCCCCGGGAGACUCCCGGAACGCCAGUCUGCCGUUGGGCGCCCCGUC
GCCGCUGCAGCCCGCCACCGGGGACGUAGCCAGCGACAGCUACAACAACGUCUUCCGCGACACGGAGGCGCUGCGCGAGCUCGGGG
UCACUCACUACCGCUUCUCCAUCUCGUGGGCGCGAGUGCUCCCCAAUGGCAGCGCGGGCGUCCCCAACCGCGAGGGGCUGCGCUAC
UACCGGCGCCUGCUGGAGCGGCUGCGGGAGCUGGGCGUGCAGCCCGUGGUCACCCUGUACCACUGGGAUCUGCCCCAGCGCCUGCA
GGACGCCUACGGCGGCUGGGCCAACCGCGCCCUGGCCGACCACUUCAGGGAUUACGCGGAGCUCUGCUUCCGCCACUUCGGCGGUC
AGGUCAAGUACUGGAUCACCAUCGACAACCCCUACGUGGUGGCCUGGCACGGCUACGCCACCGGGCGCCUGGCCCCCGGCAUCCGG
GGCAGCCCGCGGCUCGGGUACCUGGUGGCGCACAACCUCCUCCUGGCUCAUGCCAAAGUCUGGCAUCUCUACAAUACUUCUUUCCG
UCCCACUCAGGGAGGUCAGGUGUCCAUUGCCCUAAGCUCUCACUGGAUCAAUCCUCGAAGAAUGACCGACCACAGCAUCAAAGAAU
GUCAAAAAUCUCUGGACUUUGUACUAGGUUGGUUUGCCAAACCCGUAUUUAUUGAUGGUGACUAUCCCGAGAGCAUGAAGAAUAAC
CUUUCAUCUAUUCUGCCUGAUUUUACUGAAUCUGAGAAAAAGUUCAUCAAAGGAACUGCUGACUUUUUUGCUCUUUGCUUUGGACC
CACCUUGAGUUUUCAACUUUUGGACCCUCACAUGAAGUUCCGCCAAUUGGAAUCUCCCAACCUGAGGCAACUGCUUUCCUGGAUUG
ACCUUGAAUUUAACCAUCCUCAAAUAUUUAUUGUGGAAAAUGGCUGGUUUGUCUCAGGGACCACCAAGAGAGAUGAUGCCAAAUAU
AUGUAUUACCUCAAAAAGUUCAUCAUGGAAACCUUAAAAGCCAUCAAGCUGGAUGGGGUGGAUGUCAUCGGGUAUACCGCAUGGUC
CCUCAUGGAUGGUUUCGAGUGGCACAGAGGUUACAGCAUCAGGCGUGGACUCUUCUAUGUUGACUUUCUAAGCCAGGACAAGAUGU
UGUUGCCAAAGUCUUCAGCCUUGUUCUACCAAAAGCUGAUAGAGAAAAAUGGCUUCCCUCCUUUACCUGAAAAUCAGCCCCUAGAA
GGGACAUUUCCCUGUGACUUUGCUUGGGGAGUUGUUGACAACUACAUUCAAGUAGAUACCACUCUGUCUCAGUUUACCGACCUGAA
UGUUUACCUGUGGGAUGUCCACCACAGUAAAAGGCUUAUUAAAGUGGAUGGGGUUGUGACCAAGAAGAGGAAAUCCUACUGUGUUG
ACUUUGCUGCCAUCCAGCCCCAGAUCGCUUUACUCCAGGAAAUGCACGUUACACAUUUUCGCUUCUCCCUGGACUGGGCCCUGAUU
CUCCCUCUGGGUAACCAGUCCCAGGUGAACCACACCAUCCUGCAGUACUAUCGCUGCAUGGCCAGCGAGCUUGUCCGUGUCAACAU
CACCCCAGUGGUGGCCCUGUGGCAGCCUAUGGCCCCGAACCAAGGACUGCCGCGCCUCCUGGCCAGGCAGGGCGCCUGGGAGAACC
 
CCUACACUGCCCUGGCCUUUGCAGAGUAUGCCCGACUGUGCUUUCAAGAGCUCGGCCAUCACGUCAAGCUUUGGAUAACGAUGAAU
GAGCCGUAUACAAGGAAUAUGACAUACAGUGCUGGCCACAACCUUCUGAAGGCCCAUGCCCUGGCUUGGCAUGUGUACAAUGAAAA
GUUUAGGCAUGCUCAGAAUGGGAAAAUAUCCAUAGCCUUGCAGGCUGAUUGGAUAGAACCAGCCUGCCCUUUCUCCCAAAAGGACA
AAGAGGUGGCCGAGAGAGUUUUGGAAUUUGACAUUGGCUGGCUGGCUGAGCCCAUUUUCGGCUCUGGAGAUUAUCCAUGGGUGAUG
AGGGACUGGCUGAACCAAAGAAACAAUUUUCUUCUUCCUUAUUUCACUGAAGAUGAAAAAAAGCUAAUCCAGGGUACCUUUGACUU
UUUGGCUUUAAGCCAUUAUACCACCAUCCUUGUAGACUCAGAAAAAGAAGAUCCAAUAAAAUACAAUGAUUACCUAGAAGUGCAAG
AAAUGACCGACAUCACGUGGCUCAACUCCCCCAGUCAGGUGGCGGUAGUGCCCUGGGGGUUGCGCAAAGUGCUGAACUGGCUGAAG
UUCAAGUACGGAGACCUCCCCAUGUACAUAAUAUCCAACGGAAUCGAUGACGGGCUGCAUGCUGAGGACGACCAGCUGAGGGUGUA
UUAUAUGCAGAAUUACAUAAACGAAGCUCUCAAAGCCCACAUACUGGAUGGUAUCAAUCUUUGCGGAUACUUUGCUUAUUCGUUUA
ACGACCGCACAGCUCCGAGGUUUGGCCUCUAUCGUUAUGCUGCAGAUCAGUUUGAGCCCAAGGCAUCCAUGAAACAUUACAGGAAA
AUUAUUGACAGCAAUGGUUUCCCGGGCCCAGAAACUCUGGAAAGAUUUUGUCCAGAAGAAUUCACCGUGUGUACUGAGUGCAGUUU
UUUUCACACCCGAAAGUCUUGA
SEQ ID NO: 96: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGAUCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCUGA
SEQ ID NO: 97: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
 
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGAUCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 98: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGAUCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCUGA
SEQ ID NO: 99: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
 
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGAUCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 100: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
UCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
SEQ ID NO: 101: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
 
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
UCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAA
SEQ ID NO: 102: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
UCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 103: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
 
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
UCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAA
SEQ ID NO: 104: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 105: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
 
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 106: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGAUCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGCCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAAUCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAAUCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 107: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAGCCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGAUCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAAUCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAAUCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 108: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 109: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
 
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 110: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 111: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 112: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
 
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 113: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 114: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 115: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 116: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
 
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
SEQ ID NO: 117: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAA
SEQ ID NO: 118: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
SEQ ID NO: 119: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAA
SEQ ID NO: 120: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
AUCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 121: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
 
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
AUCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 122: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGAUCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGCCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAAUCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAAUCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
AUCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 123: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAGCCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGAUCUGCCCCAGCGCCUGCAGGACGCUUACGGC
 
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAAUCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAAUCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
AUCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 124: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGAUCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 125: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
 
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGAUCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 126: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGAUCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGCC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAAUCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAAUCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGAUCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
 
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 127: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAGCCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGAUCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAAUCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAAUCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGAUCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 128: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
 
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
UCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAA
SEQ ID NO: 129: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
UCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 130: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGAUCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGCCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAAUCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAAUCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
 
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
UCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 131: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAGCCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGAUCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAAUCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAAUCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
UCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAA
SEQ ID NO: 132: 
ATGCCCGCCAGCGCCCCCCCCCGCCGCCCCCGCCCCCCCCCCCCCAGCCTGAGCCTGCTGCTGGTGCTGCTGGGCCTGGGCGGCCG
CCGCCTGCGCGCCGAGCCCGGCGACGGCGCCCAGACCTGGGCCCGCTTCAGCCGCCCCCCCGCCCCCGAGGCCGCCGGCCTGTTCC
AGGGCACCTTCCCCGACGGCTTCCTGTGGGCCGTGGGCAGCGCCGCCTACCAGACCGAGGGCGGCTGGCAGCAGCACGGCAAGGGC
GCCAGCATCTGGGACACCTTCACCCACCACCCCCTGGCCCCCCCCGGCGACAGCCGCAACGCCAGCCTGCCCCTGGGCGCCCCCAG
CCCCCTGCAGCCCGCCACCGGCGACGTGGCCAGCGACAGCTACAACAACGTGTTCCGCGACACCGAGGCCCTGCGCGAGCTGGGCG
TGACCCACTACCGCTTCAGCATCAGCTGGGCCCGCGTGCTGCCCAACGGCAGCGCCGGCGTGCCCAACCGCGAGGGCCTGCGCTAC
TACCGCCGCCTGCTGGAGCGCCTGCGCGAGCTGGGCGTGCAGCCCGTGGTGACCCTGTACCACTGGGACCTGCCCCAGCGCCTGCA
 
GGACGCCTACGGCGGCTGGGCCAACCGCGCCCTGGCCGACCACTTCCGCGACTACGCCGAGCTGTGCTTCCGCCACTTCGGCGGCC
AGGTGAAGTACTGGATCACCATCGACAACCCCTACGTGGTGGCCTGGCACGGCTACGCCACCGGCCGCCTGGCCCCCGGCATCCGC
GGCAGCCCCCGCCTGGGCTACCTGGTGGCCCACAACCTGCTGCTGGCCCACGCCAAGGTGTGGCACCTGTACAACACCAGCTTCCG
CCCCACCCAGGGCGGCCAGGTGAGCATCGCCCTGAGCAGCCACTGGATCAACCCCCGCCGCATGACCGACCACAGCATCAAGGAGT
GCCAGAAGAGCCTGGACTTCGTGCTGGGCTGGTTCGCCAAGCCCGTGTTCATCGACGGCGACTACCCCGAGAGCATGAAGAACAAC
CTGAGCAGCATCCTGCCCGACTTCACCGAGAGCGAGAAGAAGTTCATCAAGGGCACCGCCGACTTCTTCGCCCTGTGCTTCGGCCC
CACCCTGAGCTTCCAGCTGCTGGACCCCCACATGAAGTTCCGCCAGCTGGAGAGCCCCAACCTGCGCCAGCTGCTGAGCTGGATCG
ACCTGGAGTTCAACCACCCCCAGATCTTCATCGTGGAGAACGGCTGGTTCGTGAGCGGCACCACCAAGCGCGACGACGCCAAGTAC
ATGTACTACCTGAAGAAGTTCATCATGGAGACCCTGAAGGCCATCAAGCTGGACGGCGTGGACGTGATCGGCTACACCGCCTGGAG
CCTGATGGACGGCTTCGAGTGGCACCGCGGCTACAGCATCCGCCGCGGCCTGTTCTACGTGGACTTCCTGAGCCAGGACAAGATGC
TGCTGCCCAAGAGCAGCGCCCTGTTCTACCAGAAGCTGATCGAGAAGAACGGCTTCCCCCCCCTGCCCGAGAACCAGCCCCTGGAG
GGCACCTTCCCCTGCGACTTCGCCTGGGGCGTGGTGGACAACTACATCCAGGTGGACACCACCCTGAGCCAGTTCACCGACCTGAA
CGTGTACCTGTGGGACGTGCACCACAGCAAGCGCCTGATCAAGGTGGACGGCGTGGTGACCAAGAAGCGCAAGAGCTACTGCGTGG
ACTTCGCCGCCATCCAGCCCCAGATCGCCCTGCTGCAGGAGATGCACGTGACCCACTTCCGCTTCAGCCTGGACTGGGCCCTGATC
CTGCCCCTGGGCAACCAGAGCCAGGTGAACCACACCATCCTGCAGTACTACCGCTGCATGGCCAGCGAGCTGGTGCGCGTGAACAT
CACCCCCGTGGTGGCCCTGTGGCAGCCCATGGCCCCCAACCAGGGCCTGCCCCGCCTGCTGGCCCGCCAGGGCGCCTGGGAGAACC
CCTACACCGCCCTGGCCTTCGCCGAGTACGCCCGCCTGTGCTTCCAGGAGCTGGGCCACCACGTGAAGCTGTGGATCACCATGAAC
GAGCCCTACACCCGCAACATGACCTACAGCGCCGGCCACAACCTGCTGAAGGCCCACGCCCTGGCCTGGCACGTGTACAACGAGAA
GTTCCGCCACGCCCAGAACGGCAAGATCAGCATCGCCCTGCAGGCCGACTGGATCGAGCCCGCCTGCCCCTTCAGCCAGAAGGACA
AGGAGGTGGCCGAGCGCGTGCTGGAGTTCGACATCGGCTGGCTGGCCGAGCCCATCTTCGGCAGCGGCGACTACCCCTGGGTGATG
CGCGACTGGCTGAACCAGCGCAACAACTTCCTGCTGCCCTACTTCACCGAGGACGAGAAGAAGCTGATCCAGGGCACCTTCGACTT
CCTGGCCCTGAGCCACTACACCACCATCCTGGTGGACAGCGAGAAGGAGGACCCCATCAAGTACAACGACTACCTGGAGGTGCAGG
AGATGACCGACATCACCTGGCTGAACAGCCCCAGCCAGGTGGCCGTGGTGCCCTGGGGCCTGCGCAAGGTGCTGAACTGGCTGAAG
TTCAAGTACGGCGACCTGCCCATGTACATCATCAGCAACGGCATCGACGACGGCCTGCACGCCGAGGACGACCAGCTGCGCGTGTA
CTACATGCAGAACTACATCAACGAGGCCCTGAAGGCCCACATCCTGGACGGCATCAACCTGTGCGGCTACTTCGCCTACAGCTTCA
ACGACCGCACCGCCCCCCGCTTCGGCCTGTACCGCTACGCCGCCGACCAGTTCGAGCCCAAGGCCAGCATGAAGCACTACCGCAAG
ATCATCGACAGCAACGGCTTCCCCGGCCCCGAGACCCTGGAGCGCTTCTGCCCCGAGGAGTTCACCGTGTGCACCGAGTGCAGCTT
CTTCCACACCCGCAAGAGCTAG
SEQ ID NO: 133: 
ATGCCGGCGTCGGCGCCGCCGCGTCGACCGCGTCCGCCGCCGCCGTCGCTATCGCTATTATTAGTACTACTAGGTCTAGGTGGTCG
TCGTCTACGTGCGGAACCGGGTGATGGTGCGCAAACGTGGGCGCGATTTTCGCGACCGCCGGCGCCGGAAGCGGCGGGTTTATTTC
AAGGTACGTTTCCGGATGGTTTTTTATGGGCGGTAGGTTCGGCGGCGTATCAAACGGAAGGTGGTTGGCAACAACATGGTAAAGGT
GCGTCGATATGGGATACGTTTACGCATCATCCGTTAGCGCCGCCGGGTGATTCGCGAAATGCGTCGCTACCGCTAGGTGCGCCGTC
GCCGTTACAACCGGCGACGGGTGATGTTGCGTCGGATTCGTATAATAATGTATTTCGTGATACGGAAGCGCTACGTGAACTAGGTG
TTACGCATTATCGTTTTTCGATATCGTGGGCGCGTGTATTACCGAATGGTTCGGCGGGTGTACCGAATCGTGAAGGTTTACGATAT
TATCGACGTTTATTAGAACGTCTACGTGAACTAGGTGTTCAACCGGTAGTTACGCTATATCATTGGGATCTACCGCAACGTTTACA
AGATGCGTATGGTGGTTGGGCGAATCGAGCGCTAGCGGATCATTTTCGTGATTATGCGGAACTATGTTTTCGACATTTTGGTGGTC
AAGTAAAATATTGGATAACGATAGATAATCCGTATGTTGTAGCGTGGCATGGTTATGCGACGGGTCGTCTAGCGCCGGGTATACGT
GGTTCGCCGCGATTAGGTTATTTAGTAGCGCATAATCTATTATTAGCGCATGCGAAAGTTTGGCATCTATATAATACGTCGTTTCG
TCCGACGCAAGGTGGTCAAGTTTCGATAGCGTTATCGTCGCATTGGATAAATCCGCGTCGTATGACGGATCATTCGATAAAAGAAT
GTCAAAAATCGCTAGATTTTGTACTAGGTTGGTTTGCGAAACCGGTTTTTATAGATGGTGATTATCCGGAATCGATGAAAAATAAT
CTATCGTCGATATTACCGGATTTTACGGAATCGGAAAAAAAATTTATAAAAGGTACGGCGGATTTTTTTGCGTTATGTTTTGGTCC
GACGTTATCGTTTCAACTATTAGATCCGCATATGAAATTTCGACAATTAGAATCGCCGAATTTACGTCAACTATTATCGTGGATAG
ATTTAGAATTTAATCATCCGCAAATATTTATAGTAGAAAATGGTTGGTTTGTTTCGGGTACGACGAAACGTGATGATGCGAAATAT
ATGTATTATTTAAAAAAATTTATAATGGAAACGTTAAAAGCGATAAAATTAGATGGTGTAGATGTAATAGGTTATACGGCGTGGTC
GTTAATGGATGGTTTTGAATGGCATCGAGGTTATTCGATACGTCGTGGTCTATTTTATGTTGATTTTCTATCGCAAGATAAAATGT
TATTACCGAAATCGTCGGCGTTATTTTATCAAAAATTAATAGAAAAAAATGGTTTTCCGCCGCTACCGGAAAATCAACCGTTAGAA
GGTACGTTTCCGTGTGATTTTGCGTGGGGTGTAGTAGATAATTATATACAAGTAGATACGACGTTATCGCAATTTACGGATCTAAA
TGTATATCTATGGGATGTTCATCATTCGAAACGTTTAATAAAAGTAGATGGTGTTGTAACGAAAAAACGTAAATCGTATTGTGTAG
ATTTTGCGGCGATACAACCGCAAATAGCGCTATTACAAGAAATGCATGTAACGCATTTTCGTTTTTCGTTAGATTGGGCGTTAATA
CTACCGTTAGGTAATCAATCGCAAGTTAATCATACGATATTACAATATTATCGTTGTATGGCGTCGGAATTAGTTCGAGTTAATAT
AACGCCGGTAGTTGCGTTATGGCAACCGATGGCGCCGAATCAAGGTTTACCGCGATTACTAGCGCGACAAGGTGCGTGGGAAAATC
CGTATACGGCGTTAGCGTTTGCGGAATATGCGCGATTATGTTTTCAAGAATTAGGTCATCATGTAAAATTATGGATAACGATGAAT
GAACCGTATACGCGAAATATGACGTATTCGGCGGGTCATAATCTATTAAAAGCGCATGCGCTAGCGTGGCATGTATATAATGAAAA
ATTTCGACATGCGCAAAATGGTAAAATATCGATAGCGTTACAAGCGGATTGGATAGAACCGGCGTGTCCGTTTTCGCAAAAAGATA
AAGAAGTAGCGGAACGTGTATTAGAATTTGATATAGGTTGGCTAGCGGAACCGATATTTGGTTCGGGTGATTATCCGTGGGTAATG
CGTGATTGGTTAAATCAACGTAATAATTTTTTATTACCGTATTTTACGGAAGATGAAAAAAAATTAATACAAGGTACGTTTGATTT
TCTAGCGTTATCGCATTATACGACGATACTAGTTGATTCGGAAAAAGAAGATCCGATAAAATATAATGATTATTTAGAAGTACAAG
AAATGACGGATATAACGTGGCTAAATTCGCCGTCGCAAGTAGCGGTTGTTCCGTGGGGTTTACGAAAAGTATTAAATTGGCTAAAA
TTTAAATATGGTGATTTACCGATGTATATAATATCGAATGGTATAGATGATGGTTTACATGCGGAAGATGATCAATTACGAGTATA
TTATATGCAAAATTATATAAATGAAGCGCTAAAAGCGCATATATTAGATGGTATAAATCTATGTGGTTATTTTGCGTATTCGTTTA
ATGATCGAACGGCGCCGCGTTTTGGTTTATATCGATATGCGGCGGATCAATTTGAACCGAAAGCGTCGATGAAACATTATCGAAAA
ATAATAGATTCGAATGGTTTTCCGGGTCCGGAAACGTTAGAACGTTTTTGTCCGGAAGAATTTACGGTATGTACGGAATGTTCGTT
TTTTCATACGCGAAAATCGTAG
SEQ ID NO: 134: 
ATGCCCGCATCTGCACCACCCAGACGCCCTAGGCCTCCCCCACCCTCGTTGAGCCTGCTGCTGGTGCTCCTGGGGCTCGGCGGACG
GAGGTTGAGGGCAGAACCTGGAGATGGTGCTCAAACCTGGGCTAGATTCAGTAGGCCTCCAGCCCCTGAGGCCGCTGGCCTATTCC
AGGGGACTTTTCCCGACGGATTTCTGTGGGCAGTGGGCAGCGCCGCTTACCAGACAGAAGGCGGATGGCAGCAGCACGGCAAAGGC
GCATCCATTTGGGATACTTTTACGCACCACCCATTGGCTCCACCCGGAGACTCTAGGAACGCCTCTCTACCCCTGGGAGCGCCAAG
 
CCCCCTGCAGCCGGCTACGGGAGACGTTGCCTCTGACTCCTATAACAACGTGTTCCGGGATACCGAGGCCCTGCGGGAGTTGGGGG
TGACACATTACCGGTTCAGTATTTCATGGGCACGGGTCCTGCCGAATGGATCAGCAGGCGTGCCCAACCGAGAGGGCCTGAGGTAT
TACAGACGGCTTCTCGAGCGGTTGAGGGAACTGGGAGTCCAGCCAGTCGTAACCTTGTACCACTGGGATCTGCCACAGAGGTTACA
AGATGCTTATGGCGGATGGGCTAATCGGGCACTTGCAGATCACTTCCGGGATTATGCAGAACTCTGTTTCAGACATTTTGGTGGTC
AAGTAAAATACTGGATTACCATCGATAATCCATACGTGGTAGCTTGGCACGGTTATGCCACTGGACGGCTGGCTCCAGGGATACGC
GGATCCCCTCGCCTCGGGTATCTCGTGGCCCATAACTTACTCCTGGCGCACGCCAAGGTCTGGCATCTCTACAATACGAGCTTTCG
GCCTACTCAAGGAGGGCAGGTCTCAATTGCCCTGTCTTCACACTGGATTAATCCTCGGCGAATGACCGACCACTCTATAAAGGAGT
GCCAAAAGTCTCTTGATTTCGTTCTCGGCTGGTTTGCCAAGCCTGTGTTCATCGATGGGGATTACCCAGAGTCTATGAAGAATAAC
CTGAGCAGCATTTTGCCCGATTTTACCGAATCAGAGAAGAAATTCATAAAGGGTACTGCCGACTTTTTTGCCCTGTGCTTTGGTCC
GACTTTATCATTCCAACTGCTCGATCCGCACATGAAATTCCGGCAGCTGGAGAGCCCTAATCTGAGGCAGCTGTTGTCTTGGATAG
ACCTGGAATTTAACCATCCACAGATCTTTATTGTAGAGAATGGCTGGTTCGTGTCCGGTACTACCAAGAGGGACGATGCCAAGTAC
ATGTATTATCTGAAAAAGTTCATCATGGAAACTCTGAAGGCTATCAAATTGGACGGAGTGGATGTCATTGGCTACACCGCTTGGTC
TTTGATGGATGGGTTTGAATGGCACCGGGGATATTCTATACGCCGAGGCCTGTTTTATGTCGATTTCCTGAGCCAGGACAAGATGC
TGCTGCCCAAGAGCTCAGCGCTCTTTTACCAGAAGCTTATAGAGAAGAACGGATTTCCACCATTACCTGAAAATCAGCCACTGGAA
GGGACATTCCCTTGCGATTTCGCGTGGGGGGTCGTTGACAACTATATACAGGTCGATACCACTCTTAGCCAATTTACAGACTTAAA
TGTGTACCTCTGGGATGTTCATCATTCAAAGCGTCTCATCAAGGTGGACGGAGTCGTGACTAAGAAAAGGAAAAGCTACTGTGTGG
ACTTTGCAGCTATACAGCCACAAATCGCACTGCTACAAGAGATGCACGTTACTCATTTCAGGTTTAGCTTAGACTGGGCTCTGATA
TTGCCCCTCGGGAACCAATCTCAAGTCAATCACACCATTTTGCAGTATTACAGGTGTATGGCTAGCGAACTCGTGCGTGTCAACAT
TACTCCAGTGGTTGCCCTCTGGCAGCCGATGGCTCCCAACCAAGGCCTGCCCCGACTCCTGGCCAGGCAGGGCGCTTGGGAAAATC
CGTATACAGCACTGGCTTTCGCTGAGTACGCAAGGCTCTGCTTCCAGGAATTAGGACATCACGTGAAACTCTGGATTACGATGAAT
GAGCCCTATACCAGAAACATGACTTACAGCGCAGGCCACAATCTTTTAAAGGCCCACGCACTCGCCTGGCATGTCTATAATGAGAA
GTTTAGGCACGCTCAGAATGGAAAGATCTCCATCGCCCTCCAGGCTGATTGGATAGAGCCCGCATGCCCATTCTCTCAGAAGGACA
AAGAGGTGGCTGAAAGGGTGCTAGAGTTTGATATTGGTTGGCTGGCCGAGCCTATATTCGGGTCCGGGGATTACCCCTGGGTGATG
CGCGACTGGCTGAATCAGCGGAACAATTTCCTGCTCCCATACTTCACCGAGGATGAAAAAAAGCTCATTCAGGGTACATTCGATTT
TCTCGCGTTGTCCCACTACACGACAATACTCGTGGACTCTGAGAAAGAAGACCCAATCAAGTATAATGATTATCTAGAGGTTCAGG
AGATGACTGACATCACCTGGTTGAATTCTCCTTCTCAGGTGGCCGTGGTGCCCTGGGGACTCCGAAAAGTGTTGAATTGGTTAAAA
TTCAAGTACGGCGATCTACCGATGTATATCATTAGTAATGGCATTGACGATGGCCTGCACGCCGAAGATGACCAACTCAGGGTATA
CTATATGCAGAATTACATCAACGAGGCTCTGAAGGCACACATTTTGGACGGGATCAACCTATGTGGCTACTTCGCCTACTCCTTCA
ATGATAGGACTGCTCCTCGATTTGGCCTGTATAGGTATGCCGCTGACCAGTTCGAACCTAAGGCATCGATGAAGCATTATCGTAAA
ATTATCGACAGCAACGGGTTCCCAGGGCCAGAGACCTTGGAAAGGTTCTGCCCAGAGGAGTTTACCGTATGTACCGAATGCTCCTT
TTTTCATACTAGAAAGTCATAG
SEQ ID NO: 135: 
ATGCCTGCCTCTGCACCGCCGCGCCGGCCTAGACCACCGCCCCCATCCCTGAGCCTGTTGCTTGTGCTACTGGGTTTGGGCGGTCG
GCGTCTCCGCGCCGAGCCGGGCGACGGTGCCCAAACCTGGGCCAGGTTTTCCCGGCCTCCTGCCCCGGAGGCAGCTGGCCTGTTCC
AGGGTACATTCCCTGATGGTTTTCTCTGGGCAGTCGGCTCTGCTGCATATCAGACAGAGGGCGGGTGGCAACAGCACGGCAAGGGT
GCAAGCATTTGGGATACATTCACACACCACCCTTTGGCACCCCCAGGCGACTCCAGGAACGCCAGCCTGCCCCTGGGTGCTCCAAG
CCCCCTTCAGCCTGCGACTGGAGACGTTGCCTCTGACAGCTATAATAACGTCTTTCGAGATACGGAGGCACTCAGAGAGCTGGGCG
TTACCCACTACCGGTTCAGTATATCTTGGGCCCGGGTCCTCCCCAACGGAAGCGCCGGGGTGCCTAATCGTGAGGGCCTGAGATAC
TACCGGAGGCTCCTAGAAAGACTTCGAGAATTGGGGGTCCAGCCCGTTGTCACCTTGTATCACTGGGATCTGCCACAGCGCTTGCA
GGACGCTTACGGGGGTTGGGCCAATAGAGCCCTGGCTGATCATTTTAGAGATTATGCGGAACTCTGCTTCAGACACTTCGGAGGCC
AGGTGAAATATTGGATCACGATCGATAATCCATACGTGGTGGCCTGGCATGGATATGCTACCGGGAGACTGGCGCCGGGAATCCGC
GGGTCTCCCCGGCTCGGATATTTAGTCGCACACAATCTGCTGCTGGCCCACGCCAAAGTGTGGCACCTCTACAATACCTCCTTTAG
GCCGACGCAGGGAGGCCAGGTCAGCATCGCACTGAGCTCTCATTGGATAAATCCACGGAGGATGACCGACCACTCCATCAAGGAGT
GCCAAAAGAGTCTGGATTTTGTGCTTGGATGGTTCGCAAAACCAGTCTTTATCGACGGCGATTACCCTGAAAGTATGAAAAACAAC
CTGAGCTCTATTCTGCCCGATTTCACTGAGAGTGAAAAGAAATTTATAAAGGGAACTGCGGATTTTTTTGCTCTATGTTTCGGACC
TACCCTCTCGTTCCAACTGCTGGATCCTCATATGAAGTTCCGGCAGCTAGAGTCCCCGAACCTGAGACAACTCTTGAGTTGGATAG
ACCTGGAGTTTAACCATCCCCAGATTTTCATCGTTGAAAATGGCTGGTTCGTATCTGGTACGACGAAACGTGATGACGCCAAGTAT
ATGTACTACCTGAAAAAATTTATTATGGAGACCCTTAAAGCGATCAAGTTGGATGGCGTCGACGTTATCGGATACACAGCATGGTC
CCTCATGGATGGTTTTGAGTGGCACAGAGGTTACAGTATTCGACGAGGGCTCTTCTACGTCGACTTTCTGAGTCAGGATAAGATGC
TTCTGCCGAAATCTAGCGCTCTGTTCTACCAGAAGCTTATCGAAAAGAATGGCTTCCCTCCTCTGCCCGAGAATCAGCCCCTGGAG
GGTACATTCCCATGCGACTTCGCCTGGGGCGTGGTGGATAACTATATTCAGGTCGACACTACGCTGAGTCAGTTTACCGACCTGAA
CGTCTACCTGTGGGATGTGCACCATAGCAAGCGGTTGATCAAGGTGGATGGGGTAGTGACAAAAAAACGAAAGTCTTATTGCGTCG
ATTTCGCTGCGATTCAGCCTCAGATCGCGCTTTTACAGGAGATGCATGTTACACACTTTCGATTTAGCTTAGACTGGGCCTTGATT
CTGCCCCTGGGCAACCAATCTCAGGTTAATCATACCATCCTGCAGTATTACCGGTGCATGGCTTCAGAGTTGGTTAGGGTCAATAT
TACTCCCGTGGTGGCTCTCTGGCAGCCCATGGCCCCCAACCAGGGTCTGCCTAGACTCCTGGCCCGACAAGGCGCCTGGGAAAATC
CGTACACCGCACTGGCATTCGCCGAATATGCACGTTTGTGTTTTCAGGAGCTGGGGCATCATGTTAAGTTATGGATCACCATGAAT
GAGCCATATACCAGGAACATGACCTATTCCGCCGGACACAACCTCCTGAAGGCACATGCCCTGGCCTGGCATGTGTATAATGAGAA
GTTTAGGCACGCCCAGAATGGTAAAATCTCTATCGCGCTGCAGGCCGACTGGATCGAACCTGCCTGTCCATTCAGCCAGAAGGACA
AAGAAGTCGCCGAAAGAGTGTTGGAATTCGATATTGGCTGGCTTGCTGAGCCGATTTTTGGGAGCGGTGATTACCCCTGGGTAATG
CGGGACTGGCTGAATCAAAGGAACAACTTTTTACTTCCTTACTTTACCGAGGATGAAAAGAAGCTGATCCAGGGTACCTTCGATTT
CCTTGCCCTCAGTCACTACACCACGATCCTGGTGGATTCGGAAAAAGAGGATCCAATCAAATATAATGATTACCTGGAGGTGCAGG
AGATGACAGATATCACTTGGCTCAACTCACCTTCACAGGTCGCCGTTGTCCCTTGGGGTTTGCGTAAAGTCCTGAATTGGCTAAAA
TTCAAGTATGGCGATCTTCCCATGTACATTATCTCTAATGGCATAGATGATGGTCTTCACGCAGAAGACGACCAGCTCAGAGTTTA
TTATATGCAGAACTACATAAATGAAGCTCTTAAAGCTCACATCCTGGATGGGATCAACTTGTGTGGCTACTTCGCCTACAGCTTTA
ACGATAGGACGGCCCCCCGTTTCGGGTTGTACCGCTATGCTGCCGATCAGTTCGAGCCCAAGGCAAGCATGAAGCACTACCGTAAG
ATCATAGATTCCAATGGGTTTCCAGGCCCGGAAACACTGGAGCGGTTCTGTCCAGAGGAGTTCACGGTATGCACAGAGTGTTCTTT
CTTCCACACGAGGAAATCCTAG
SEQ ID NO: 136: ATGCCCGCCAGCGCCCCCCCCGGCGGCCCGGCCCCCCCCCCCAGTCTCTCTCTGCTCCTGGTGCTGCTGGGGCTGGAGGTCG 
GAGGTTGAGAGCTGAGCCAGGCGATGGGGCTCAGACATGGGCACGTTTTAGCCGTCCCCCAGCCCCTGAGGCTGCTGGGCTATTCC
AGGGTACTTTTCCTGATGGATTCCTGTGGGCCGTTGGCAGCGCCGCTTACCAGACCGAGGGCGGCTGGCAACAACATGGGAAAGGC
GCTTCCATTTGGGATACATTCACCCATCATCCCCTGGCCCCCCCCGGGGATTCGCGCAATGCATCGTTGCCCCTGGGTGCTCCTAG
CCCACTGCAGCCTGCAACCGGAGATGTGGCATCAGACTCATATAATAACGTGTTCCGCGACACCGAAGCGCTTAGGGAGTTGGGCG
TGACTCATTACAGGTTTTCCATTAGTTGGGCCAGGGTCCTACCGAACGGTTCTGCCGGCGTGCCAAATCGGGAGGGGCTACGGTAC
TATAGGAGACTGCTGGAAAGACTACGCGAGTTGGGGGTCCAGCCCGTTGTCACCCTTTACCACTGGGACCTCCCCCAGCGCCTGCA
AGACGCCTATGGGGGCTGGGCAAACAGAGCACTAGCCGACCACTTTCGGGACTACGCCGAACTTTGCTTCCGACACTTCGGGGGAC
AGGTCAAATATTGGATTACGATTGATAATCCCTATGTCGTGGCGTGGCACGGCTATGCCACAGGGAGGCTGGCACCGGGCATCAGA
GGATCGCCGAGGTTGGGGTATCTAGTGGCCCACAACCTTCTGCTCGCACACGCCAAGGTCTGGCACCTGTACAACACTTCCTTCCG
ACCCACACAGGGGGGCCAGGTTTCAATTGCTCTGAGCTCCCATTGGATTAATCCTCGCAGGATGACTGATCATTCTATTAAAGAGT
GCCAGAAGTCCTTAGACTTCGTTCTGGGCTGGTTTGCTAAACCTGTGTTTATCGACGGAGATTACCCAGAATCGATGAAGAACAAC
TTGTCTAGCATTCTGCCTGACTTCACAGAGAGCGAGAAGAAGTTCATAAAAGGAACCGCAGATTTTTTCGCACTGTGCTTCGGGCC
CACCCTGTCCTTTCAGCTTCTCGACCCTCATATGAAATTCCGCCAGCTCGAGTCTCCAAATCTGAGGCAGCTGTTGAGCTGGATCG
ACCTTGAATTCAACCACCCACAGATTTTCATCGTCGAGAACGGCTGGTTCGTGAGCGGGACGACAAAAAGGGACGACGCTAAGTAC
ATGTACTACCTGAAGAAATTTATCATGGAGACTTTAAAGGCGATCAAGCTAGATGGCGTTGATGTGATTGGTTACACGGCCTGGTC
ACTTATGGACGGTTTTGAATGGCACAGAGGCTATAGCATTCGCCGCGGTCTGTTTTATGTCGATTTTCTGAGCCAGGACAAGATGC
TCCTGCCGAAGAGCTCTGCACTTTTTTATCAAAAGCTGATTGAGAAGAATGGGTTTCCCCCTCTGCCCGAAAACCAACCCCTTGAA
GGAACTTTCCCATGTGACTTCGCCTGGGGCGTGGTGGACAACTACATCCAGGTGGATACCACCCTCAGCCAATTTACCGATCTCAA
CGTTTATCTGTGGGACGTGCACCATTCTAAGAGATTAATTAAGGTCGACGGCGTGGTCACCAAGAAGCGGAAGAGCTACTGCGTGG
ATTTTGCTGCCATCCAGCCCCAAATAGCCTTGCTTCAAGAAATGCACGTTACTCATTTCCGATTCTCGCTCGATTGGGCCCTGATT
CTGCCACTGGGTAATCAGAGTCAGGTGAATCACACCATTTTGCAGTATTACCGCTGCATGGCATCTGAGCTTGTCAGGGTGAACAT
CACGCCCGTGGTAGCACTATGGCAACCCATGGCACCAAACCAGGGCCTGCCTCGCCTGCTCGCCAGACAGGGGGCATGGGAGAATC
CTTACACCGCGCTGGCTTTTGCGGAATATGCGAGGCTGTGTTTCCAGGAATTGGGGCACCATGTCAAATTGTGGATCACTATGAAC
GAGCCCTACACAAGAAATATGACTTATAGTGCCGGGCATAACCTCTTAAAGGCTCACGCTCTGGCTTGGCACGTTTACAATGAGAA
GTTTCGTCATGCTCAGAATGGTAAAATCAGCATAGCGTTACAGGCCGATTGGATAGAGCCCGCCTGCCCATTCTCGCAAAAGGATA
AGGAAGTCGCTGAGAGGGTCCTCGAGTTCGACATCGGCTGGCTCGCTGAGCCTATCTTCGGTAGCGGGGACTACCCCTGGGTGATG
CGAGACTGGCTCAATCAGAGGAACAATTTCCTGCTACCCTATTTTACCGAGGATGAGAAAAAGTTGATACAGGGCACATTTGATTT
TTTGGCACTGTCCCATTATACCACGATTCTCGTCGACTCCGAGAAAGAGGATCCCATTAAATACAACGATTATCTCGAAGTGCAGG
AGATGACCGATATCACCTGGTTAAACTCCCCCTCGCAAGTGGCAGTGGTACCTTGGGGCCTGAGGAAAGTGCTCAACTGGCTGAAA
TTCAAGTATGGGGATCTCCCAATGTACATTATCTCTAACGGCATAGATGATGGTCTCCATGCCGAAGACGATCAGCTTCGCGTTTA
CTATATGCAAAACTACATAAATGAGGCCTTGAAGGCTCATATTCTGGACGGGATCAATCTATGTGGGTACTTCGCCTACAGTTTCA
ATGACCGCACCGCCCCACGCTTCGGGCTGTACAGGTACGCCGCAGATCAGTTTGAGCCTAAAGCCAGCATGAAACATTACCGCAAA
ATCATTGACTCTAATGGTTTCCCCGGACCCGAGACCCTCGAACGCTTTTGCCCCGAGGAATTCACCGTGTGCACAGAATGTTCTTT
TTTTCACACACGAAAGTCATAG
SEQ ID NO: 137: 
ATGCCTGCCAGCGCCCCACCCCGGCGGCCCCGGCCCCCACCACCCAGCCTGAGCCTGCTGCTGGTGCTGCTGGGCCTGGGCGGCCG
CCGCCTGCGGGCAGAGCCTGGAGATGGAGCCCAGACCTGGGCCCGCTTCAGCCGGCCCCCAGCCCCTGAGGCCGCCGGCCTCTTCC
AGGGCACCTTCCCAGATGGCTTCCTGTGGGCCGTGGGCAGCGCTGCCTACCAGACAGAGGGCGGCTGGCAGCAGCACGGCAAAGGA
GCCAGCATCTGGGACACCTTCACCCACCACCCCCTGGCCCCTCCTGGGGACAGCAGAAATGCCAGCCTGCCCCTGGGCGCCCCCAG
CCCCCTGCAGCCTGCCACTGGAGATGTGGCCAGTGACAGCTACAACAACGTGTTCCGGGACACAGAGGCCCTGCGGGAGCTGGGCG
TGACCCACTACCGCTTCAGCATCTCCTGGGCCCGGGTGCTGCCCAATGGATCTGCTGGGGTGCCCAACCGGGAGGGCCTGCGCTAC
TACCGCCGCCTGCTGGAGCGGCTGCGGGAGCTGGGCGTGCAGCCTGTGGTGACCCTGTACCACTGGGACCTGCCCCAGAGGCTGCA
GGATGCCTACGGCGGCTGGGCCAACAGAGCCCTGGCCGACCACTTCCGGGACTATGCAGAGCTGTGCTTCCGCCACTTTGGAGGCC
AGGTGAAGTACTGGATCACCATCGACAACCCCTACGTGGTGGCCTGGCACGGCTACGCCACCGGCCGCCTGGCCCCTGGCATCCGG
GGCAGCCCCCGGCTGGGCTACCTGGTGGCCCACAACCTGCTGCTGGCCCACGCCAAGGTGTGGCACCTGTACAACACCAGCTTCCG
GCCCACCCAGGGCGGCCAGGTGTCCATCGCCCTGAGCAGCCACTGGATCAACCCCAGGAGGATGACAGACCACAGCATCAAGGAGT
GCCAGAAGAGCCTGGACTTTGTGCTGGGCTGGTTTGCCAAGCCTGTGTTCATTGATGGAGACTACCCAGAGAGCATGAAGAACAAC
CTGAGCAGCATCCTGCCTGACTTCACAGAAAGTGAGAAGAAGTTCATCAAGGGCACAGCTGACTTCTTTGCCCTGTGCTTCGGCCC
CACCCTGTCCTTCCAGCTGCTGGACCCCCACATGAAGTTCCGGCAGCTGGAGAGCCCCAACCTGCGGCAGCTGCTGTCCTGGATTG
ACCTGGAGTTCAACCACCCCCAGATCTTCATCGTGGAGAATGGCTGGTTTGTGTCTGGCACCACCAAGAGAGATGATGCCAAGTAC
ATGTACTACCTGAAGAAGTTCATCATGGAGACCCTGAAGGCCATCAAGCTGGATGGAGTGGATGTGATTGGCTACACAGCCTGGAG
CCTGATGGATGGCTTTGAGTGGCACCGGGGCTACAGCATCAGAAGAGGCCTCTTCTACGTGGACTTCCTGAGCCAGGACAAGATGC
TGCTGCCCAAGAGCAGCGCCCTCTTCTACCAGAAGCTGATTGAGAAGAATGGCTTCCCCCCCCTGCCTGAGAACCAGCCCCTGGAG
GGCACCTTCCCCTGTGACTTTGCCTGGGGCGTGGTGGACAACTACATCCAGGTGGACACCACCCTGAGCCAGTTCACAGACCTGAA
TGTGTACCTGTGGGATGTGCACCACAGCAAGAGGCTCATCAAGGTGGATGGAGTGGTGACCAAGAAGAGGAAGAGCTACTGTGTGG
ACTTTGCTGCCATCCAGCCCCAGATCGCCCTGCTGCAGGAGATGCACGTGACCCACTTCCGCTTCAGCCTGGACTGGGCCCTGATC
CTGCCCCTGGGCAACCAGAGCCAGGTGAACCACACCATCCTGCAGTACTACCGCTGCATGGCCAGCGAGCTGGTGCGGGTGAACAT
CACCCCTGTGGTGGCCCTGTGGCAGCCCATGGCCCCCAACCAGGGCCTGCCCCGGCTGCTGGCCCGGCAGGGCGCCTGGGAGAACC
CCTACACAGCCCTGGCCTTTGCTGAATATGCCCGGCTGTGCTTCCAGGAGCTGGGCCACCACGTGAAGCTGTGGATCACCATGAAT
GAGCCCTACACCAGGAACATGACCTACAGCGCTGGCCACAACCTGCTGAAGGCCCACGCCCTGGCCTGGCACGTGTACAATGAGAA
GTTCAGACATGCCCAGAATGGCAAGATCAGCATCGCCCTGCAGGCCGACTGGATTGAACCAGCCTGCCCCTTCAGCCAGAAGGACA
AGGAGGTGGCCGAGCGGGTGCTGGAGTTTGATATTGGCTGGCTGGCCGAGCCCATCTTTGGAAGTGGGGACTACCCCTGGGTGATG
AGGGACTGGCTGAACCAGAGGAACAACTTCCTGCTGCCCTACTTCACAGAAGATGAGAAGAAGCTGATCCAGGGCACCTTTGACTT
CCTGGCCCTGAGCCACTACACCACCATCCTGGTGGACAGCGAGAAGGAGGACCCCATCAAGTACAATGACTACCTGGAGGTGCAGG
AGATGACAGACATCACCTGGCTGAACAGCCCCAGCCAGGTGGCCGTGGTGCCCTGGGGCCTGAGGAAGGTGCTGAACTGGCTGAAG
TTCAAGTATGGAGACCTGCCCATGTACATCATCAGCAATGGAATTGATGATGGCCTGCATGCTGAAGATGACCAGCTGCGGGTGTA
CTACATGCAGAACTACATCAATGAGGCCCTGAAGGCCCACATCCTGGATGGCATCAACCTGTGTGGCTACTTTGCCTACAGCTTCA
ATGACAGAACAGCCCCCAGGTTTGGCCTGTACCGCTATGCTGCTGACCAGTTTGAGCCCAAGGCCTCCATGAAGCACTACAGGAAG
ATCATCGACAGCAATGGCTTCCCTGGCCCTGAGACCCTGGAGCGCTTCTGCCCAGAGGAGTTCACCGTGTGCACAGAGTGCAGCTT
 CTTCCACACCAGGAAGAGCTAG
SEQ ID NO: 138: 
ATGCCCGCCAGCGCCCCCCCCAGAAGACCCAGACCCCCCCCCCCCAGCCTGAGCCTGCTGCTGGTGCTGCTGGGCCTGGGCGGCAG
AAGACTGAGAGCCGAGCCCGGCGACGGCGCCCAGACCTGGGCCAGATTCAGCAGACCCCCCGCCCCCGAGGCCGCCGGCCTGTTCC
AGGGCACCTTCCCCGACGGCTTCCTGTGGGCCGTGGGCAGCGCCGCCTACCAGACCGAGGGCGGCTGGCAGCAGCACGGCAAGGGC
GCCAGCATCTGGGACACCTTCACCCACCACCCCCTGGCCCCCCCCGGCGACAGCAGAAACGCCAGCCTGCCCCTGGGCGCCCCCAG
CCCCCTGCAGCCCGCCACCGGCGACGTGGCCAGCGACAGCTACAACAACGTGTTCAGAGACACCGAGGCCCTGAGAGAGCTGGGCG
TGACCCACTACAGATTCAGCATCAGCTGGGCCAGAGTGCTGCCCAACGGCAGCGCCGGCGTGCCCAACAGAGAGGGCCTGAGATAC
TACAGAAGACTGCTGGAGAGACTGAGAGAGCTGGGCGTGCAGCCCGTGGTGACCCTGTACCACTGGGACCTGCCCCAGAGACTGCA
GGACGCCTACGGCGGCTGGGCCAACAGAGCCCTGGCCGACCACTTCAGAGACTACGCCGAGCTGTGCTTCAGACACTTCGGCGGCC
AGGTGAAGTACTGGATCACCATCGACAACCCCTACGTGGTGGCCTGGCACGGCTACGCCACCGGCAGACTGGCCCCCGGCATCAGA
GGCAGCCCCAGACTGGGCTACCTGGTGGCCCACAACCTGCTGCTGGCCCACGCCAAGGTGTGGCACCTGTACAACACCAGCTTCAG
ACCCACCCAGGGCGGCCAGGTGAGCATCGCCCTGAGCAGCCACTGGATCAACCCCAGAAGAATGACCGACCACAGCATCAAGGAGT
GCCAGAAGAGCCTGGACTTCGTGCTGGGCTGGTTCGCCAAGCCCGTGTTCATCGACGGCGACTACCCCGAGAGCATGAAGAACAAC
CTGAGCAGCATCCTGCCCGACTTCACCGAGAGCGAGAAGAAGTTCATCAAGGGCACCGCCGACTTCTTCGCCCTGTGCTTCGGCCC
CACCCTGAGCTTCCAGCTGCTGGACCCCCACATGAAGTTCAGACAGCTGGAGAGCCCCAACCTGAGACAGCTGCTGAGCTGGATCG
ACCTGGAGTTCAACCACCCCCAGATCTTCATCGTGGAGAACGGCTGGTTCGTGAGCGGCACCACCAAGAGAGACGACGCCAAGTAC
ATGTACTACCTGAAGAAGTTCATCATGGAGACCCTGAAGGCCATCAAGCTGGACGGCGTGGACGTGATCGGCTACACCGCCTGGAG
CCTGATGGACGGCTTCGAGTGGCACAGAGGCTACAGCATCAGAAGAGGCCTGTTCTACGTGGACTTCCTGAGCCAGGACAAGATGC
TGCTGCCCAAGAGCAGCGCCCTGTTCTACCAGAAGCTGATCGAGAAGAACGGCTTCCCCCCCCTGCCCGAGAACCAGCCCCTGGAG
GGCACCTTCCCCTGCGACTTCGCCTGGGGCGTGGTGGACAACTACATCCAGGTGGACACCACCCTGAGCCAGTTCACCGACCTGAA
CGTGTACCTGTGGGACGTGCACCACAGCAAGAGACTGATCAAGGTGGACGGCGTGGTGACCAAGAAGAGAAAGAGCTACTGCGTGG
ACTTCGCCGCCATCCAGCCCCAGATCGCCCTGCTGCAGGAGATGCACGTGACCCACTTCAGATTCAGCCTGGACTGGGCCCTGATC
CTGCCCCTGGGCAACCAGAGCCAGGTGAACCACACCATCCTGCAGTACTACAGATGCATGGCCAGCGAGCTGGTGAGAGTGAACAT
CACCCCCGTGGTGGCCCTGTGGCAGCCCATGGCCCCCAACCAGGGCCTGCCCAGACTGCTGGCCAGACAGGGCGCCTGGGAGAACC
CCTACACCGCCCTGGCCTTCGCCGAGTACGCCAGACTGTGCTTCCAGGAGCTGGGCCACCACGTGAAGCTGTGGATCACCATGAAC
GAGCCCTACACCAGAAACATGACCTACAGCGCCGGCCACAACCTGCTGAAGGCCCACGCCCTGGCCTGGCACGTGTACAACGAGAA
GTTCAGACACGCCCAGAACGGCAAGATCAGCATCGCCCTGCAGGCCGACTGGATCGAGCCCGCCTGCCCCTTCAGCCAGAAGGACA
AGGAGGTGGCCGAGAGAGTGCTGGAGTTCGACATCGGCTGGCTGGCCGAGCCCATCTTCGGCAGCGGCGACTACCCCTGGGTGATG
AGAGACTGGCTGAACCAGAGAAACAACTTCCTGCTGCCCTACTTCACCGAGGACGAGAAGAAGCTGATCCAGGGCACCTTCGACTT
CCTGGCCCTGAGCCACTACACCACCATCCTGGTGGACAGCGAGAAGGAGGACCCCATCAAGTACAACGACTACCTGGAGGTGCAGG
AGATGACCGACATCACCTGGCTGAACAGCCCCAGCCAGGTGGCCGTGGTGCCCTGGGGCCTGAGAAAGGTGCTGAACTGGCTGAAG
TTCAAGTACGGCGACCTGCCCATGTACATCATCAGCAACGGCATCGACGACGGCCTGCACGCCGAGGACGACCAGCTGAGAGTGTA
CTACATGCAGAACTACATCAACGAGGCCCTGAAGGCCCACATCCTGGACGGCATCAACCTGTGCGGCTACTTCGCCTACAGCTTCA
ACGACAGAACCGCCCCCAGATTCGGCCTGTACAGATACGCCGCCGACCAGTTCGAGCCCAAGGCCAGCATGAAGCACTACAGAAAG
ATCATCGACAGCAACGGCTTCCCCGGCCCCGAGACCCTGGAGAGATTCTGCCCCGAGGAGTTCACCGTGTGCACCGAGTGCAGCTT
CTTCCACACCAGAAAGAGCTAG
SEQ ID NO: 49:
SEQ ID NO: 50: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 51:
   MPASAPPRRPRPPPPSLSLLLVLLGLGGRRLRA
SEQ ID NO: 52: 
LFQGTFPDGFLWAVGSAAYQTEGGWQQHGKGASIWDTFTHHPLAPPGDSRNASLPLGAPSPLQPATGDVASDSYNNVFRDTEALRE
LGVTHYRFSISWARVLPNGSAGVPNREGLRYYRRLLERLRELGVQPVVTLYHWDLPQRLQDAYGGWANRALADHFRDYAELCFRHF
GGQVKYWITIDNPYVVAWHGYATGRLAPGIRGSPRLGYLVAHNLLLAHAKVWHLYNTSFRPTQGGQVSIALSSHWINPRRMTDHSI
KECQKSLDFVLGWFAKPVFIDGDYPESMKNNLSSILPDFTESEKKFIKGTADFFALCFGPTLSFQLLDPHMKFRQLESPNLRQLLS
WIDLEFNHPQIFIVENGWFVSGTTKRDDAKYMYYLKKFIMETLKAIKLDGVDVIGYTAWSLMDGFEWHRGYSIRRGLFYVDFLSQD
KMLLPKSSALFYQKLIEKNGF
SEQ ID NO: 53:
LFQGTFPDGFLWAVGSAAYQTEGGWQQHGKGASIWDTFTHHPLAPPGDSRNASLPLGAPSPLQPATGDVASDSYNNVFRDTEALRE
 
LGVTHYRFSISWARVLPNGSAGVPNREGLRYYRRLLERLRELGVQPVVTLYHWDLPQRLQDAYGGWANRALADHFRDYAELCFRHF
GGQVKYWITIDNPYVVAWHGYATGRLAPGIRGSPRLGYLVAHNLLLAHAKVWHLYNTSFRPTQGGQVSIALSSHWINPRRMTDHSI
KECQKSLDFVLGWFAKPVFIDGDYPESMKNNLSSILPDVTESEKKFIKGTADFFALSFGPTLSFQLLDPHMKFRQLESPNLRQLLS
WIDLEFNHPQIFIVENGWFVSGTTKRDDAKYMYYLKKFIMETLKAIKLDGVDVIGYTAWSLMDGFEWHRGYSIRRGLFYVDFLSQD
KMLLPKSSALFYQKLIEKNGF
SEQ ID NO: 54: 
LEGTFPCDFAWGVVDNYIQVDTTLSQFTDLNVYLWDVHHSKRLIKVDGVVTKKRKSYCVDFAAIQPQIALLQEMHVTHFRFSLDWA
LILPLGNQSQVNHTILQYYRCMASELVRVNITPVVALWQPMAPNQGLPRLLARQGAWENPYTALAFAEYARLCFQELGHHVKLWIT
MNEPYTRNMTYSAGHNLLKAHALAWHVYNEKFRHAQNGKISIALQADWIEPACPFSQKDKEVAERVLEFDIGWLAEPIFGSGDYPW
VMRDWLNQRNNFLLPYFTEDEKKLIQGTFDFLALSHYTTILVDSEKEDPIKYNDYLEVQEMTDITWLNSPSQVAVVPWGLRKVLNW
LKFKYGDLPMYIISNGIDDGLHAEDDQLRVYYMQNYINEALKAHILDGINLCGYFAYSFNDRTAPRFGLYRYAADQFEPKASMKHY
RKIIDSNG
SEQ ID NO: 55: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGF
SEQ ID NO: 56: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGF
SEQ ID NO: 57: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKS
SEQ ID NO: 58: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKS
SEQ ID NO: 59: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 60: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 76: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKSLLAFIAFLFFASIIISLSLIFYYSKKGRRSYK
SEQ ID NO: 61:
   GGGAGACAUAAACCCUGGCGCGCUCGCGGCCCGGCACUCUUCUGGUCCCCACAGACUCAGAGAGAACCCACC
SEQ ID NO: 62: 
GCUGGAGCCUCGGUGGCCAUGCUUCUUGCCCCUUGGGCCUCCCCCCAGCCCCUCCUCCCCUUCCUGCACCCGUACCCCCGUGGUCU
UUGAAUAAAGUCUGAGUGGGCGGCA
SEQ ID NO: 63:
   YCCANCCCWNUCYCC
SEQ ID NO: 77:
   TGCTGCCGTAATACGACTCACTATAAGG
SEQ ID NO: 78:

TTTTTTTTTTTTTTTTTTTTTTTTTTTTTTTTTTTTTTTTTTTTTTTTTTTTTTTTTTTTTTTTTTTTTTTTTTTTTTTTTTTTTTT
TTTTTTTTTTTTTTTTTTTTTTTTTTTTTTTTTTTGCCGCCCACTCAGACTTTATTC

## Claims

1. Klotho messenger-RNA (mRNA), wherein the mRNA has a 5' CAP region, a 5' untranslated region (5'-UTR), a coding region encoding a Klotho polypeptide, a 3' untranslated region (3'-UTR) and a polyadenosine tail (poly-A tail),
wherein the Klotho polypeptide comprises the signal peptide, the KL1 domain, and the KL2 domain of human Klotho,
wherein the coding region encoding the Klotho polypeptide comprises an RNA sequence having at least 93 % sequence identity to SEQ ID NO: 18, and
wherein the coding region encoding the Klotho polypeptide has a GC content between 54 % and 64 %.

2. The Klotho mRNA according to claim 1, wherein the coding region encoding the Klotho polypeptide comprises a sequence selected from SEQ ID NO: 1, 2, 3, 4, 5, 6, 7, 8, 9, 10, 11, 12, 13, 14, 15, 16, 18, 19, 20, 21, 64, 65, 66, 67, 68, 69, 70, 71, 79, 80, 81, 82, 83, 84, 85, 86, 87, 88, 89, 90, 91, 92, 93, 94, 95, 96, 97, 98, 99, 120, 121, 122, 123, 124, 125, 126, 127; preferably wherein the coding region encoding the Klotho polypeptide is selected from SEQ ID NO: 18, 19, 20, 21, 96, 97, 98, 99, 68, 69, 70, 71, 124, 125, 126, 127; preferably wherein the Klotho mRNA is selected from SEQ ID NO: 29, 30, 31, 32, 100, 101, 102, 103, 72, 73, 74, 75, 128, 129, 130, 131, preferably SEQ ID NO: 29, 100, 72, 128.

3. The Klotho mRNA according to any one of claims 1 or 2, wherein the Klotho polypeptide has at least 95 % sequence identity to SEQ ID NO: 49or 57, preferably wherein the Klotho polypeptide is selected from SEQ ID NO: 49, 57, 58, or 76, preferably 49 or 57, especially 57.

4. The Klotho mRNA according to any one of claims 1 to 3, wherein in the Klotho mRNA, at least 5%, preferably at least 10%, preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

5. Pharmaceutical formulation comprising the Klotho mRNA according to any one of claims 1 to 4 and a pharmaceutically acceptable carrier.

6. The pharmaceutical formulation according to claim 5, wherein the formulation comprises a further mRNA, wherein the further mRNA has a further 5' CAP region, a further 5'-UTR, a further coding region encoding a further polypeptide, a further 3'-UTR and a further poly-A tail; preferably wherein the further polypeptide is Bactericidal/Permeability Increasing Protein Family B, member 4 (BPIFB4) protein or an isoform or variant thereof, preferably Longevity-Associated Variant of BPIFB4 (LAV-BPIFB4); and/or wherein the further polypeptide is a protein selected from Apolipoprotein E, Tumor protein p53, Sirtuin 1 protein, FOXO1 transcription factor, Cholinergic receptor nicotinic alpha 3 subunit, SH2B adaptor protein 3, Cyclin dependent kinase inhibitor 2A, Elongation of very-long-chain fatty acids-like 2, Werner protein, Paraoxonase 1, Superoxide dismutase 2, Lamin A protein, Cholesteryl ester transfer protein, Apolipoprotein C3, Microsomal triglyceride transfer protein, Phosphatidylinositol 3-kinase (PI3K), Insulin-like growth factor 1 (IGF-1) receptor, Protein-L-isoaspartyl methyltransferase, Growth hormone, cAMP-response element binding protein, Mitogen-activated protein kinase, epidermal growth factor receptor, Nuclear factor kappa B, Phospholipase C beta, Methionine sulfoxide reductase A, Mediator of cell motility 1, Nei like DNA Glycosylase 1, Peroxisome proliferator-activated receptor gamma 2, Eukaryotic translation initiation factor 3 subunit K, ATM serine/threonine kinase, B-cell lymphoma 2, Cell division cycle 42, Diacylglycerol O -acyltransferase 1, Early growth response 1, Fibroblast growth factor 23, Fibroblast growth factor 21, Fructosamine 3 kinase related protein, Phosphoglycolate phosphatase, Insulin receptor substrate 1, Polycomb complex protein BMI-1, Neuregulin 1, Signal transducer and activator of transcription, E2F Transcription Factor 1, Vascular endothelial growth factor A, Xenobiotic metabolizing enzymes, Myc proto-oncogene protein, C-X-C chemokine receptor type 4, Silent information regulator 2, Extracellular signal-regulated kinase, and SLC31.

7. Kit for administering the Klotho mRNA according to any one of claims 1 to 4 to an individual, preferably a human, comprising
- the Klotho mRNA according to any one of claims 1 to 4, and
- a device for administering the Klotho mRNA.

8. A DNA molecule comprising a DNA sequence encoding the coding region of the Klotho mRNA as defined in any one of claims 1 to 4.

9. A cell comprising the DNA molecule as defined claim 8, preferably wherein the cell is a mammalian cell, preferably a Human Embryonic Kidney (HEK) 293 cell.

10. A therapeutic delivery device comprising cells as defined in claim 9, wherein the cells are encapsulated in a biocompatible scaffold.

11. An extracellular vesicle (EV) comprising the Klotho mRNA as defined in any one of claims 1 to 4, preferably wherein the EV is derived from the cell as defined in claim 9.

12. A population of EVs as defined in claim 11, wherein the average number of Klotho mRNA molecules per EV throughout the population of EVs is above one per EV.

13. A method for producing the EV according to claim 11 or the population of EVs according to claim 12 comprising:
- Culturing the cell as defined in claim 9 under conditions suitable for the formation of EVs; and
- Isolating the EVs.

14. A pharmaceutical formulation comprising the EV as defined in claim 11 or the population of EVs as defined in claim 12 and a pharmaceutically acceptable carrier.

15. The Klotho mRNA according to any one of claims 1 to 4, the pharmaceutical formulation according to claim 5 or 6, the EV according to claim 11, or the population of EVs according to claim 12 for use as a medicament.

## Patentansprüche

1. Klotho-Messenger-RNA (mRNA), wobei die mRNA eine 5'-Cap-Region, eine 5'-untranslatierte Region (5'-UTR), eine kodierende Region, die ein Klotho-Polypeptid kodiert, eine 3'-untranslatierte Region (3'-UTR) und einen Poly-Adenosin-Schwanz (Poly-A-Schwanz) aufweist,
wobei das Klotho-Polypeptid das Signalpeptid, die KL1-Domäne und die KL2-Domäne des humanen Klotho umfasst,
wobei die kodierende Region, die das Klotho-Polypeptid kodiert, eine RNA-Sequenz umfasst, die mindestens 93 % Sequenzidentität zu SEQ ID NO: 18 aufweist, und
wobei die kodierende Region, die das Klotho-Polypeptid kodiert, einen GC-Gehalt zwischen 54 % und 64 % aufweist.

2. Klotho-mRNA nach Anspruch 1, wobei die kodierende Region, die das Klotho-Polypeptid kodiert, eine Sequenz ausgewählt aus SEQ ID NO: 1, 2, 3, 4, 5, 6, 7, 8, 9, 10, 11, 12, 13, 14, 15, 16, 18, 19, 20, 21, 64, 65, 66, 67, 68, 69, 70, 71, 79, 80, 81, 82, 83, 84, 85, 86, 87, 88, 89, 90, 91, 92, 93, 94, 95, 96, 97, 98, 99, 120, 121, 122, 123, 124, 125, 126, 127 umfasst; vorzugsweise wobei die kodierende Region, die das Klotho-Polypeptid kodiert, ausgewählt ist aus SEQ ID NO: 18, 19, 20, 21, 96, 97, 98, 99, 68, 69, 70, 71, 124, 125, 126, 127; vorzugsweise wobei die Klotho-mRNA ausgewählt ist aus SEQ ID NO: 29, 30, 31, 32, 100, 101, 102, 103, 72, 73, 74, 75, 128, 129, 130, 131, vorzugsweise SEQ ID NO: 29, 100, 72, 128.

3. Klotho-mRNA nach einem der Ansprüche 1 oder 2, wobei das Klotho-Polypeptid mindestens 95 % Sequenzidentität zu SEQ ID NO: 49 oder 57 aufweist, vorzugsweise wobei das Klotho-Polypeptid ausgewählt ist aus SEQ ID NO: 49, 57, 58 oder 76, vorzugsweise 49 oder 57, insbesondere 57.

4. Klotho-mRNA nach einem der Ansprüche 1 bis 3, wobei in der Klotho-mRNA mindestens 5 %, vorzugsweise mindestens 10 %, vorzugsweise mindestens 30 %, insbesondere mindestens 50 % aller
- Cytidin-Reste durch 5-Methyl-Cytidin-Reste ersetzt sind, und/oder
- Cytidin-Reste durch 2-Amino-2-desoxy-Cytidin-Reste ersetzt sind, und/oder
- Cytidin-Reste durch 2-Fluor-2-desoxy-Cytidin-Reste ersetzt sind, und/oder
- Cytidin-Reste durch 2-Thio-Cytidin-Reste ersetzt sind, und/oder
- Cytidin-Reste durch 5-Iod-Cytidin-Reste ersetzt sind, und/oder
- Uridin-Reste durch Pseudo-Uridin-Reste ersetzt sind, und/ oder
- Uridin-Reste durch 1-Methyl-Pseudo-Uridin-Reste ersetzt sind, und/oder
- Uridin-Reste durch 2-Thio-Uridin-Reste ersetzt sind, und/ oder
- Uridin-Reste durch 5-Methyl-Uridin-Reste ersetzt sind, und/oder
- Adenosin-Reste durch N6-Methyl-Adenosin-Reste ersetzt sind.

5. Pharmazeutische Formulierung, umfassend die Klotho-mRNA nach einem der Ansprüche 1 bis 4 und einen pharmazeutisch akzeptablen Träger.

6. Pharmazeutische Formulierung nach Anspruch 5, wobei die Formulierung eine weitere mRNA umfasst, wobei die weitere mRNA eine weitere 5'-Cap-Region, eine weitere 5'-UTR, eine weitere kodierende Region, die ein weiteres Polypeptid kodiert, eine weitere 3'-UTR und einen weiteren Poly-A-Schwanz aufweist; vorzugsweise wobei das weitere Polypeptid ein Protein der Bakterizid/Permeabilitäts-steigernden Protein-Familie B, Mitglied 4 (BPIFB4) oder eine Isoform oder Variante davon ist, vorzugsweise die Langlebigkeits-assoziierte Variante von BPIFB4 (LAV-BPIFB4); und/oder wobei das weitere Polypeptid ein Protein ausgewählt aus Apolipoprotein E, Tumorprotein p53, Sirtuin-1-Protein, Transkriptionsfaktor FOX01, cholinerger Rezeptor nikotinischer alpha-3-Untereinheit, SH2B-Adapterprotein 3, Cyclinabhängiger Kinaseinhibitor 2A, Elongation of very-long-chain fatty acids-like 2, Werner-Protein, Paraoxonase-1, Superoxiddismutase 2, Lamin-A-Protein, Cholesterinester-Transferprotein, Apolipoprotein C3, mikrosomales Triglyzerid-Transferprotein, Phosphatidylinositol-3-Kinase (PI3K), Insulinähnlicher Wachstumsfaktor-1-(IGF-1)-Rezeptor, Protein-L-isoaspartyl-Methyltransferase, Wachstumshormon, cAMP response element binding protein, Mitogen-aktivierte Proteinkinase, epidermaler Wachstumsfaktor-Rezeptor, nuklearer Faktor Kappa B, Phospholipase C beta, Methioninsulfoxid-Reduktase A, Mediator der Zellmotilität 1, Nei-ähnliche DNA-Glykosylase 1, Peroxisomen-Proliferator-aktivierter Rezeptor gamma 2, Eukaryotischer Translationsinitiationsfaktor 3 Untereinheit K, ATM-Serin/Threonin-Kinase, B-Zell-Lymphom 2, Zellteilungszyklus 42, Diacylglycerol-O-acyltransferase 1, Early Growth Response 1, Fibroblasten-Wachstumsfaktor 23, Fibroblasten-Wachstumsfaktor 21, Fructosamin-3-Kinase-verwandtes Protein, Phosphoglykolat-Phosphatase, Insulinrezeptor-Substrat 1, Polycomb-Komplex-Protein BMI-1, Neuregulin 1, Signaltransduktor und Transkriptionsaktivator, E2F-Transkriptionsfaktor 1, vaskulärer endothelialer Wachstumsfaktor A, Xenobiotika-metabolisierende Enzyme, Myc-Protoonkogen-Protein, C-X-C-Chemokin-Rezeptor Typ 4, Silent Information Regulator 2, extrazellulär-signalregulierte Kinase, und SLC31, ist.

7. Kit zur Verabreichung der Klotho-mRNA nach einem der Ansprüche 1 bis 4 an ein Individuum, vorzugsweise einen Menschen, umfassend
- die Klotho-mRNA nach einem der Ansprüche 1 bis 4, und
- eine Vorrichtung zur Verabreichung der Klotho-mRNA.

8. DNA-Molekül, umfassend eine DNA-Sequenz, die die kodierende Region der Klotho-mRNA wie in einem der Ansprüche 1 bis 4 definiert, kodiert.

9. Zelle, umfassend das DNA-Molekül wie in Anspruch 8 definiert, vorzugsweise wobei die Zelle eine Säugetierzelle ist, vorzugsweise eine humane embryonale Nieren (HEK)-293-Zelle.

10. Therapeutische Abgabevorrichtung, umfassend Zellen wie in Anspruch 9 definiert, wobei die Zellen in einem biokompatiblen Gerüst verkapselt sind.

11. Extrazelluläres Vesikel (EV), umfassend die Klotho-mRNA wie in einem der Ansprüche 1 bis 4 definiert, vorzugsweise wobei das EV von der Zelle wie in Anspruch 9 definiert, abgeleitet ist.

12. Population von EVs wie in Anspruch 11 definiert, wobei die durchschnittliche Anzahl von Klotho-mRNA-Molekülen pro EV in der gesamten Population von EVs über eins pro EV liegt.

13. Verfahren zur Herstellung des EV nach Anspruch 11 oder der Population von EVs nach Anspruch 12, umfassend:
- Kultivieren der Zelle wie in Anspruch 9 definiert unter Bedingungen, die für die Bildung von EVs geeignet sind; und
- Isolieren der EVs.

14. Pharmazeutische Formulierung, umfassend das EV wie in Anspruch 11 definiert oder die Population von EVs wie in Anspruch 12 definiert und einen pharmazeutisch akzeptablen Träger.

15. Die Klotho-mRNA nach einem der Ansprüche 1 bis 4, die pharmazeutische Formulierung nach Anspruch 5 oder 6, das EV nach Anspruch 11 oder die Population von EVs nach Anspruch 12 zur Anwendung als Medikament.

## Revendications

1. ARN messager (ARNm) de Klotho, dans lequel l'ARNm a une région 5' CAP, une région 5' non traduite (5'-UTR), une région codante codant pour un polypeptide Klotho, une région 3' non traduite (3'-UTR) et une queue polyadénosine (poly(A)),
dans lequel le polypeptide Klotho comprend le peptide signal, le domaine KL1 et le domaine KL2 du Klotho humain,
dans lequel la région codante codant pour le polypeptide Klotho comprend une séquence d'ARN ayant au moins 93 % d'identité de séquence avec SEQ ID NO: 18, et
dans lequel la région codante codant pour le polypeptide Klotho a une teneur en GC comprise entre 54 % et 64 %.

2. ARNm de Klotho selon la revendication 1, dans lequel la région codante codant pour le polypeptide Klotho comprend une séquence sélectionnée parmi SEQ ID NO: 1, 2, 3, 4, 5, 6, 7, 8, 9, 10, 11, 12, 13, 14, 15, 16, 18, 19, 20, 21, 64, 65, 66, 67, 68, 69, 70, 71, 79, 80, 81, 82, 83, 84, 85, 86, 87, 88, 89, 90, 91, 92, 93, 94, 95, 96, 97, 98, 99, 120, 121, 122, 123, 124, 125, 126, 127; de préférence dans lequel la région codante codant pour le polypeptide Klotho est sélectionnée parmi SEQ ID NO: 18, 19, 20, 21, 96, 97, 98, 99, 68, 69, 70, 71, 124, 125, 126, 127; de préférence dans lequel l'ARNm de Klotho est sélectionné parmi SEQ ID NO: 29, 30, 31, 32, 100, 101, 102, 103, 72, 73, 74, 75, 128, 129, 130, 131, de préférence SEQ ID NO: 29, 100, 72, 128.

3. ARNm de Klotho selon l'une quelconque des revendications 1 ou 2, dans lequel le polypeptide Klotho a au moins 95 % d'identité de séquence avec SEQ ID NO: 49 ou 57, de préférence dans lequel le polypeptide Klotho est sélectionné parmi SEQ ID NO: 49, 57, 58, ou 76, de préférence 49 ou 57, en particulier 57.

4. ARNm de Klotho selon l'une quelconque des revendications 1 à 3, dans lequel dans l'ARNm de Klotho, au moins 5 %, de préférence au moins 10 %, de préférence au moins 30 %, en particulier au moins 50 % de tous
- les résidus cytidine sont remplacés par des résidus 5-méthyl-cytidine, et/ou
- les résidus cytidine sont remplacés par des résidus 2-amino-2-désoxy-cytidine, et/ou
- les résidus cytidine sont remplacés par des résidus 2-fluoro-2-désoxy-cytidine, et/ou
- les résidus cytidine sont remplacés par des résidus 2-thio-cytidine, et/ou
- les résidus cytidine sont remplacés par des résidus 5-iodo-cytidine, et/ou
- les résidus uridine sont remplacés par des résidus pseudo-uridine, et/ou
- les résidus uridine sont remplacés par des résidus 1-méthyl-pseudo-uridine, et/ou
- les résidus uridine sont remplacés par des résidus 2-thio-uridine, et/ou
- les résidus uridine sont remplacés par des résidus 5-méthyl-uridine, et/ou
- les résidus adénosine sont remplacés par des résidus N6-méthyl-adénosine.

5. Formulation pharmaceutique comprenant l'ARNm de Klotho selon l'une quelconque des revendications 1 à 4 et un véhicule pharmaceutiquement acceptable.

6. Formulation pharmaceutique selon la revendication 5, dans laquelle la formulation comprend un ARNm supplémentaire, dans laquelle l'ARNm supplémentaire a une région 5' CAP supplémentaire, une région 5'-UTR supplémentaire, une région codante supplémentaire codant pour un polypeptide supplémentaire, une région 3'-UTR supplémentaire et une queue poly(A) supplémentaire; de préférence dans laquelle le polypeptide supplémentaire est la protéine de la famille B de la protéine augmentant la perméabilité/bactéricide, membre 4 (BPIFB4) ou une isoforme ou variante de celle-ci, de préférence la variante associée à la longévité de BPIFB4 (LAV-BPIFB4); et/ou dans laquelle le polypeptide supplémentaire est une protéine sélectionnée parmi l'Apolipoprotéine E, la protéine tumorale p53, la protéine Sirtuin 1, le facteur de transcription FOXO1, la sous-unité alpha 3 du récepteur nicotinique cholinergique, la protéine adaptatrice SH2B 3, l'inhibiteur de kinase dépendant de la cycline 2A, l'élongation des acides gras à très longue chaîne-like 2, la protéine Werner, la Paraoxonase 1, la Superoxyde dismutase 2, la protéine Lamine A, la protéine de transfert des esters de cholestérol, l'Apolipoprotéine C3, la protéine de transfert des triglycérides microsomaux, la Phosphatidylinositol 3-kinase (PI3K), le récepteur du facteur de croissance analogue à l'insuline 1 (IGF-1), la Protéine-L-isoaspartyl méthyltransférase, l'hormone de croissance, la protéine de liaison à l'élément de réponse à l'AMP cyclique, la protéine kinase activée par les mitogènes, le récepteur du facteur de croissance épidermique, le facteur nucléaire kappa B, la Phospholipase C bêta, la Méthionine sulfoxyde réductase A, le Médiateur de la motilité cellulaire 1, la Nei like DNA Glycosylase 1, le récepteur gamma 2 activé par les proliférateurs de peroxysomes, la sous-unité K du facteur d'initiation de la traduction eucaryote 3, l'ATM sérine/thréonine kinase, le B-cell lymphoma 2, le Cell division cycle 42, la Diacylglycérol O-acyl-transférase 1, l'Early growth response 1, le facteur de croissance des fibroblastes 23, le facteur de croissance des fibroblastes 21, la protéine apparentée à la Fructosamine 3 kinase, la Phosphoglycolate phosphatase, le substrat 1 du récepteur de l'insuline, la protéine du complexe Polycomb BMI-1, la Neurégu-line 1, le transducteur de signal et activateur de transcription, le facteur de transcription E2F 1, le facteur de croissance de l'endothélium vasculaire A, les enzymes métabolisant les xénobiotiques, la protéine proto-oncogène Myc, le récepteur de type 4 de la chimiokine C-X-C, le régulateur d'information silencieux 2, la kinase régulée par les signaux extracellulaires, et SLC31.

7. Kit pour administrer l'ARNm de Klotho selon l'une quelconque des revendications 1 à 4 à un individu, de préférence un humain, comprenant
- l'ARNm de Klotho selon l'une quelconque des revendications 1 à 4, et
- un dispositif pour administrer l'ARNm de Klotho.

8. Molécule d'ADN comprenant une séquence d'ADN codant pour la région codante de l'ARNm de Klotho telle que définie dans l'une quelconque des revendications 1 à 4.

9. Cellule comprenant la molécule d'ADN telle que définie dans la revendication 8, de préférence dans laquelle la cellule est une cellule de mammifère, de préférence une cellule HEK (Human Embryonic Kidney) 293.

10. Dispositif d'administration thérapeutique comprenant des cellules telles que définies dans la revendication 9, dans lequel les cellules sont encapsulées dans une matrice biocompatible.

11. Vésicule extracellulaire (VE) comprenant l'ARNm de Klotho tel que défini dans l'une quelconque des revendications 1 à 4, de préférence dans laquelle la VE est dérivée de la cellule telle que définie dans la revendication 9.

12. Population de VE telles que définies dans la revendication 11, dans laquelle le nombre moyen de molécules d'ARNm de Klotho par VE dans l'ensemble de la population de VE est supérieur à un par VE.

13. Procédé de production de la VE selon la revendication 11 ou de la population de VE selon la revendication 12 comprenant:
- la culture de la cellule telle que définie dans la revendication 9 dans des conditions appropriées pour la formation de VE; et
- l'isolement des VE.

14. Formulation pharmaceutique comprenant la VE telle que définie dans la revendication 11 ou la population de VE telle que définie dans la revendication 12 et un véhicule pharmaceutique-ment acceptable.

15. L'ARNm de Klotho selon l'une quelconque des revendications 1 à 4, la formulation pharmaceutique selon la revendication 5 ou 6, la VE selon la revendication 11, ou la population de VE selon la revendication 12 pour utilisation en tant que médicament.
